# EUROPEAN PATENT SPECIFICATION

(11) **EP 2 156 183 B1**
(45) Date of publication and mention of the grant of the patent: **07.08.2013**
(21) Application number: 08750553.3
(22) Date of filing: 09.05.2008
(51) Int. Cl.: G01N 33/53, G01N 33/68, C07K 14/705

(54) **PROGNOSTIC ASSAY FOR DETERMINING T CELL RESPONSE TO HLA ANTIGENS AND USE THEREOF IN FIELD OF TISSUE TRANSPLANTATION**
PROGNOSTISCHES TESTVERFAHREN ZUR BESTIMMUNG DER T-ZELLANTWORT AUF HLA-ANTIGENE UND VERWENDUNG DAVON AUF DEM GEBIET DER GEWEBETRANSPLANTATION
ANALYSE PRONOSTIQUE POUR DÉTERMINER UNE RÉPONSE DE LYMPHOCYTE T À DES ANTIGÈNES HLA ET UTILISATION DE CELLE-CI DANS LE DOMAINE DE LA TRANSPLANTATION TISSULAIRE

(30) Priority: 09.05.2007 WO PCT/GB2007/001690; 15.08.2007 GB 0715949; 14.11.2007 GB 0722378
(43) Date of publication of application: 24.02.2010
(73) Proprietor: Circassia Limited, Oxford OX4 4GA (GB); THE UNIVERSITY OF BIRMINGHAM, Birmingham B15 2TT (GB)
(72) Inventor: BALL, Simon, Birmingham B15 2LN (GB)
(74) Representative: Woods, Geoffrey Corlett
(86) International application number: PCT/GB2008/001614
(87) International publication number: WO 2008/139163

(56) References cited:
- WO-A-93/17699
- WO-A-03/016905
- US-B1- 7 135 302
- HANVESAKUL R ET AL: "Indirect recognition of T-cell epitopes derived from the [alpha]3 and transmembrane domain of HLA-A2" AMERICAN JOURNAL OF TRANSPLANTATION 200705 GB, vol. 7, no. 5, May 2007 (2007-05), pages 1148-1157, XP002493273 ISSN: 1600-6135 1600-6143
- WHITE J ET AL: "Soluble class I MHC with beta2-microglobulin covalently linked peptides: specific binding to a T cell hybridoma" JOURNAL OF IMMUNOLOGY, THE WILLIAMS AND WILKINS CO. BALTIMORE, vol. 162, no. 5, 1 January 1999 (1999-01-01), pages 2671-2676, XP002959841 ISSN: 0022-1767
- REINSMOEN N L: "Cellular methods used to evaluate the immune response in transplantation" TISSUE ANTIGENS, MUNKSGAARD, COPENHAGEN, DK, vol. 59, no. 4, 1 August 2002 (2002-08-01), pages 241-250, XP002249198 ISSN: 0001-2815
- NAKAJIMA ET AL: "Cytotoxic T lyhocytes directed against donor HLA class I antigens on airway epithelial cells are present in bronchoalveolar lavage fluid from lung transplant recipients during acute rejection" JOURNAL OF THORACIC AND CARDIOVASCULAR SURGERY, MOSBY-YEAR BOOK, INC., ST. LOUIS, MO, US, vol. 117, no. 3, 1 March 1999 (1999-03-01), pages 565-571, XP005693718 ISSN: 0022-5223
- LAKKIS FADI G ET AL: "Memory T cells: A hurdle to immunologic tolerance." JOURNAL OF THE AMERICAN SOCIETY OF NEPHROLOGY, vol. 14, no. 9, September 2003 (2003-09), pages 2402-2410, XP002493274 ISSN: 1046-6673
- HEEGER P S ET AL: "Pretransplant frequency of donor-specific, IFN-gamma-producing lymphocytes is a manifestation of immunologic memory and correlates with the risk of posttransplant rejection episodes." JOURNAL OF IMMUNOLOGY (BALTIMORE, MD. : 1950) 15 AUG 1999, vol. 163, no. 4, 15 August 1999 (1999-08-15), pages 2267-2275, XP002493275 ISSN: 0022-1767
- IACOMINI J ET AL: "Measuring T cell alloreactivity to predict kidney transplant outcomes: Are we there yet?" JOURNAL OF THE AMERICAN SOCIETY OF NEPHROLOGY 200602 US, vol. 17, no. 2, February 2006 (2006-02), pages 328-330, XP002493276 ISSN: 1046-6673

## Description

### Field of the Invention

The invention relates to a prognostic method.

### Background to the Invention

Genetic differences between molecules of the major histo-compatibility complex constitute the predominant barrier to solid organ and bone marrow transplantation. In humans these molecules are known as human leukocyte antigens (HLA). There are two major types of HLA referred to as class I and class II.

The assay of an individual's immune response to HLA can allow choices to be made with respect to the suitability of a potential transplant donor and with respect to the treatment strategy used to prevent subsequent transplant loss. The failure of a transplant is determined by various factors; for example the failure rate of first deceased donor renal transplants is approximately 3% per annum beyond the first year, but is considerably higher in re-transplants. The only currently available strategy to identify immune responses against an allogeneic transplant involve the detection of antibodies against HLA (and sometimes other molecules such as MIICA). The pre-operative detection of donor specific anti-HLA antibodies (DSA) contraindicates transplantation. The presence of anti-HLA antibodies during the post transplant course is associated with diminished graft survival and the histological identification of either acute or chronic antibody mediated rejection. The detection of anti-HLA antibodies is specific in that its presence is closely associated with an increased frequency of graft failure but it lacks sensitivity because most graft failures are not associated with anti-HLA DSA.

Additionally, a proportion of transplants result in damage of immune origin to non-graft tissue. Such damage is typically characterised as graft versus host disease (GVHD). GVHD is an important complication of allogeneic transplantation, particularly bone marrow transplantation (BMT). It can be classified into acute and chronic presentations on chronological and clinical grounds. Chronic GVHD (cGVHD) generally involves a wider range of organs than acute GVHD (aGVHD) and in many ways resembles an autoimmune disease in the untransplanted. It is currently diagnosed using clinical and histopathological criteria. As with other transplant disorders, there are some indications of an association with antibodies, in this case autoantibodies such as anti-nuclear (ANA), anti-mitochondrial (AMA), anti-smooth muscle (ASMA), anti-cardiolipin (ACLA), anti-liver-kidney microsomal (LKM), anti-DNA, anti-neutrophil cytoplasmatic (ANCA), and anti-thyroid antibodies. However, also as with other transplants, this association is not reliable in predicting or diagnosing GVHD.

There is therefore a need for alternative assays to predict transplant outcomes in general and thereby glide treatment strategies.

### Summary of the Invention

The pathogenesis of graft rejection (in particular chronic graft rejection) is poorly understood. The inability of antibody-based assays to accurately predict outcome indicates that more complex immune responses may be occurring. The invention described herein relates to findings on the immune response to peptide fragments of class I HLA. The present inventors have found that subjects exhibit T cell responses to particular antigens from various domains of the HLA class I molecule. These antigens derive from regions of the HLA molecule which are relatively conserved between HLA allotypes, for example the transmembrane and alpha-3 domains. Accordingly, by determining T cell reactivity to such antigens, there is provided a method to identify individuals at risk of undergoing graft damage or rejection of immune origin, and/or damage of immune origin to non-graft tissue. Graft rejection as referred to herein is intended to include any response by the immune system of a recipient subject to a transplanted graft or organ provided by a donor. Accordingly, the present invention provides an *in vitro* method of determining whether an individual is at risk of, or undergoing, graft damage or rejection of immune origin and/or damage of immune origin to non-graft tissue, wherein the graft is an allograft or a xenograft, the method comprising:
(a) testing whether the individual has T cells which respond to a peptide of 9 to 30 amino acids including at least one MHC class II-binding T cell epitope, said peptide comprising a sequence of contiguous amino acids from the α3 domain and/or transmembrane domain of a MHC class I molecule which is recognised by a T cell which recognises an epitope from a MHC class I molecule; and
(b) thereby determining whether the individual is at risk of, or undergoing, graft damage or rejection of immune origin and/or damage of immune origin to non-graft tissue.

### Description of the Figures

Figure 1 shows a 3D structure representation of the extracellular portion of the human MHC class I, HLA-A2, showing α1, α2, α3, and transmembrane domains, and a β2 microglobulin bound thereto.
Figure 2 shows a schematic representation of the extracellular portion of HLA-A2, showing amino acid and DNA sequences of the α1, α2, α3, and transmembrane domains.
Figure 3 shows a Table listing 53 peptides (p1 to p53) covering regions of HLA-A2, their molecular weight, and their actual sequence. The 53 peptides were used in Examples 1 and 2.
Figure 4 shows data resulting from binding affinity studies in Example 1 of the 53 peptides shown in Figure 2 to a range of purified MHC II molecules DR1, DR3, DR4, DR7, DR11, DR13, DR15, DR51-DRB5, DR52-DRB4, and DR53-DRB3
Figure 5 shows a schematic representation of the extracellular portion of the human class MHC class I, HLA-A2, and peptide p39 (residues 192-206 of HLA-A2).
Figure 6 shows a schematic representation of the extracellular portion of the human class MHC class I, HLA-A2, and peptides p50 (residues 268-282 of HLA-A2) and p51 (residues 270-284 of HLA-A2).
Figure 7 shows a schematic representation of the extracellular portion of the human class MHC class I, HLA-A2, and peptides p52 (residues 280-294 of HLA-A2) and p53 (residues 282-296 of HLA-A2).
Figure 8 shows a bar graph showing Elispot count data of reactive cells/500,000 PBMCs in a single patient for the peptides studied in Example 1.

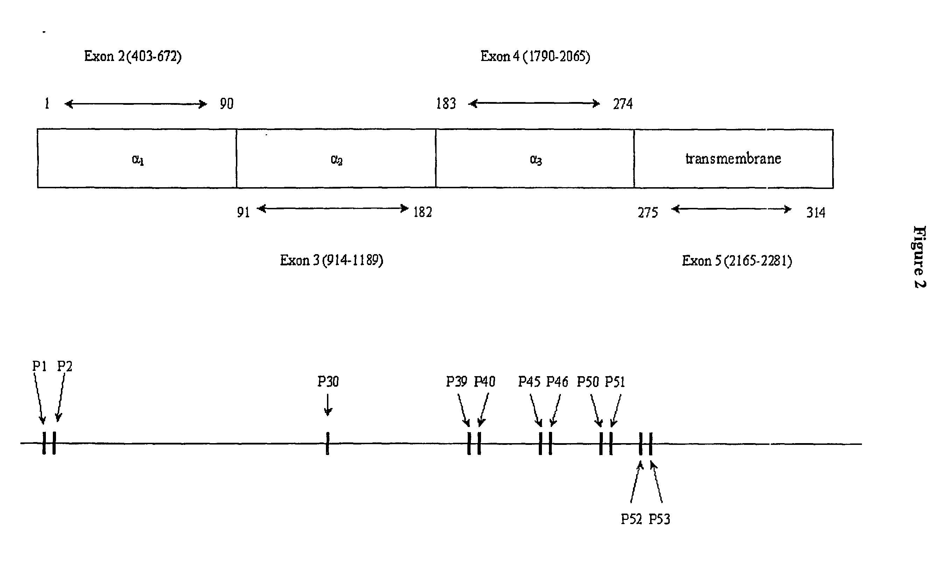

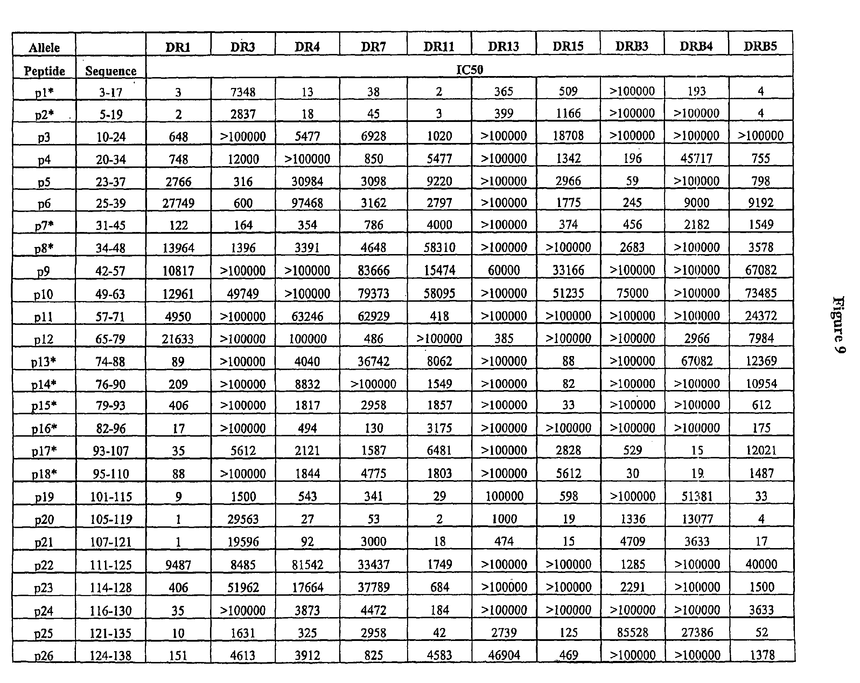

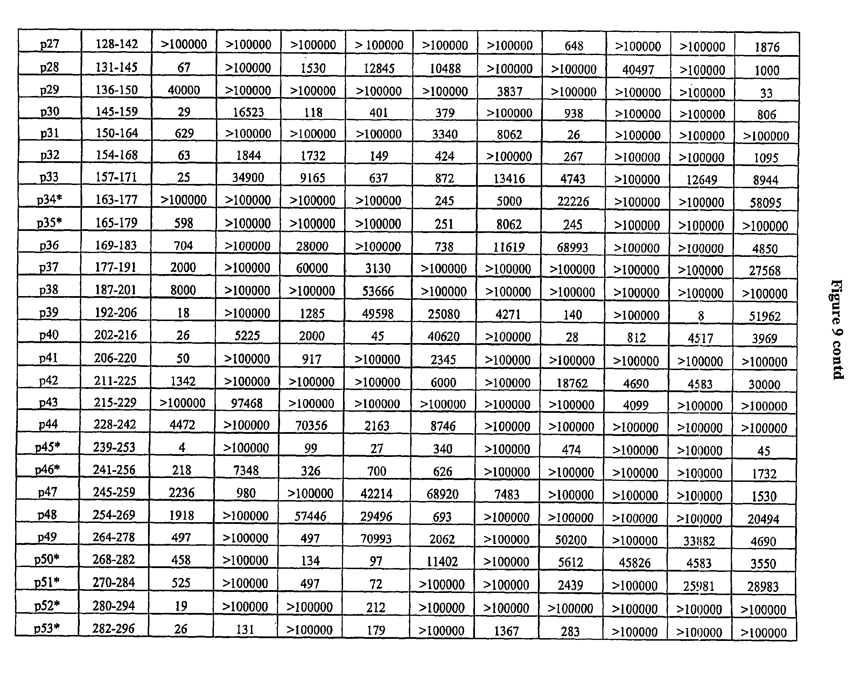

Figure 9 shows data resulting from the binding affinity studies in Example 1 of the 53 peptides shown in Figure 2 to a range of purified MHC II molecules DR1, DR3, DR4, DR7, DR11, DR13, DR15, DR51-DRB5, DR52-DRB4, and DR53-DRB3. The IC50 expressed in nM were evaluated from at least three independent experiments for each of 53 different peptides. Biotinylated reference peptides were good binders to the HLA-DR molecules and exhibited the following IC50: HA 306-318 (PKYVKQNTLKLAT) for HLA-DRB1*0101 (1 nM; pH 6), HLADRB1* 0401 (22 nM; pH 6), HLA-DRB1*1101 (19 nM; pH 5) and HLA-DRB5*0101 (8 nM; pH 5.5); YKL (AAYAAAKAAALAA) for HLADRB1*0701 (6 nM; pH 5); MT 2-16 (AKTIAYDEEARRGLE) for DRB1*0301 (303 nM; pH 4.5); B1 21-36 (TERVRLVTRHIYNREE) for HLA-DRB1*1301 (131 nM; pH 4.5); A3 152-166 (EAEQLRAYLDGTGVE) for HLA-DRB1*1501 (59 nM; pH 4.5); LOL 191-210 (ESWGAVWRIDTPDKLTGPFT) for HLA-DRB3*0101 (20 nM; pH 5.5) and E2/E168 (AGDLLAIETDKATI) for HLA-DRB4*0101 (27 nM; pH 5). * means adjacent pairs of peptides used in combination in subsequent functional assays.

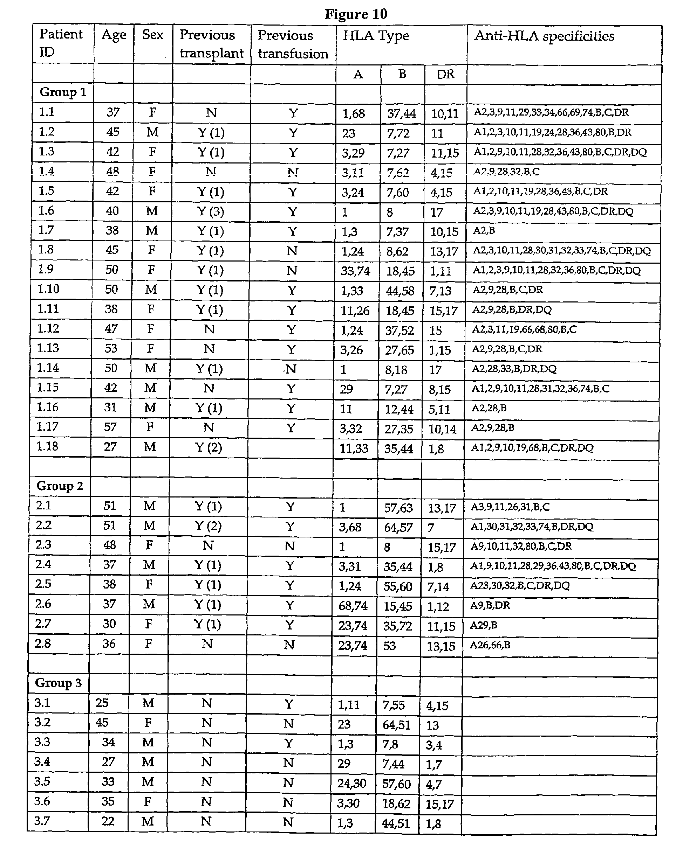

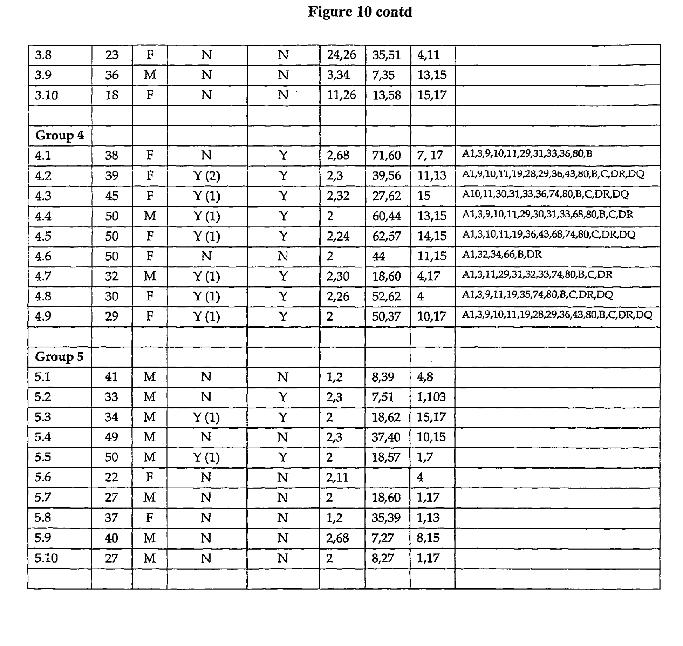

Figure 10 shows the age, sex, tissue type, transplantation, transfusion and sensitisation history of study subjects in Example 2. The different patient groups are defined on the basis of the presence of HLA-A2 in the subject and their production of antibodies to HLA-A2 or to other HLA. Group 1: HLA-A2 negative with antibodies to HLA-A2; Group 2: HLA-A2 negative with antibodies to none -A2 HLA; Group 3: HLA-A2 negative with no history of anti HLA antibody formation; Group 4: HLA-A2 positive with antibodies to none -A2 HLA; Group 5: HLA-A2 positive with no history of anti HLA antibody formation.

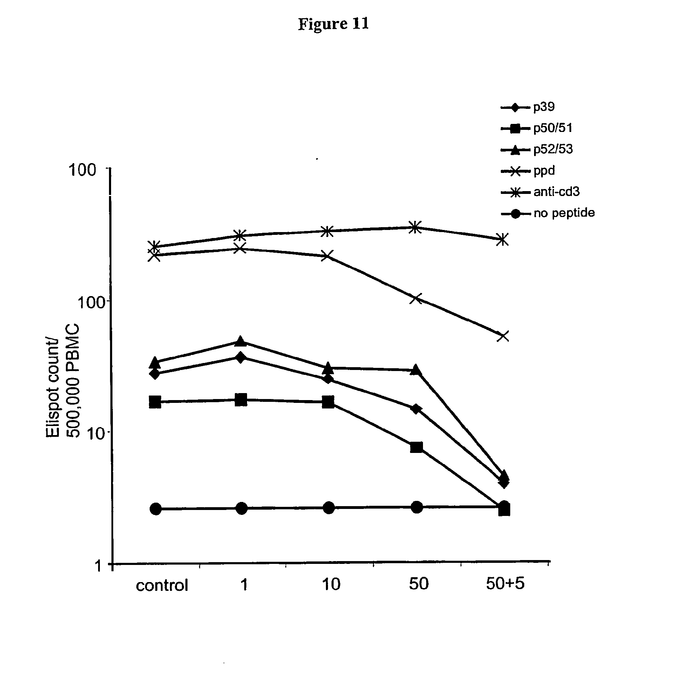

Figure 11 shows a bar graph showing Elispot count data of reactive cells/500,000 PBMCs in a single patient for the peptides studied in Example 2. The mean number of responding cells per well (5x10⁵ PBMCs) is shown for one subject cultured in the absence or presence of increasing concentrations (1, 10 & 50µgml⁻¹) of antibody against MHC class II (Tu39, Becton Dickinson, Oxford, United Kingdom). Antibody against HLA-DR (L243 Becton Dickinson) at 50µgml⁻¹ was added at the highest concentration of anti-MHC class II. PBMC's were cultured in the presence of peptide (p39, P50/51 or p52/53) at 20 µgml⁻¹, PPD at 10 µgml⁻¹, the anti-CD3 positive control supplied with the γ-interferon elispot plate (Mabtech) or medium alone.

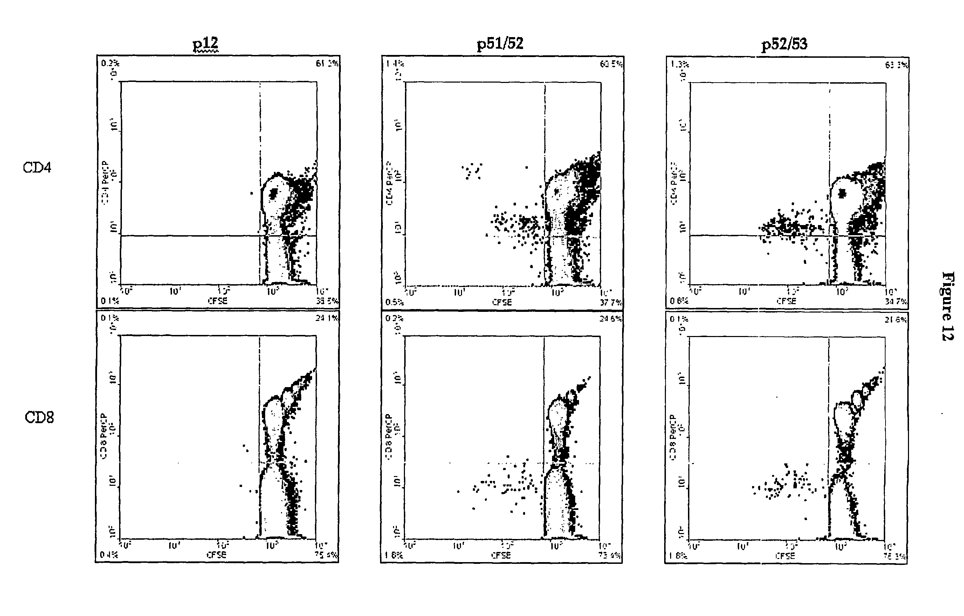

Figure 12 shows proliferation of T cells in response to some of the peptides in Figure 2. Mean γ-interferon elispot frequencies per 5x10⁵ PBMCs (y-axis) for each peptide or peptide pair (x-axis) are shown for individuals who made a response significantly greater than background. 21 different peptides or pairs of peptides were studied at a concentration of 20µgml⁻¹ (shown) or 4µgml⁻¹. Positive controls were ppd at 10µgml⁻¹ or tetanus toxoid at 1µgml⁻¹ or in some later experiments anti-CD3 supplied with the γ-interferon elispot plate (Mabtech). The HLA-DR types of the responders are shown in parentheses.
Figure 13 shows a schematic representation of the peptide p39 (residues 192-206 of HLA-A2). It also shows the difference(s) between p39 and the corresponding sequence in various other HLA-A and HLA-B alleles.
Figure 14 shows a schematic representation of the peptide p40 (residues 202-216 of HLA-A2). It also shows the difference(s) between p39 and the corresponding sequence in various other HLA-A and HLA-B alleles.
Figure 15 shows a schematic representation of the peptides p50 (residues 268-282 of HLA-A2) and p51 (residues 270-284 of HLA-A2). It also shows the difference(s) between p39 and the corresponding sequence in various other HLA-A and HLA-B alleles.
Figure 16 shows a schematic representation of the peptides p52 (residues 280-294 of HLA-A2) and p53 (residues 282-296 of HLA-A2). It also shows the difference(s) between p39 and the corresponding sequence in various other HLA-A and HLA-B alleles.
Figures 17 and 18 each show a bar graph showing Elispot count data of reactive cells in a single chronic Graft Versus Host Disease (cGVHD) individual for the peptides studied in Example 4.
Figures 19 to 21 each show a bar graph showing Elispot count data of reactive cells in a single Chronic Graft Nephropathy (CAN) individual. for the peptides studied in Example 4.
Figures 22A, B and C respectively show Elispot count data of reactive cells in normal individuals (control series P), bone marrow transplant recipients with cGVHD (patient series C) and bone marrow transplant recipients without cGVHD (patient series B), for the peptides studied in Example 4.
Figures 23A and B show a bar graph for each individual patient in series C and series B respectively, with each bar graph showing Elispot count data of reactive cells for the peptides studied in Example 4.
Figures 24A and B, C & D respectively show Elispot count data of reactive cells in the non-transplant control group, and the Green, Amber & Red patient groups derived from 100 renal transplant recipients, for the peptides studied in Example 5.

### Description of the Sequences

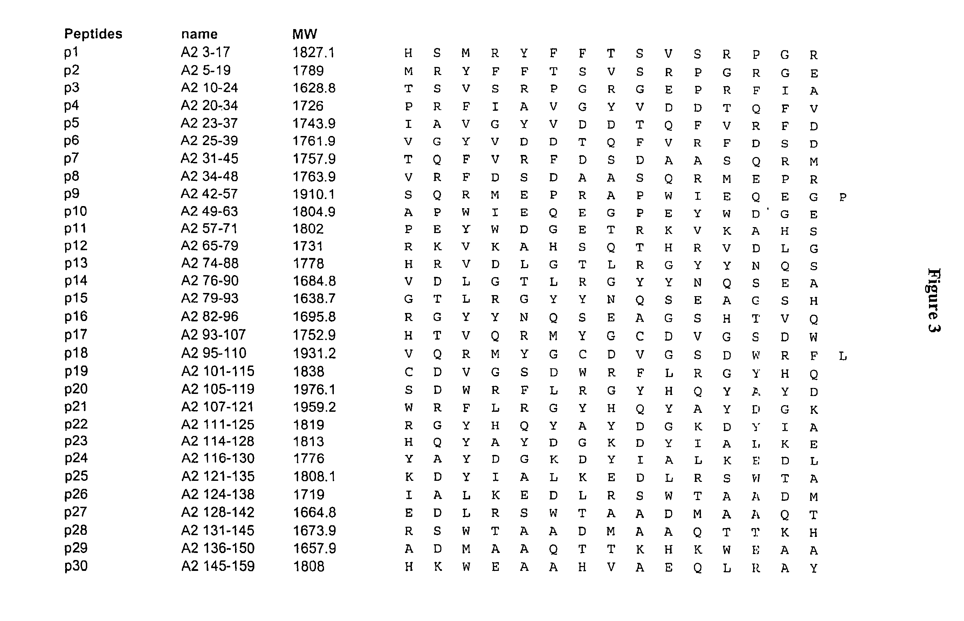

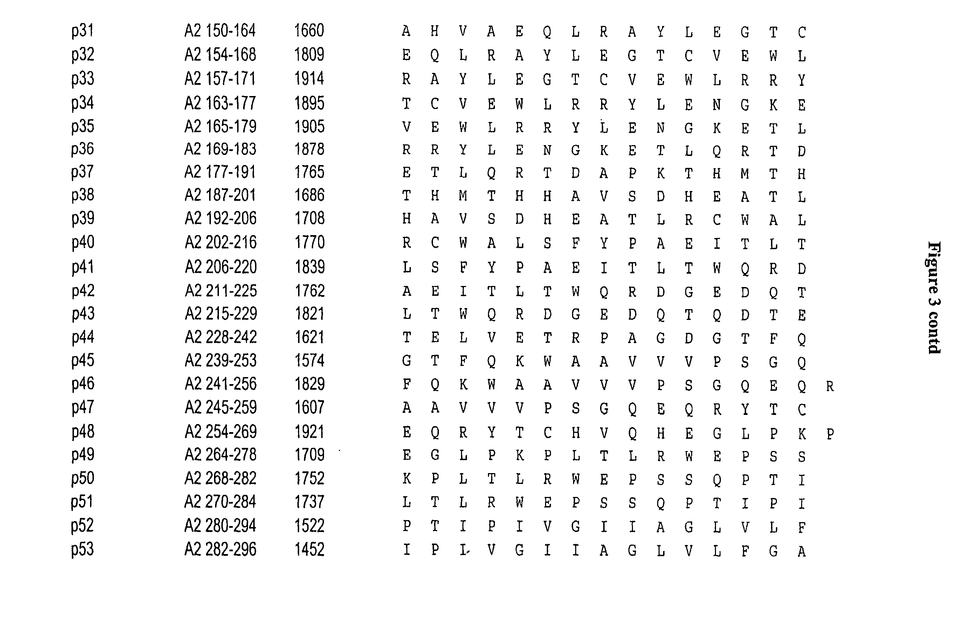

SEQ ID NO: 1 shows the full-length amino acid sequence of human MHC class I antigen, HLA-A2, (i.e. the HLA*020101 allele).
SEQ ID NO: 2 shows the mature version of the amino acid sequence of human MHC class I antigen, HLA-A2, (i.e. the HLA*020101 allele). In other words, SEQ ID NO: 2 shows the sequence of HLA-A2 without its signal sequence. SEQ ID NO: 2 corresponds to residues 25-365 of SEQ ID NO: 1.
SEQ ID NO: 3 shows the nucleic acid sequence encoding the full length human MHC class I antigen, HLA-A2, (i.e. the HLA*020101 allele) shown in SEQ ID NO: 1.
SEQ ID NO: 4 shows the amino acid sequence of p1 in Figure 3.
SEQ ID NO: 5 shows the amino acid sequence of p2 in Figure 3.
SEQ ID NO: 6 shows the amino acid sequence of p3 in Figure 3.
SEQ ID NO: 7 shows the amino acid sequence of p4 in Figure 3.
SEQ ID NO: 8 shows the amino acid sequence of p5 in Figure 3.
SEQ ID NO: 9 shows the amino acid sequence of p6 in Figure 3.
SEQ ID NO: 10 shows the amino acid sequence of p7 in Figure 3.
SEQ ID NO: 11 shows the amino acid sequence of p8 in Figure 3.
SEQ ID NO: 12 shows the amino acid sequence of p9 in Figure 3.
SEQ ID NO: 13 shows the amino acid sequence of p10 in Figure 3.
SEQ ID NO: 14 shows the amino acid sequence of p11 in Figure 3.
SEQ ID NO: 15 shows the amino acid sequence of p12 in Figure 3.
SEQ ID NO: 16 shows the amino acid sequence of p13 in Figure 3.
SEQ ID NO: 17 shows the amino acid sequence of p14 in Figure 3.
SEQ ID NO: 18 shows the amino acid sequence of p 15 in Figure 3.
SEQ ID NO: 19 shows the amino acid sequence of p16 in Figure 3.
SEQ ID NO: 20 shows the amino acid sequence of p17 in Figure 3.
SEQ ID NO: 21 shows the amino acid sequence of p18 in Figure 3.
SEQ ID NO: 22 shows the amino acid sequence of p19 in Figure 3.
SEQ ID NO: 23 shows the amino acid sequence of p20 in Figure 3.
SEQ ID NO: 24 shows the amino acid sequence of p21 in Figure 3.
SEQ ID NO: 25 shows the amino acid sequence of p22 in Figure 3.
SEQ ID NO: 26 shows the amino acid sequence of p23 in Figure 3.
SEQ ID NO: 27 shows the amino acid sequence of p24 in Figure 3.
SEQ ID NO: 28 shows the amino acid sequence of p25 in Figure 3.
SEQ ID NO: 29 shows the amino acid sequence of p26 in Figure 3.
SEQ ID NO: 30 shows the amino acid sequence of p27 in Figure 3.
SEQ ID NO: 31 shows the amino acid sequence of p28 in Figure 3.
SEQ ID NO: 32 shows the amino acid sequence of p29 in Figure 3.
SEQ ID NO: 33 shows the amino acid sequence of p30 in Figure 3.
SEQ ID NO: 34 shows the amino acid sequence of p31 in Figure 3.
SEQ ID NO: 35 shows the amino acid sequence of p32 in Figure 3.
SEQ ID NO: 36 shows the amino acid sequences of p33 in Figure 3.
SEQ ID NO: 37 shows the amino acid sequence of p34 in Figure 3.
SEQ ID NO: 38 shows the amino acid sequence of p35 in Figure 3.
SEQ ID NO: 39 shows the amino acid sequence of p36 in Figure 3.
SEQ ID NO: 40 shows the amino acid sequence of p37 in Figure 3.
SEQ ID NO: 41 shows the amino acid sequence of p38 in Figure 3.
SEQ ID NO: 42 shows the amino acid sequence of p39 in Figure 3.
SEQ ID NO: 43 shows the amino acid sequence of p40 in Figure 3.
SEQ ID NO: 44 shows the amino acid sequence of p41 in Figure 3.
SEQ ID NO: 45 shows the amino acid sequence of p42 in Figure 3.
SEQ ID NO: 46 shows the amino acid sequence of p43 in Figure 3.
SEQ ID NO: 47 shows the amino acid sequence of p44 in Figure 3.
SEQ ID NO: 48 shows the amino acid sequence of p45 in Figure 3.
SEQ ID NO: 49 shows the amino acid sequence of p46 in Figure 3.
SEQ ID NO: 50 shows the amino acid sequence of p47 in Figure 3.
SEQ ID NO: 51 shows the amino acid sequence of p48 in Figure 3.
SEQ ID NO: 52 shows the amino acid sequence of p49 in Figure 3.
SEQ ID NO: 53 shows the amino acid sequence of p50 in Figure 3.
SEQ ID NO: 54 shows the amino acid sequence of p51 in Figure 3.
SEQ ID NO: 55 shows the amino acid sequence of p52 in Figure 3.
SEQ ID NO: 56 shows the amino acid sequence of p53 in Figure 3.
SEQ ID NO: 57 shows the amino acid sequence of p50/51 (overlapping amino acids 268-282 and 270-284 SEQ ID NO: 2).
SEQ ID NO: 58 shows the amino acid sequence of p52/53 (overlapping amino acids 280-294 and 282-296 of SEQ ID NO: 2).
SEQ ID NO: 59 shows the amino acid sequence of p45/46 (overlapping amino acids 239-253 & 241-256 of SEQ ID NO: 1).
SEQ ID NO: 60 shows the amino acid sequence of p39 analogue 1.
SEQ ID NO: 61 shows the amino acid sequence of p39 analogue 2.
SEQ ID NO: 62 shows the amino acid sequence of p39 analogue 3.
SEQ ID NO: 63 shows the amino acid sequence of p40 analogue.
SEQ ID NO: 64 shows the amino acid sequence of p50/51 analogue 1.
SEQ ID NO: 65 shows the amino acid sequence of p52/53 analogue 1.
SEQ ID NO: 66 shows the amino acid sequence of p50/51 analogue 2.
SEQ ID NO: 67 shows the amino acid sequence of p50/51 analogue 3.
SEQ ID NO: 68 shows the amino acid sequence of p50/51 analogue 4.
SEQ ID NO: 69 shows the amino acid sequence of p52/53 analogue 2.
SEQ ID NO: 70 shows the amino acid sequence of p52/53 analogue 3.
SEQ ID NO: 71 shows the amino acid sequence of p52/53 analogue 4.
SEQ ID NO: 72 shows the amino acid sequence of p52/53 analogue 5.
SEQ ID NO: 73 shows the amino acid sequence of p45/46 analogue 1.
SEQ ID NO: 74 shows the amino acid sequence of p45/46 analogue 2.
SEQ ID NO: 75 shows the nucleic acid sequence encoding p1.
SEQ ID NO: 76 shows the nucleic acid sequence encoding p2.
SEQ ID NO: 77 shows the nucleic acid sequence encoding p30.
SEQ ID NO: 78 shows the nucleic acid sequence encoding p39.
SEQ ID NO: 79 shows the nucleic acid sequence encoding p40.
SEQ ID NO: 80 shows the nucleic acid sequence encoding p50.
SEQ ID NO: 81 shows the nucleic acid sequence encoding p51.
SEQ ID NO: 82 shows the nucleic acid sequence encoding p52.
SEQ ID NO: 83 shows the nucleic acid sequence encoding p53.
SEQ ID NO: 84 shows the nucleic acid sequence encoding p45.
SEQ ID NO: 85 shows the nucleic acid sequence encoding p46.
SEQ ID NO: 86 shows the nucleic acid sequence encoding p50/51
SEQ ID NO: 87 shows the nucleic acid sequence encoding p52/53
SEQ ID NO: 88 shows the nucleic acid sequence encoding p45/46
SEQ ID NO: 89 shows the nucleic acid sequence encoding p20
SEQ ID NO: 90 shows the nucleic acid sequence encoding p21.
SEQ ID NO: 91 shows the nucleic acid sequence encoding p39 analogue 1.
SEQ ID NO: 92 shows the nucleic acid sequence encoding p50/51 analogue 1.
SEQ ID NO: 93 shows the nucleic acid sequence encoding p52/53 analogue 1.
SEQ ID NO: 94 shows the nucleic acid sequence encoding p40 analogue.
SEQ ID NO: 95 shows the nucleic acid sequence encoding p45/46 analogue 1.
SEQ ID NO: 96 shows the nucleic acid sequence encoding p45/46 analogue 2.
SEQ ID NO: 97 shows the amino acid sequence of p39 (ser)
SEQ ID NO: 98 shows the amino acid sequence of p39 (ser) analogue 1.
SEQ ID NO: 99 shows the amino acid sequence of p39 (b).
SEQ ID NO: 100 shows the amino acid sequence of p39 (b) analogue 1.
SEQ ID NO: 101 shows the amino acid sequence of p40 (ser).
SEQ ID NO: 102 shows the amino acid sequence of p40 (ser) analogue 1.
SEQ ID NO: 103 shows the amino acid sequence of p50 analogue 1.
SEQ ID NO: 104 shows the amino acid sequence of p50 analogue 2.
SEQ ID NO: 105 shows the amino acid sequence of p51 analogue 1.
SEQ ID NO: 106 shows the amino acid sequence of p51 analogue 2.
SEQ ID NO: 107 shows the amino acid sequence of p52 analogue 1.
SEQ ID NO: 108 shows the amino acid sequence of p52 analogue 2.
SEQ ID NO: 109 shows the amino acid sequence of p52 analogue 3.
SEQ ID NO: 110 shows the amino acid sequence of p52 with KR attached at the N-terminus and RK attached to the C-terminus.
SEQ ID NO: 111 shows the amino acid sequence of p52 analogue 1 with KR attached at the N-terminus and RK attached to the C-terminus.
SEQ ID NO: 112 shows the amino acid sequence of p52 analogue 2 with KR attached at the N-terminus and RK attached to the C-terminus.
SEQ ID NO: 113 shows the amino acid sequence of p52 analogue 3 with KR attached at the N-terminus and RK attached to the C-terminus.
SEQ ID NO: 114 shows the amino acid sequence of p53 analogue 1.
SEQ ID NO: 115 shows the amino acid sequence of p53 analogue 2.
SEQ ID NO: 116 shows the amino acid sequence of p53 analogue 3.
SEQ ID NO: 117 shows the amino acid sequence of p53 with KR attached at the N-terminus and RK attached to the C-terminus.
SEQ ID NO: 118 shows the amino acid sequence of p53 analogue 1 with KR attached at the N-terminus and RK attached to the C-terminus.
SEQ ID NO: 119 shows the amino acid sequence of p53 analogue 2 with KR attached at the N-terminus and RK attached to the C-terminus.
SEQ ID NO. 120 shows the amino acid sequence of p53 analogue 3 with KR attached at the N-terminus and RK attached to the C-terminus.
SEQ ID NO: 121 shows the amino acid sequence of p20/21 (overlapping amino acids 105-119 and 107-121 of SEQ ID NO: 2).
SEQ ID NO: 122 shows the amino acid sequence of reference peptide HA 306-318.
SEQ ID NO: 123 shows the amino acid sequence of reference peptide YKL.
SEQ ID NO: 124 shows the amino acid sequence of reference peptide MT 2-16.
SEQ ID NO: 125 shows the amino acid sequence of reference peptide B1 21-36.
SEQ ID NO: 126 shows the amino acid sequence of reference peptide A3 152-166.
SEQ ID NO: 127 shows the amino acid sequence of reference peptide LOL 191-210.
SEQ ID NO: 128 shows the amino acid sequence of reference peptide E2/E168.
SEQ ID NO: 129 shows the amino acid sequence of reference peptide p45 analogue 1.
SEQ ID NO: 130 shows the amino acid sequence of reference peptide p45 analogue 2.
SEQ ID NO: 131 shows the amino acid sequence of reference peptide p46 analogue 1.
SEQ ID NO: 132 shows the amino acid sequence of reference peptide p46 analogue 2.

### Detailed Description of the Invention

The invention provides a method of detecting whether an individual is undergoing or is at risk of graft damage or rejection, in particular chronic graft damage or rejection, or damage of immune origin to non-graft tissue.

The individual is typically a mammal, preferably a human. The individual is typically a patient suffering from a disorder characterised by organ failure necessitating a transplant, for example, End Stage Organ Disease. By the term "organ", it is meant to include any bodily structure or group of cells containing different tissues that are organized to carry out a specific function of the body, for example, the heart, lungs, brain, kidney skin, liver, bone marrow, etc. In one embodiment the graft is an allograft, i.e. the individual and the donor are of the same species. In a further embodiment the graft is a xenograft, i.e. the individual and donor are of different species.

The individual may typically have recently suffered or be suffering from an infection, or may exhibit damaging levels of nephrotoxic drugs in their system.

The individual may exhibit one or more risk factors which are predictive of graft rejection. The main factors which increase the risk of graft dysfunction are: Previous episodes of acute rejection; Previous (failed) transplants; Presence of anti HLA antibodies (donor specific or not - although with donor specific responses the risk is greater); Non compliance with immunosuppression; Poor degree of HLA matching between donor and recipient; The transplant took place prior to the year 2002, 2000 or 1995; The donor was of a non-ideal age or condition according to standard clinical practice; The recipient is a smoker and/or has hypertension and/or hyperlipidemia; Delayed graft function; Deceased donor; Drug toxicity.

The individual may lack any or all of the above factors and/or may lack any other indicator that graft rejection is likely. For example, the individual may not have a detectable antibody response to a given MHC class I molecule, and/or lack any symptoms or disease markers associated with an immune response. Alternatively, the individual may have a detectable antibody response to HLA, in particular a donor specific response, but lack any other indicators of organ dysfunction.

The method is particularly applicable to patients who are about to receive or are predicted to require an organ transplant to predict the likelihood of immune origin graft damage or rejection, and/or immune origin damage to non-graft tissue. For example, the patient may be expected to receive a transplant in the next one, two, three, four, five, six, or twelve months. Alternatively, the assay is particularly applicable to individuals who have received a transplant to predict the likelihood of immune origin graft damage or rejection, and/or immune origin damage to non-graft tissue. Post-transplant, the assay is particularly applicable to patients who show evidence of chronic organ dysfunction (of the graft organ) or possible graft versus host disease (GVHD), particularly chronic GVHD and particularly in cases wherein the graft is a bone marrow transplant.

In the case of chronic graft dysfunction, the method can be used to stratify patients into those in whom the cause is of immune origin (that is chronic rejection) or due to other factors. This is of particular interest in renal transplantation since organ failure or dysfunction may be a consequence of toxicity of immunosuppressive agents such as calcineurin inhibitors or due to infection, for example polyoma virus infection. Alternatively it may be due to chronic rejection in which case the appropriate treatment is typically to augment immunosuppression (and at the very least not to reduce it). Existing techniques such as renal transplant biopsy discriminate between many of these cause of graft dysfunction poorly and although the presence of anti-HLA antibodies (particularly when donor specific) is specific for rejection but is insensitive (particularly for chronic rejection). The T cell based assay of the invention will enable tailoring of immunosuppression to specific individual needs.

In the case of GVHD, the assay can be used to detect whether an individual is undergoing or is at risk of damage of immune origin to non-graft tissue. This is particularly applicable to patients who have undergone transplant more than about 100 days ago, however it is also applicable to patients who have undergone transplant more than about 50, 60, 70, 80, or 90 days ago, or patients who are expected to receive a transplant in the future, for example in the next one, two, three, four, five, six, or twelve months. In this embodiment of the invention the transplant is typically a bone marrow transplant. The non-graft tissue is typically at least one selected from liver, skin, mucosa, gastrointestinal tract, lungs, eye, thymus, connective tissue, and exocrine gland

The assay of the invention can form part of a treatment regime in which, depending on the results of the assay, the treatment strategy is altered. For example, where the assay indicates that the cause of organ dysfunction / failure or non-graft tissue damage may be of immune origin, the treatment strategy can be altered by including the administration or altering the administration of immunosuppressive agents and/or tolerising the individual to the MHC class I molecule(s). Where the assay indicates an absence of an immune response, the immunosuppressive therapy may be reduced or halted.

The method of the invention concerns determining whether an individual has a T cell response to particular antigens from major histocompatibility complex (MHC) class I human leukocyte antigens (HLA). This may be determined by any suitable method, for example any method which can be used to detect the presence of antigen-experienced T cells. In one embodiment it is determined by measuring the level of T cells specific to the antigen(s). It can be determined by determining whether T cells of the individual recognise the antigen(s). A positive response by the patient's T cells to the antigens indicates that the patient is more likely to reject a transplant. A negative response indicates that the patient is less likely to reject a transplant.

The T cells which recognise the antigen in the method are generally T cells which have been pre-sensitised *in vivo* to antigen from a MHC class I molecule. These antigen-experienced T cells are generally present in the peripheral blood of a individual, i.e. within the population of peripheral blood mononuclear cells (PBMCs) in the individual. The T cells may be CD4 and/or CD8 T cells.

In the method the T cells can be contacted with the antigen *in vitro* or *in vivo,* preferably *in vitro* in a sample from the individual.

Generally the T cells which are contacted in the method are taken from the individual in a blood sample, although other types of samples which contain T cells can be used. The sample may be added directly to the assay or may be processed first. Typically the processing may comprise standard techniques such as gradient centrifugation to separate the T cells, with resuspension in any suitable volume. Alternatively, the processing may comprise diluting of the sample, for example with water, buffer or media. The sample may be diluted from 1.5 to 100 fold, for example 2 to 50 or 5 to 10 fold.

The processing may comprise separation of components of the sample. Typically mononuclear cells (MCs) are separated from the samples. The MCs will comprise the T cells and antigen presenting cells (APCs). Thus in the method the APCs present in the separated MCs can present the peptide to the T cells. In another embodiment only T cells, such as only CD4 T cells, can be purified from the sample. PBMCs, MCs and T cells can be separated from the sample using techniques known in the art.

Preferably the T cells used in the assay are in the form of unprocessed or diluted samples, are freshly isolated T cells (such as in the form of freshly isolated MCs or PBMCs) which are used directly *ex vivo,* i.e. they are not cultured before being used in the method or are thawed cells (which were previously frozen). However the T cells can be cultured before use, for example in the presence of the antigen, and generally also exogenous growth promoting cytokines. During culturing the antigen is typically present on the surface of APCs, such as the APC used in the method. Pre-culturing of the T cells may lead to an increase in the sensitivity of the method. Thus the T cells can be converted into cell lines, such as short term cell lines using techniques known in the art.

The APC which is typically present in the method may come from the same individual as the T cell or from a different individual. The APC may be a naturally occurring APC or an artificial APC. The APC is a cell which is capable of presenting the antigen to a T cell. It is typically a B-cell, dendritic cell or macrophage. It is typically separated from the same sample as the T cell and is typically co-purified with the T cell. Thus the APC may be present in MCs or PBMCs. The APC is typically a freshly isolated *ex vivo* cell or a cultured cell. It may be in the form of a cell line, such as a short term or immortalised cell line. The APC may express empty MHC class II molecules on its surface.

In one embodiment the antigen is added directly to an assay comprising T cells and APCs. As discussed above the T cells and APCs in such an assay could be in the form of MCs. When an antigen which can be recognised by the T cell without the need for presentation by APCs then APCs are not required. Analogues which mimic the original antigen bound to a MHC molecule are an example of such an antigen.

In one embodiment the antigen is provided to the APC in the absence of the T cell. The APC is then provided to the T cell, typically after being allowed to present the antigen on its surface. The antigen may have been taken up inside the APC and presented, or simply be taken up onto the surface without entering inside the APC.

Typically 10⁵ to 10⁷, preferably 2.5x10⁵ to 10⁶ PBMCs are added to each assay. In the case where peptide is added directly to the assay its concentration is from 10⁻¹ to 10³µg/ml, preferably 0.5 to 50µg/ml or 1 to 10µg/ml.

Typically the length of time for which the T cells are incubated with the antigen is from 4 to 24 hours (preferably 5 to 18 hours) for effector T cells or for more than 24 hours for central memory cells. When using *ex vivo* PBMCs it has been found that 5.0 x10⁶ PBMCs can be incubated in 10µg/ml of peptide for 5 hours at 37°C.

The antigen may be in any suitable form. The antigen generally comprises one or more T cell epitopes from a MHC class I molecule, or a variant thereof. The MHC class I molecule can be, for example, HLA-A2, HLA-A25, HLA-A26, HLA-A29, HLA-A31, HLA-A32, HLA-A33, HLA-A34, HLA-A43, HLA-A66, HLA-A68, HLA-A69, HLA-A74, HLA-A1, HLA-A3, HLA-A11, HLA-A24, HLA-A30, HLA-A36, HLA-A80, HLA-A1, HLA-A3, HLA-A11, or HLA-A23. The antigen may typically be a fragment (such as a peptide, or a variant or analogue thereof) from a MHC class I molecule. Peptides, variants and analogues suitable for use in the method of the invention will be discussed further below. The antigen may also be in the form of an analogue which mimics the epitope of the naturally occurring protein bound to a MHC molecule.

In one embodiment T cell responses to 2 or more different antigens, or 2 or more different fragments of the same antigen are investigated, for example using combinations of antigens or peptides.

In one embodiment, T cell responses are detected by determining whether or not the T cells are secreting one or more cytokines. The cytokine is typically IFN-γ or IL-2, but may be any suitable cytokine. Other cytokines include, for example, IL-12, IL-4, IL-5 and IL-10. Cytokines can typically be detected by allowing them to bind to a specific capture agent which may be immobilised on a support such as a plate, bead or the cytokine-secreting cell itself, and then measuring the presence of the specific binding agent/cytokine complex typically with a second binding detection agent. A washing step can be incorporated to remove material which is not specifically bound to the capture agent.

Typically the second agent binds the cytokine at a site which is different from the site which binds the first agent. The second agent may be directly conjugated to an enzyme such as alkaline phosphatase, or fluorescent label or may comprise a biotin moiety to be detected by a third agent comprising streptavidin, which is directly conjugated to an enzyme or fluorescent label. The conjugated enzyme then changes colour of a reagent. The agent is preferably an antibody, mono- or polyclonal. Antibodies to cytokines are commercially available, or can be made using standard techniques.

The preferred method employed to detect cytokines will be based on sandwich immunoassays detecting the frequency of cytokine-secreting cells such as colour or fluorescent ELISPOT, limiting dilution assays, intracellular cytokine staining and cytokine secretion assays with or without enrichment of cytokine-secreting cells as pioneered by Miltenyi Biotec. Alternatively, the amount of cytokine secreted can be measured for example by an ELISA based system such as the whole blood Quantiferon® system with the capture antibody immobilised on a plate, and its modifications (for example as available from Cellestis) or Luminex® suspension array technology using Beadlyte® kits with the capture antibody immobilised on a bead. Cytokine mRNA expression can also be measured with assays such as RT-PCR.

In one embodiment the detection system which is used is the *ex-vivo* ELISpot assay described in WO 98/23960. In that assay IFN-γ secreted from the T cell is bound by a first IFN-γ specific antibody which is immobilised on a solid support. The bound IFN-γ is then detected using a second IFN-γ specific antibody which is labelled with a detectable label. Such a labelled antibody can be obtained from MABTECH (Stockholm, Sweden). Other detectable labels which can be used are discussed below.

### Kits

The invention also provides a kit for carrying out the methods of the invention comprising a means for determining whether an individual has a T cell response to a polypeptide derived from a MHC class I HLA, or a variant thereof. Typically the means to detect recognition allows or aids detection based on the techniques discussed above. Thus, the means may allow detection of cytokine (such as IFN-γ and/or IL-2) secreted by the T cells after recognition. The kit may thus additionally include a specific binding agent for the cytokine, such as an antibody. The agent is typically immobilised on a solid support. This means that after binding the agent the cytokine will remain in the vicinity of the T cell that secreted it. Thus 'spots' of cytokine/agent complex are formed on the support, each spot representing a T cell which is secreting the cytokine. Quantifying the spots, and typically comparing against a control, allows determination of the relative numbers of cells that secrete the cytokine.

The kit may also comprise a means to detect the cytokine/agent complexes. A detectable change may occur in the agent itself after binding cytokine, such as a colour change. Alternatively a second agent directly or indirectly labelled for detection may be allowed to bind the cytokine /agent complex to allow the determination of the spots. As discussed above the second agent may be specific for the cytokine, but binds a different site on the cytokine than the first agent.

The immobilised support may be a plate with wells, such as a microtitre plate. Each assay can therefore be carried out in a separate well in the plate.

The kit may additionally comprise medium for the T cells, detection agents or washing buffers to be used in the detection steps. The kit may additionally comprise reagents suitable for the separation from the sample, such as the separation of PBMCs or T cells from the sample. The kit may be designed to allow detection of the T cells directly in the sample without requiring any separation of the components of the sample.

The kit may also comprise controls, such as positive or negative controls. The positive control may allow the detection system to be tested. Thus the positive control typically mimics recognition of the peptide in any of the above methods. Typically in the kits designed to determine recognition *in vitro* the positive control is a cytokine, such as IFN-γ and/or IL-2.

The kit may also comprise a means to take a sample containing T cells from the human, such as a blood sample. The kit may comprise a means to separate mononuclear cells or T cells from a sample from the individual.

### Peptides

As discussed above, the antigen is typically a fragment (such as a peptide) of a MHC class I molecule. The peptide typically comprises at least one epitope from a MHC class I molecule. The peptide may be from a naturally occurring protein which is recognised by a T cell that recognises a natural T cell epitope sequence from MHC class I. The peptide may comprise a sequence which is recognised by a T cell which recognises an epitope from a MHC class I molecule. Such sequences may have at least 70%, 80%, 90% homology to the original epitope sequence. In one embodiment the peptide comprises at least one MHC Class II-binding T cell epitope from a MHC class I molecule.

The peptide typically consists of less than 30 contiguous amino acids from a MHC class I human molecule, or a variant thereof. The peptide typically consists of less than 30 contiguous amino acids from the α3 domain and/or transmembrane domain of a MHC class I molecule, or a variant thereof. The peptides may comprise, consist of, or consist essentially of the sequences shown in any of SEQ ID NOS: 4 to 96. Variants or analogues of the above peptides may also be used.

The term "a MHC class I molecule" used herein, refers to any gene product (for example, a protein as identified as SEQ ID NO: 1), defined as a MHC class I molecule in the 14th International HLA & Immunogenetics Workshop, 2005. The gene product may comprise at least 50% identity with a gene product encoded by a human MHC class I HLA allele (for example, as identified as SEQ ID NO: 3) and/or human HLA homologues from other species, or a variant or functional fragment thereof. More preferably, the MHC class I HLA gene product has at least 60%, preferably 70%, preferably 80%, preferably 90%, preferably 95%, and most preferably 99% identity with products encoded by human MHC class I HLA alleles and/or human MHC class I HLA homologues from other species, or a variant or functional fragment thereof. The HLA may be HLA-A, HLA-B or HLA-C.

The inventor based his investigations on the most common MHC class I antigen, HLA-A2. Sequences for MHC class I antigen, HLA-A2, are known in the art, and may be found in publicly available databases, such as NCBI, e.g. at: http://www.ncbi.nlm.nih.gov/entrez/viewer.fcgi?db=nucleotid e&val=37518361

For example, the full-length protein sequence of human MHC class I antigen, HLA-A2, (i.e. the HLA*020101 allele) is identified as SEQ ID NO: 1. The mature protein sequence of human MHC class I antigen, HLA-A2, (i.e. the HLA*020101 allele) is identified as SEQ ID NO: 2. Furthermore, the nucleic acid sequence encoding human MHC class I antigen, HLA-A2, (i.e. the HLA*020101 allele) may be identified as SEQ ID NO: 3.

The nucleic acid sequence identified by SEQ ID NO: 3 comprises 8 exons, and 7 introns. These may be defined as follows:- exon 1: 200bp - 272bp; intron 1: 273bp - 402bp; exon 2: 403bp - 672bp; intron 2: 673bp - 913bp; exon 3: 914bp - 1189bp; intron 3: 1190bp - 1789bp; exon 4: 1790bp - 2065bp; intron 4: 2066bp - 2164bp; exon 5: 2165bp - 2281bp; intron 5: 2282bp - 2719bp; exon 6: 2720bp - 2752bp; intron 6: 2753bp - 2894bp; exon 7: 2895bp - 2942bp; intron 7: 2943bp - 3111bp; and exon 8: 3112bp-3116bp.

The MHC class I molecule may be HLA-A2. The term "HLA-A2" used herein, refers to any gene product (for example, a protein as identified as SEQ ID NO: 1 or 2), defined as HLA-A2 in the 14^{th} International HLA & Immunogenetics Workshop, 2005. The gene product may comprise at least 50% identity with a gene product encoded by a human HLA-A2 allele (for example, as identified as SEQ ID NO: 3) and/or human HLA-A2 homologues from other species, or a variant or functional fragment thereof. More preferably, the HLA-A2 gene product has at least 60%, preferably 70%, preferably 80%, preferably 90%, preferably 95%, and most preferably 99% identity with products encoded by human HLA-A2 alleles and/or human HLA-A2 homologues from other species, or a variant or functional fragment thereof. In preferred embodiments, the HLA has the amino acid sequence shown in SEQ ID NO: 1 or SEQ ID NO: 2.

A MHC class I HLA comprises five domains, namely α1 domain, an α2 domain, an α3 domain, a transmembrane domain and a cytosolic domain.

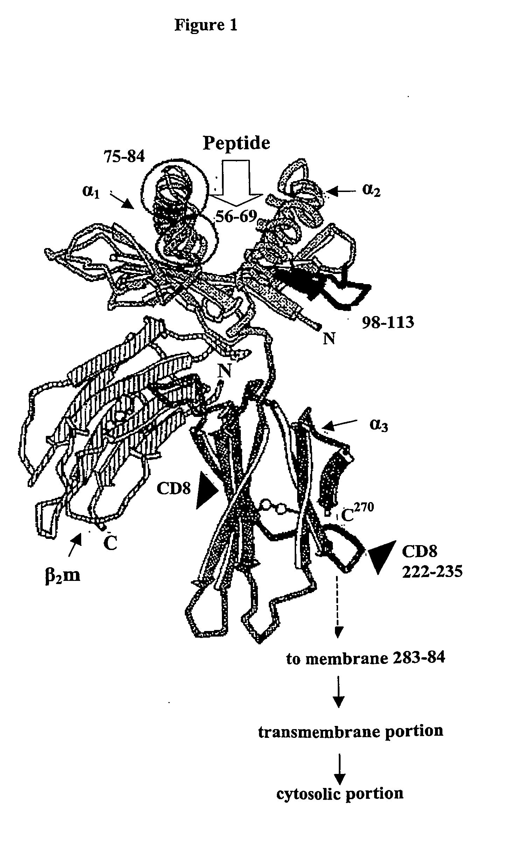

It will be appreciated from SEQ ID NO: 1 that full-length human class I histocompatability molecule, HLA-A2 protein, consists of 365 amino acid residues. It will be appreciated from SEQ ID NO: 2 that mature human class I histocompatability molecule, HLA-A2 protein, consists of 341 amino acid residues. Figure 1 shows a schematic representation of the extracellular portion of HLA-A2. It can be seen that HLA-A2 comprises an α1 domain, an α2 domain, an α3 domain, and also a transmembrane domain and a cytosolic domain, the latter two domains not being shown in the Figure. It will be appreciated that the α1 and α2 domains define a substantially polymorphic region of the HLA-A2 molecule, and that the α3 domain and the transmembrane domain define a substantially non-polymorphic region of the HLA-A2 molecule. Furthermore, a molecule of beta-2-microglobulin (β₂m) binds to the junction of the α1 and α2 domains, and to the α3 domain by non-covalent interactions. Not shown in Figure 1 is the presence of a short peptide bound non-covalently in the groove between the alpha helices of α1 and α2 domains. It will be appreciated that the combination of the peptide and adjacent portions of alpha helices makes up the epitope seen by CD8+ T cells. This discussion concerning the structure and function of the five domains in HLA-A2 also applies to other MHC class I HLAs, such as HLA-B or HLA-C.

The discussion below focuses on peptides (or variants or analogues thereof) from HLA-A2. The entire HLA-A2 molecule comprises 365 amino acids (SEQ ID NO: 1). However, such discussion also applies to other MHC class I HLAs, such as HLA-B or HLA-C. It is preferred that the peptide, variant or analogue thereof from HLA-A2 comprises less than 30 amino acids, and more preferably, less than 20 amino acids, and most preferably, about 15 amino acids.

Since T lymphocytes recognise proteins on the basis of their primary sequence as short peptides bound to MHC molecules, the method of the invention uses peptides to specifically detect T cell responses. The peptide, variant or analogue thereof from a MHC class I molecule may comprise a contiguous sequence of amino acids from the α3 domain of a MHC class I molecule; the transmembrane domain; the cytosolic domain; or any combination thereof. For example, the peptide may comprise a contiguous sequence of amino acids from the α3 domain and/or the transmembrane domain of a MHC class I molecule. Preferred peptides comprise sequence to which T cell responses have been detected in a patient. Such peptides must therefore bind to MHC class II molecules. Accordingly, preferred sequences are those which comprise at least one MHC class II-binding T cell epitope. Also disclosed are peptides from the α1 and/or α2 domains.

The method of the invention may typically use a polypeptide (or variant of analogue thereof) consisting of less than 30 contiguous amino acids from the α3 domain and/or transmembrane domain of a MHC class I molecule, or a variant thereof. In preferred embodiments, the polypeptide consists of less than 20 contiguous amino acids or about 15 contiguous amino acids from the α3 domain and/or transmembrane domain of a MHC class 1 molecule. Specific sequences preferred for use in the invention (SEQ ID NOs: 42, 43, 48, 49 and 53 to 74) are discussed in more detail below.

In HLA-A2, the α1 domain is encoded by exon 2, i.e. nucleotides 403-672 of SEQ ID NO: 3. Hence, polypeptides are disclosed which comprise less than 30 contiguous amino acids from the amino acid sequence defined as residues 1-90 of SEQ ID NO: 2, i.e. the residues encoded by nucleotides 403-672 of SEQ ID NO: 3. The α2 domain is encoded by exon 3, i.e. nucleotides 914-1189 of SEQ ID NO: 3. Hence, polypeptides are disclosed which comprise less than 30 contiguous amino acids from the amino acid sequence defined as residues 91-182 of SEQ ID NO: 2, i.e. the residues encoded by nucleotides 914-1189 of SEQ ID NO: 3.

The α3 domain is encoded by exon 4, i.e. nucleotides 1790-2065 of SEQ ID NO: 3. Hence, the polypeptide of the invention may comprise less than 30 contiguous amino acids from the amino acid sequence defined as residues 183-274 of SEQ ID NO: 2, i.e. the residues encoded by nucleotides 1790-2065 of SEQ ID NO: 3. The transmembrane domain is encoded by exon 5, i.e. nucleotides 2165-2281 of SEQ ID NO: 3. Hence, the polypeptide of the invention may comprise less than 30 contiguous amino acids from the amino acid sequence defined as residues 275-314 of SEQ ID NO: 2, i.e. the residues encoded by nucleotides 2165-2281 of SEQ ID NO: 3.

The results of the binding studies discussed the Examples 1 and 2 revealed a significant number of polypeptides that bind relatively 'promiscuously' to MHC class II, i.e. peptide sequences which bind to multiple MHC class II types. Some of the peptides are derived from the hypervariable or polymorphic region of HLA-A2 (i.e. the α1 & α2 domains). For example, peptide p20 (SEQ ID NO: 23) is derived from amino acid residues 105-119 of HLA-A2 (SEQ ID NO: 2), and peptide p21 (SEQ ID NO: 24) is derived from residues 107-121). Both peptides correspond to peptide that has been eluted from DR 1. A positive response was observed in 10 out of 15 patients who had made anti-HLA-A2. Hence, a single 17 amino acid peptide that binds promiscuously to MHC class II molecules accounts for much of the immunogenicity in this region whatever the MHC class II.

Accordingly, a single (or two closely overlapping peptides) may be used to detect a high proportion of potential T cell immune responses to MHC class I molecules. Some polypeptides disclosed herein derive from a substantially polymorphic region of a MHC class I HLA molecule, for example, from the α1 and/or α2 domain thereof. Hence, polypeptides disclosed herein comprise substantially the amino acid sequence:
(a) HSMRYFFTSVSRPGR (SEQ ID NO: 4). This peptide corresponds to amino acids 3-17 of HLA-A2 protein (i.e. SEQ ID NO: 2), and is derived from the α1 domain of the HLA-A2 molecule. This peptide is designated p1 when referred to herein, and has a molecular weight of 1827.1.
(b) MRYFFTSVSRPGRGE (SEQ ID NO: 5). This peptide corresponds to amino acids 5-19 of HLA-A2 protein (i.e. SEQ ID NO: 2), and is derived from the α1 domain of the HLA-A2 molecule. This peptide is designated p2 when referred to herein, and has a molecular weight of 1789.
(c) HKWEAAHVAEQLRAY (SEQ ID NO: 33). This peptide corresponds to amino acids 145-159 of HLA-A2 protein (i.e. SEQ ID NO: 2), and is derived from the α2 domain of the HLA-A2 molecule. This peptide is designated p30 when referred to herein, and has a molecular weight of 1808.
(d) SDWRFLRGYHQYAYD (SEQ ID NO: 23). This peptide corresponds to amino acids 105-119 of HLA-A2 protein (i.e. SEQ ID NO: 2), and is derived from the α2 domain of the HLA-A2 molecule. This peptide is designated p20 when referred to herein, and has a molecular weight of 1976.1.
(e) WRFLRGYHQYAYDGK (SEQ ID NO: 24). This peptide corresponds to amino acids 107-121 of HLA-A2 protein (i.e. SEQ ID NO: 2), and is derived from the α2 domain of the HLA-A2 molecule. This peptide is designated p21 when referred to herein, and has a molecular weight of 1959.2.

It should be appreciated that each of the peptides (a) to (e) are derived from the polymorphic region of HLA-A2. However, a significant number of individuals also respond to peptides derived from elsewhere in the HLA-A2 molecule, and in particular, regions of HLA-A2 that are of limited polymorphism, for example, the α3 and transmembrane domains. Such regions of the MHC class I molecule have not previously been reported to be recognised in T cell immune responses. Because they are of limited polymorphism, these peptides act as sites of potential cross-reactivity between different HLA molecules. Accordingly, peptides from these regions are preferred for use in the method of the invention since a response to these molecules suggests that a patient is more likely to initiate an immune response against a graft, regardless of graft allotype.

Accordingly, preferred polypeptides, variants or analogues thereof for use in the method of the invention are derived from a substantially limited or non-polymorphic region of the MHC class I HLA molecule, such as the α3 and/or transmembrane domain. Therefore, most preferred peptides used according to the invention comprise substantially the amino acid sequence:
(f) HAVSDHEATLRCWAL (SEQ ID NO: 42). This peptide corresponds to amino acids 192-206 of HLA-A2 protein (i.e. SEQ ID NO: 2), and is derived from the α3 domain of the HLA-A2 molecule. This peptide is designated p39 when referred to herein, and has a molecular weight of 1708.
(g) RCWALSFYPAEITLT (SEQ ID NO: 43). This peptide corresponds to amino acids 202-216 of HLA-A2 protein (i.e. SEQ ID NO: 2), and is derived from the α3 domain of the HLA-A2 molecule. This peptide is designated p40 when referred to herein, and has a molecular weight of 1770.
(h) KPLTLRWEPSSQPTI (SEQ ID NO: 53). This peptide corresponds to amino acids 268-282 of HLA-A2 protein (i.e. SEQ ID NO: 2), and is derived from the α3 domain and the transmembrane domain of the HLA-A2 molecule. This peptide is designated p50 when referred to herein, and has a molecular weight of 1752.
(i) LTLRWEPSSQPTIPI (SEQ ID NO: 54). This peptide corresponds to amino acids 270-284 of HLA-A2 protein (i.e. SEQ ID NO: 2), and is derived from the α3 domain and the transmembrane domain of the HLA-A2 molecule. This peptide is designated p51 when referred to herein, and has a molecular weight of 1737.
(j) PTIPIVGIIAGLVLF (SEQ ID NO: 55). This peptide corresponds to amino acids 280-294 of HLA-A2 protein (i.e. SEQ ID NO: 2), and is derived from the transmembrane domain of the HLA-A2 molecule. This peptide is designated p52 when referred to herein, and has a molecular weight of 1522.
(k) IPIVGIIAGLVLFGA (SEQ ID NO: 56). This peptide corresponds to amino acids 282-296 of HLA-A2 protein (i.e. SEQ ID NO: 2), and is derived from the transmembrane domain of the HLA-A2 molecule. This peptide is designated p53 when referred to herein, and has a molecular weight of 1452.
(1) GTFQKWAAVVVPSGQEQR (SEQ ID NO: 48). This peptide corresponds to amino acids 239-253 of HLA-A2 protein (i.e. SEQ ID NO: 2), and is derived from the α3 domain of the HLA-A2 molecule. This peptide is designated p45 when referred to herein, and has a molecular weight of 1574.
(m) FQKWAAVVVPSGQEQR (SEQ ID NO: 49). This peptide corresponds to amino acids 241-256 of HLA-A2 protein (i.e. SEQ ID NO: 2), and is derived from the α3 domain of the HLA-A2 molecule. This peptide is designated p46 when referred to herein, and has a molecular weight of 1829.

It should be appreciated that each of the peptides (f) to (m) are derived from the substantially non-polymorphic region of HLA-A2. It is shown here that such peptides derived from the non-polymorphic region (i.e. α3 and transmembrane domains) of HLA-A2 show a surprisingly high frequency of response in patients that have made antibody to HLA-A2 and significant responses in some others.

Polypeptides, variants or analogues, which comprise overlapping regions of any of the preferred peptides disclosed herein are also recognised by T cells from individuals, and are therefore also useful for detecting T cell response to the MHC class I molecules. Polypeptides, variants or analogues thereof derived from the non-polymorphic regions of a MHC class I HLA, such as HLA-A2, may comprise overlapping sections or regions. Hence, further polypeptides used according to the invention comprise substantially the amino acid sequence:
(n) KPLTLRWEPSSQPTIPI (SEQ ID NO: 57). This peptide corresponds to overlapping amino acids 268-282 and 270-284 of HLA-A2 protein (i.e. SEQ ID NO: 2). This peptide is designated p50/51 when referred to herein.
(o) PTIPIVGIIAGLVLFGA (SEQ ID NO: 58). This peptide corresponds to overlapping amino acids 280-294 and 282-296 of HLA-A2 protein (i.e. SEQ ID NO: 2). This peptide is designated p52/53 when referred to herein.
(p) GTFQKWAAVVVPSGQEQR (SEQ ID NO: 59). This peptide corresponds to overlapping amino acids 239-253 & 241-256 of HLA-A2 protein (i.e. SEQ ID NO: 2). This peptide is designated p45/46 when referred to herein.

Also disclosed herein is a polypeptide comprising substantially the amino acid sequence:
(q) SDWRFLRGYHQYAYDGK (SEQ ID NO: 121). This peptide corresponds to overlapping amino acids 105-119 & 107-121 of HLA-A2 protein (i.e. SEQ ID NO: 2). This peptide is designated p20/21 when referred to herein.

By analogy with anti-HLA antibodies, preferred peptides derived from the α3 and transmembrane domains could be referred to as 'public' T cell epitopes. This is relevant to the evolution of spreading immune responses to different HLA, and in designing peptides or mixtures useful for detecting T cell immune responses to MHC class I molecules.

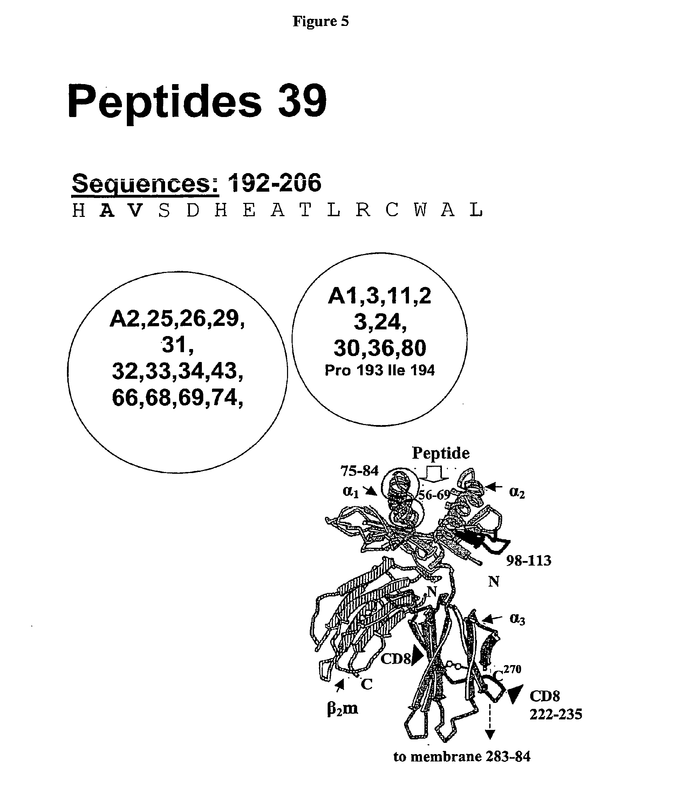

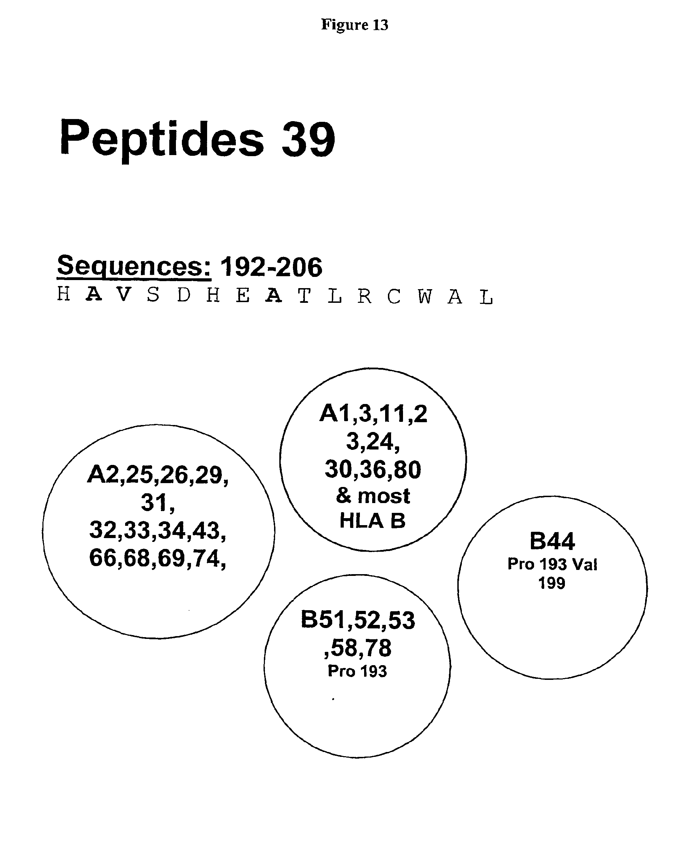

By way of example, as shown in Figures 5 and 13, the sequence of peptide p39, i.e. SEQ ID NO: 42, which is derived from amino acids 192-206 of HLA-A2, is also present in the sequence of the following HLA molecules: HLA-A2, HLA-A25, HLA-A26, HLA-A29, HLA-A31, HLA-A32, HLA-A33, HLA-A34, HLA-A43, HLA-A66, HLA-A68, HLA-A69, and HLA-A74. Therefore, detection of a T cell response to peptide p39 (or a variant or analogue thereof) indicates that an immune response to any of these HLA molecules is also likely. This in turn is predictive of rejection of a graft expressing a wide range of HLA molecules.

Furthermore, similarly, there is only a single variant of the sequence of p39 (SEQ ID NO: 42) expressed by all other HLA-A molecules, HLA-A1, HLA-A3, HLA-A11, HLA-A23, HLA-A24, HLA-A30, HLA-A36, HLA-A80, and most HLA-B molecules, as indicated in Figures 5 and 13. This variant is called p39 analogue 1 and is shown in SEQ ID NO: 60. Accordingly, testing for a T cell response to peptide p39 and p39 analogue 1 (in which proline replaces alanine at residue 193 and isoleucine replaces valine at residue 194), provides a combination which can predict an immune response to a transplant bearing any HLA-A molecule and most HLA-B molecules.

Furthermore, similarly, there is a single variant of the sequence of p39 (SEQ ID NO: 42) expressed by other HLA-B molecules, HLA-B51, HLA-B52, HLA-B53, HLA-B58, HLA-B78 as indicated in Figure 13. This variant is called p39 analogue 2 and is shown in SEQ ID NO: 61. Accordingly, testing for a T cell response to peptide p39 and p39 analogue 2 (in which proline replaces alanine at residue 193), provides a combination which can predict an immune response to a transplant bearing a wide range of HLA-A molecules and some HLA-B molecules.

Furthermore, similarly, there is a single variant of the sequence of p39 (SEQ ID NO: 42) expressed by HLA-B44 as indicated in Figure 13. This variant is called p39 analogue 3 and is shown in SEQ ID NO: 62. Accordingly, testing for a T cell response to peptide p39 and p39 analogue 3 (in which proline replaces alanine at residue 193 and valine replaces alanine at residue 199), provides a combination which can predict an immune response to a transplant bearing a wide range of HLA-A molecules as well as HLA-B44.

Other p39 variants include p39 (ser) (SEQ ID NO: 97), in which serine replaces cysteine at residue 203, p39 analogue 1 (ser) (SEQ ID NO: 98), in which proline replaces alanine at residue 193, isoleucine replace valine at residue 194, and serine replaces cysteine at residue 203, p39 (b) (SEQ ID NO: 99), in which 2-aminobutyric acid replaces cysteine at residue 203, and p39 analogue 1 (b) (SEQ ID NO: 100), in which proline replaces alanine at residue 193, isoleucine replace valine at residue 194, and 2-aminobutyric acid replaces cysteine at residue 203.

By way of a further example, as shown in Figure 13, the sequence of peptide p40, i.e. SEQ ID NO: 43, which is derived from amino acids 202-216 of HLA-A2, is also present in the sequence of the following HLA molecules: HLA-A2, HLA-A25, HLA-A26, HLA-A29, HLA-A31, HLA-A32, HLA-A33, HLA-A34, HLA-A43, HLA-A66, HLA-A68, HLA-A69, HLA-A74, and HLA-A74. Therefore detection of a T cell response to peptide p40 (or a variant or analogue thereof) indicates that an immune response to any of these HLA molecules is also likely. This in turn is predictive of rejection of a graft expressing a wide range of HLA molecules.

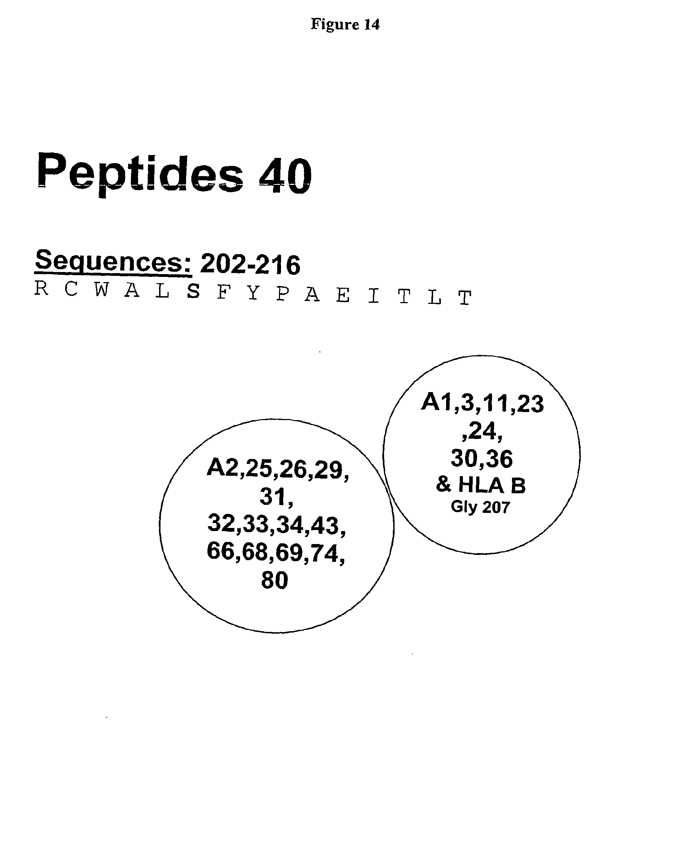

Furthermore, similarly, there is a single variant of the sequence of p40 (SEQ ID NO: 43) expressed by other HLA-A molecules, HLA-A1, HLA-A3, HLA-A11, HLA-A23, HLA-A24, HLA-A30, HLA-A36, as well as most HLA-B molecules, as indicated in Figure 14. This variant is called p40 analogue and is shown in SEQ ID NO: 63. Accordingly, testing for a T cell response to peptide p40 and p40 analogue (in which glycine replaces serine at residue 207), provides a combination which can predict an immune response to a transplant bearing a wide range of HLA-A molecules and HLA-B molecules.

Other p40 variants include p40 (ser) (SEQ ID NO: 101), in which serine replaces cysteine at residue 203, and p40 analogue 1 (ser) (SEQ ID NO: 102), in which serine replaces cysteine at residue 203 and glycine replaces serine at residue 207.

Furthermore, there are variants of the sequence of p45 including p45 analogue 1 (SEQ ID NO: 129) in which Glu replaces Gln at residue 253, and p45 analogue 2 (SEQ ID NO: 130) in which Ser replaces Ala at residue 246.

Furthermore, there are variants of the sequence of p46 including p46 analogue 1 (SEQ ID NO: 131) in which Glu replaces Gln at residue 253, and p46 analogue 2 (SEQ ID NO: 132) in which Ser replaces Ala at residue 246.

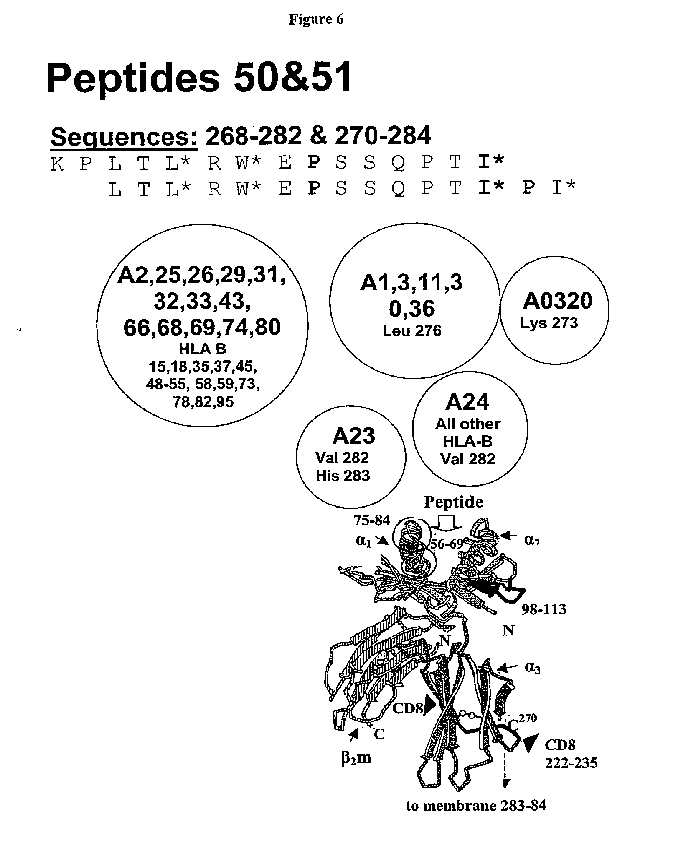

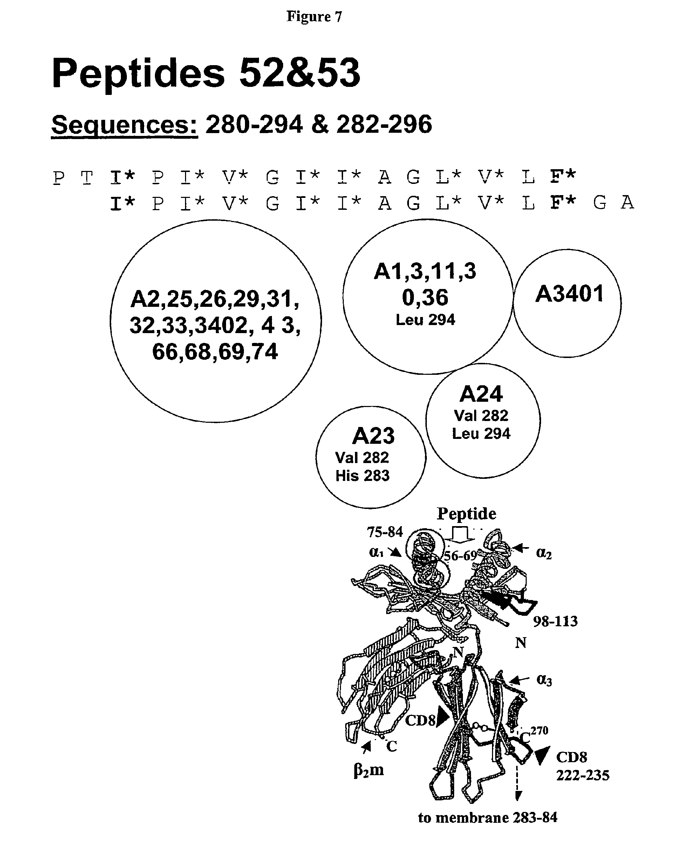

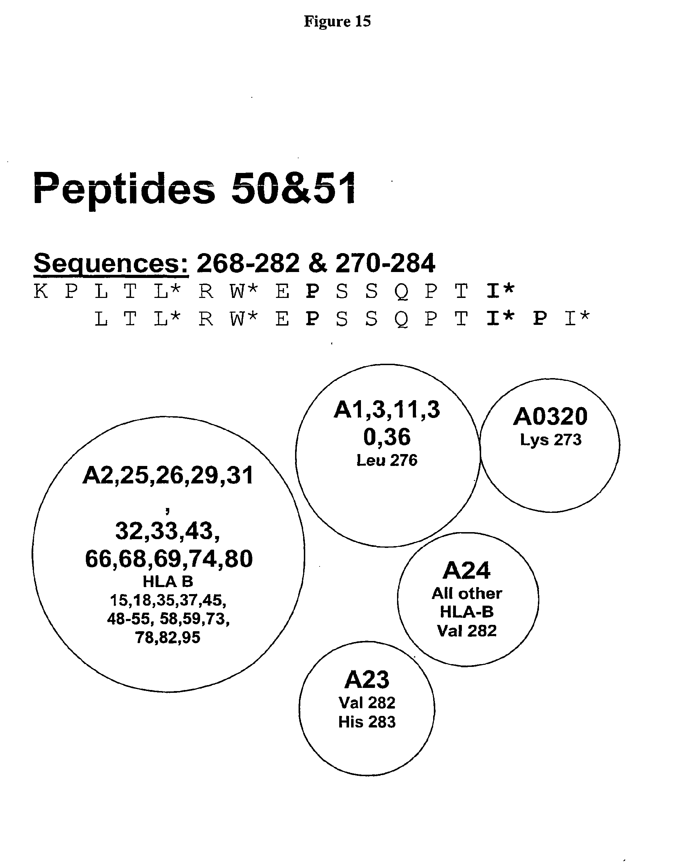

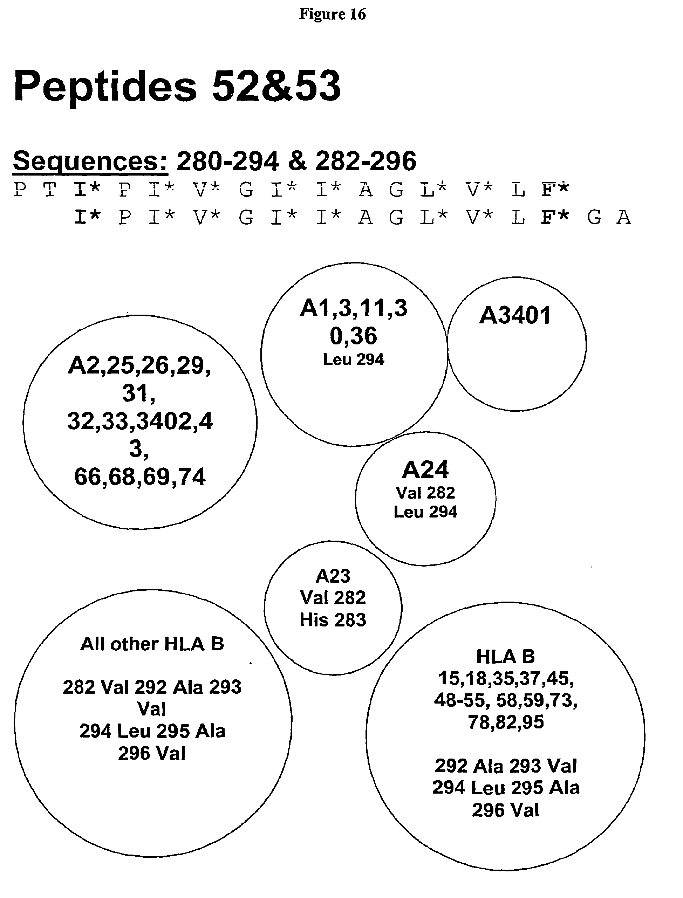

Furthermore, as illustrated in Figures 6 and 15, peptide p50 (SEQ ID NO: 53), which is derived from amino acids 268-282, and peptide p51 (SEQ ID NO: 54), which is derived from amino acids 270-284, comprise sequences that are offset by only 2 amino acids and therefore span a 17 amino acid stretch. Similarly, as illustrated in Figures 7 and 16, peptide p52 (SEQ ID NO: 55), which is derived from amino acids 280-294, and peptide p53 (SEQ ID NO: 56), which is derived from amino acids 282-296, comprise sequences that are offset by only 2 amino acids and therefore span a 17 amino acid stretch. In both cases, the identified peptides are not only present in HLA-A2, but also in HLA-A25, HLA-A26, HLA-A29, HLA-A31, HLA-A32, HLA-A33, HLA-A43, HLA-A66, HLA-A68, HLA-A69, HLA-A74, and HLA-A80. p51 (SEQ ID NO: 54) and p52 (SEQ ID NO: 55) are also present in HLA-B15, HLA-B18, HLA-B35, HLA-B37, HLA-B45, HLA-B48, HLA-B49, HLA-B50, HLA-B51, HLA-B52, HLA-B53, HLA-B54, HLA-B55, HLA-B58, HLA-B59, HLA-B73, HLA-B78, HLA-B82 and HLA-B95 (Figure 14).

Additionally, analogues of these two sequences in which a leucine is substituted for a proline at residue 276 (for the 17mer 268-284; SEQ ID NO: 64) or a leucine is substituted for a phenylanine at 294 (for the 17mer 280-296; SEQ ID NO: 65), would provide peptide sequences that would be expressed by the majority of HLA-A molecules in a way analogous to that described for p39, the only substantial omissions being HLA-A23 & HLA-A24.

p50 variants include p50 analogue 1 (SEQ ID NO: 103), in which leucine replaces proline at residue 276, and p50 analogue 2 (SEQ ID NO: 104), in which leucine replaces proline at residue 276 and valine replaces isoleucine at residue 282.

p51 variants include p51 analogue 1 (SEQ ID NO: 105), in which leucine replaces proline at residue 276, and p51 analogue 2 (SEQ ID NO: 106), in which valine replaces isoleucine at residue 282.

p52 variants include p52 analogue 1 (SEQ ID NO: 107), in which leucine replaces phenylalanine at residue 294, p52 analogue 2 (SEQ ID NO: 108), in which alanine replaces valine at residue 292, valine replaces leucine at residue 293, and leucine replaces phenylalanine at residue 294, and p52 analogue 3 (SEQ ID NO: 109), in which valine replaces isoleucine at residue 282, alanine replaces valine at residue 292, valine replaces leucine at residue 293, and leucine replaces phenylalanine at residue 294. The variants p52 rk (SEQ ID NO: 110), p52 analogue 1 rk (SEQ ID NO: 111), p52 analogue 2 rk (SEQ ID NO: 112), and p52 analogue 3 rk (SEQ ID NO: 113), respectively correspond to the peptides of SEQ ID NOs: 55 and 107 to 109 with KR attached at the N-terminus and RK attached to the C-terminus.

p53 variants include p53 analogue 1 (SEQ ID NO: 114), in which leucine replaces phenylalanine at residue 294, p53 analogue 2 (SEQ ID NO: 115), in which alanine replaces valine at residue 292, valine replaces leucine at residue 293, leucine replaces phenylalanine at residue 294, alanine replaces glycine at residue 295, and valine replaces alanine at residue 296, and p53 analogue 3 (SEQ ID NO: 116), in which valine replaces isoleucine at residue 282, alanine replaces valine at residue 292, valine replaces leucine at residue 293, leucine replaces phenylalanine at residue 294, alanine replaces glycine at residue 295, and valine replaces alanine at residue 296. The variants p53 rk (SEQ ID NO: 117), p53 analogue 1 rk (SEQ ID NO: 118), p53 analogue 2 rk (SEQ ID NO: 119), and p53 analogue 3 rk (SEQ ID NO: 120), respectively correspond to the peptides of SEQ ID NOs: 56 and 114 to 116 with KR attached at the N-terminus and RK attached to the C-terminus.

Other analogues of p50/51 and p52/53 are described in the Table 1 below.

**Table 1 - Additional analogues of p50/51 and p52/53**

| **SEQ ID NO:** | **Analogue** | **Change(s) to arrive at the analogue** | **Present in HLA** |
|---|---|---|---|
| 66 | p50/51 analogue 2 | Lysine at 273 | A0320 |
| 67 | p50/51 analogue 3 | Valine at 282 | A24 |
| | | | All HLA-B in which p50 is not present |
| 68 | p50/51 analogue 4 | Valine at 282 | A23 |
| | | Histidine at 283 | |
| 69 | p52/53 analogue 2 | Valine at 282 | A24 |
| | | Leucine at 294 | |
| 70 | p52/53 analogue 3 | Valine at 282 | A23 |
| | | Histidine at 283 | |
| 71 | p52/53 analogue 4 | Alanine at 292 | HLA-B 15, 18, 35, 37, 45, 48-55, 58, 59, 73, 78,82,95 |
| | | Valine at 293 | |
| | | Leucine at 294 | |
| | | Alanine at 295 | |
| | | Valine at 296 | |
| 72 | p52/53 analogue 5 | Valine at 282 | All other HLA-B |
| | | Alanine at 292 | |
| | | Valine at 293 | |
| | | Leucine at 294 | |
| | | Alanine at 295 | |
| | | Valine at 296 | |

Therefore, testing for a T cell response to a combination of polypeptides p50, p51, 52, and p53, or variants or analogues thereof, provides a combination which can predict an immune response to a transplant bearing any of these HLA molecules.

As will be apparent, testing for a T cell response to specific combinations of p39, p40, p50, p51, p50/51, p52, p53 and/or p52/53 and one or more of their variants discussed above can predict an immune response. This has the advantage of using as few polypeptides as possible to predict immune responses to a transplant bearing any HLA-A or HLA-B molecule.

Hence, it will be appreciated from the foregoing that preferred polypeptides, variants or analogues thereof which are derived from the non-polymorphic region of HLA-A2 are also expressed by many other HLA-A molecules, and not just HLA-A2, by acting as sites of potential cross-reactivity between different HLA molecules. Hence, the benefits of using such peptides in methods according to the invention, and in particular for uses in predicting graft rejection, may be much wider than to HLA-A2 antigen alone.

The above indications are significant because they demonstrate that a simple T cell assay with only a small number of peptides can detect immune responses to a wide range of different MHC class I molecules, due to the cross-reactivity of certain T cell epitopes. Accordingly, preferred polypeptides derived from the MHC class I molecule may include p39 (SEQ ID NO: 42), p50/51 (SEQ ID NO: 57), p52/53 (SEQ ID NO: 58), p50 (SEQ ID NO: 53), p51 (SEQ ID NO: 54), 52 (SEQ ID NO: 55), and p53 (SEQ ID NO: 56), or variants or analogues thereof, or any combination thereof. It will be appreciated that these polypeptides are all derived from HLA-A2. Corresponding polypeptides from other MHC class I antigens, such as HLA-B and HLA-C, can also be used in the method of the invention.

Some suitable variants of the preferred polypeptides described above are shown in the following:

| | |
|---|---|
| p39 (SEQ ID NO: 42) | HAVSDHEATLRCWAL |
| (q) p39 analogue 1 (SEQ ID NO: 60) | HPISDHEATLRCWAL |
| | |
| p50 (SEQ ID NO: 53) | KPLTLRWEPSSQPTI |
| p51 (SEQ ID NO: 54) | LTLRWEPSSQPTIPI |
| (r) analogue 1 17mer (SEQ ID NO: 64) | KPLTLRWELSSQPTIPI |
| | |
| p52 (SEQ ID NO: 55) | PTIPIVGIIAGLVLF |
| p53 (SEQ ID NO: 56) | IPIVGIIAGLVLFGA |
| (s) analogue 1 17mer (SEQ ID NO: 65) | PTIPIVGIIAGLVLLGA |
| p40 (SEQ ID NO: 43) | RCWALSFYPAEITLT |
| (t) p40 analogue (SEQ ID NO: 63) | RCWALGFYPAEITLT |
| | |
| p45/46 (SEQ ID NO: 59) | GTFQKWAAVVVPSGQEQR |
| (u) p45/46 analogue 1 (SEQ ID NO: 73) | GTFQKWAAVVVPSGEEQR |
| | |
| (v) p45/46 analogue 2 (SEQ ID NO: 74) | GTFQKWASVVVPSGQEQR |

Hence, preferred variants used according to the invention may comprise substantially the amino acid sequence:
(q) HPISDHEATLRCWAL (SEQ ID NO: 60). This analogue is derived from peptide p39 (SEQ ID NO: 42), and is derived from the α3 domain of the HLA-A2 molecule, except that the alanine residue is replaced with a proline residue, and the valine residue is replaced with an isoleucine residue. This analogue is designated "p39 analogue 1" when referred to herein.
(r) KPLTLRWELSSQPTIPI (SEQ ID NO: 64). This analogue is derived from peptides p50 (SEQ ID NO: 53) and p51 (SEQ ID NO: 54), and is derived from the α3 domain and the transmembrane domain of the HLA-A2 molecule, except that the proline residue at position 276 is replaced with a leucine residue. This analogue is designated "p50/p51 analogue 1" when referred to herein.
(s) PTIPIVGIIAGLVLLGA (SEQ ID NO: 65). This analogue is derived from peptides p52 (SEQ ID NO: 55) and p53 (SEQ ID NO: 56), and is derived from the transmembrane domain of the HLA-A2 molecule, except that the phenylalanine residue at 294 is replaced with a leucine residue. This analogue is designated "p52/53 analogue 1" when referred to herein.
(t) RCWALGFYPAEITLT (SEQ ID NO: 63). This analogue is derived from peptide p40 (i.e. SEQ ID NO: 43), and is derived from the α3 domain of the HLA-A2 molecule, except that the serine residue at position 207 is replaced with a glycine residue. This analogue is designated "p40 analogue" when referred to herein.
(u) GTFQKWAAVVVPSGEEQR (SEQ ID NO: 73). This analogue is derived from peptide p45/46 (SEQ ID NO: 59), and is derived from the α3 domain of the HLA-A2 molecule, except that the glutamine residue is replaced with a glutamic acid residue. This analogue is designated "p45/p46 analogue 1" when referred to herein.
(v) GTFQKWASVVVPSGQEQR (SEQ ID NO: 74). This analogue is derived from peptide p45/46 (SEQ ID NO: 59), and is derived from the α3 domain of the HLA-A2 molecule, except that the alanine residue is replaced with a serine residue. This analogue is designated "p45/46 analogue 2" when referred to herein.
The peptides variants or analogues thereof used in the method of the invention may be chemically derived from a HLA molecule polypeptide, for example by proteolytic cleavage or can be derived in an intellectual sense from the polypeptide, for example by making use of the amino acid sequence of the polypeptide and synthesising peptides based on the sequence. Peptides may be synthesised using methods well known in the art.

### Variants and analogues ofpeptides

variant or analogue peptides are mentioned herein. Such variants or analogue typically comprise a sequence that binds to the same MHC class II molecule and/or is recognised by a T cell which recognises the corresponding epitope in the polypeptide of SEQ ID NOS: 4 to 74.

Variants of SEQ ID NO's 4 to 74 may be fragments derived by truncation, e.g. by removal of one or more amino acids from the N and/or C-terminal ends of a polypeptide. Fragments may also be generated by one or more internal deletions, provided that the core 9 amino acids that makes up the T cell epitope is not substantially disrupted.

For example, a variant of SEQ ID NO: 4 may comprise a fragment of SEQ ID NO: 4, i.e. a shorter sequence. This may include a deletion of one, two, three or four amino acids from the N-terminal end of SEQ ID NO: 4 or from the C-terminal end of SEQ ID NO: 1. Such deletions may be made from both ends of SEQ ID NO: 4. A variant of SEQ ID NO: 4 may include additional amino acids (for example from the sequence of the parent protein from which the peptide derives) extending beyond the end(s) of SEQ ID NO: 4. A variant may include a combination of the deletions and additions discussed above. For example, amino acids may be deleted from one end of SEQ ID NO: 4, but additional amino acids from the full length parent protein sequence may be added at the other end of SEQ ID NO: 4. The same discussion of variants above also applies to SEQ ID NOS: 5 to 74.

A variant peptide may include one or more amino acid substitutions from the amino acid sequence of any of SEQ ID NOS: 4 to 74 or a fragment thereof. A variant peptide may comprise sequence having at least 65% sequence identity to at least 9 or more contiguous amino acids in any of SEQ ID NOS: 4 to 74. More preferably a suitable variant may comprise at least 70%, at least 75%, at least 80%, at least 85%, at least 90%, at least 95%, or at least 98% amino acid identity to at least 9 contiguous amino acids of any of SEQ ID NO: 4 to 74. This level of amino acid identity may be seen at any section of the peptide, although it is preferably the core region. The level of amino acid identity is over at least 9 contiguous amino acids but it may be at least 10, 11, 12, 13, 14, 15 or at least 16 or 17 amino acids, depending on the size of the peptides of comparison. Accordingly, any of the above-specified levels of identity may be across the entire length of sequence.

In connection with amino acid sequences, "sequence identity" refers to sequences which have the stated value when assessed using ClustalW (Thompson et al., 1994, supra) with the following parameters: Pairwise alignment parameters -Method: accurate, Matrix: PAM, Gap open penalty: 10.00, Gap extension penalty: 0.10; Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

A variant peptide may comprise 1, 2, 3, 4, 5 or more, or up to 10 amino acid substitutions from any of SEQ ID NOS: 4 to 74. Substitution variants preferably involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid or another aliphatic amino acid. Some properties of the 20 main amino acids which can be used to select suitable substituents are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

Further variants include those in which instead of the naturally occurring amino acid the amino acid which appears in the sequence is a structural analog thereof. Amino acids used in the sequences may also be modified, e.g. labelled, providing the function of the peptide is not significantly adversely affected.
Where the peptide has a sequence that varies from the sequence of any of SEQ ID NOS: 4 to 74 or a fragment thereof, the substitutions may occur across the full length of the sequence, within the sequence of any of SEQ ID NOS: 4 to 74 or outside the sequence of any of SEQ ID NOS: 4 to 74. For example, the variations described herein, such as additions, deletions, substitutions and modifications, may occur within the sequence of any of SEQ ID NOS: 4 to 74. A variant peptide may comprise or consist essentially of the amino acid sequence of any of SEQ ID NOS: 4 to 74 in which one, two, three, four or more amino acid substitutions have been made. A variant peptide may comprise a fragment of the parent protein that is larger than any of SEQ ID NOS: 4 to 74. In this embodiment, the variations described herein, such as substitutions and modifications, may occur within and/or outside the sequence of any of SEQ ID NOS: 4 to 74.

The variant peptides of the invention are 9 to 30 amino acids in length inclusive. Preferably, they may be from 9 to 20 or more preferably 13 to 17 amino acids in length. The peptides may be the same length as the peptide sequences in any one of SEQ ID NOS: 4 to 74.

The term "peptide" includes not only molecules in which amino acid residues are joined by peptide (-CO-NH-) linkages but also molecules in which the peptide bond is reversed. Such retro-inverso peptidomimetics may be made using methods known in the art, for example such as those described in Meziere et al (1997) J. Immunol.159, 3230-3237. This approach involves making pseudopeptides containing changes involving the backbone, and not the orientation of side chains. Meziere *et al* (1997) show that, at least for MHC class II and T helper cell responses, these pseudopeptides are useful. Retro-inverse peptides, which contain NH-CO bonds instead of CO-NH peptide bonds, are much more resistant to proteolysis.

Variants may be altered by the inclusion of additional residues at both / either end of an epitope sequence or internally to an epitope sequence to improve solubility, provided that the variant still functions as an MHC class II-binding T cell epitope.

Similarly, the peptide bond may be dispensed with altogether provided that an appropriate linker moiety which retains the spacing between the carbon atoms of the amino acid residues is used; it is particularly preferred if the linker moiety has substantially the same charge distribution and substantially the same planarity as a peptide bond. It will also be appreciated that the peptide may conveniently be blocked at its N-or C-terminus so as to help reduce susceptibility to exoproteolytic digestion. For example, the N-terminal amino group of the peptides may be protected by reacting with a carboxylic acid and the C-terminal carboxyl group of the peptide may be protected by reacting with an amine. Other examples of modifications include glycosylation and phosphorylation. Another potential modification is that hydrogens on the side chain amines of R or K may be replaced with methylene groups (-NH₂ → - NH(Me) or -N(Me)₂).

Analogues of peptides according to the invention may also include peptide variants that increase or decrease the peptide's half-life *in vivo.* Examples of analogues capable of increasing the half-life of peptides used according to the invention include peptoid analogues of the peptides, D-amino acid derivatives of the peptides, and peptide-peptoid hybrids. A further embodiment of the variant polypeptides used according to the invention comprises D-amino acid forms of the polypeptide. The preparation of polypeptides using D-amino acids rather than L-amino acids greatly decreases any unwanted breakdown of such an agent by normal metabolic processes, decreasing the amounts of agent which needs to be administered, along with the frequency of its administration.

The peptides provided by the present invention may be derived from splice variants of the parent proteins encoded by mRNA generated by alternative splicing of the primary transcripts encoding the parent protein chains. The peptides may also be derived from amino acid mutants, glycosylation variants and other covalent derivatives of the parent proteins which retain at least an MHC-binding property of the parent proteins. Exemplary derivatives include molecules wherein the peptides of the invention are covalently modified by substitution, chemical, enzymatic, or other appropriate means with a moiety other than a naturally occurring amino acid. Further included are naturally occurring variants of the parent proteins. Such a variant may be encoded by an allelic variant or represent an alternative splicing variant.

Variants as described above may be prepared during synthesis of the peptide or by post- production modification, or when the peptide is in recombinant form using the known techniques of site- directed mutagenesis, random mutagenesis, or enzymatic cleavage and/or ligation of nucleic acids.

### Examples

### Overview

Donor specific antibodies to HLA molecules contraindicate transplantation, and post-transplantation are associated with graft rejection or failure. T cell responses are important to this process because they provide help for antibody production. The inventor therefore systematically studied T cell responses to a single MHC class I molecule, HLA-A2, which is a common target antibody specificity in sensitised patients on the organ transplantation waiting list, i.e. patients who have made HLA-A2 antibodies. In both Examples 1 and 2, the inventor designed a series of 60 overlapping 15mer peptides that spanned the primary sequence of HLA*020101. However, only 53 (p1-p53) could be synthesised using F-moc technology. The binding affinity of these 53 peptides to 13 different MHC class II molecules was then studied by ELISA. The inventor found that peptides from several locations along the HLA-A2 molecule exhibited promiscuous binding to MHC.

The 30 peptides that bound MHC class II were then used to stimulate peripheral blood mononuclear cells (PBMC) from 40 transplant-listed patients with known antibody sensitisation histories. Responses were assessed by γ-interferon elispot, and the findings are summarised below.

In Example 3, the inventor compared the sequence of some of the 15mer peptides from HLA-A2 with sequences in other HLA polypeptides to find additional sequences of relevance. Examples 4 to 6 are studies of responses to the peptides of the invention in various groups of transplant patients and controls.

### Example 1

### Materials & Methods

### 1) In silico epitope prediction

In order to limit the numbers of peptides to be screened in direct MHC-peptide binding assays and to maximise discrimination in areas likely to be of interest, a systematic approach was adopted involving an initial computer based evaluation of the primary amino acid sequence of the HLA-A*0201 molecule, i.e. HLA-A2. The DNA sequence of HLA-A2 is identified as SEQ ID NO: 3 and the amino acid sequence is identified as SEQ ID NOs: 1 and 2.

Referring to Figure 1, there is shown a schematic representation of the extracellular portion of the human class I histocompatability molecule, HLA-A2. The stretches of beta conformation are represented by broad arrows (pointing N to C terminal). Regions of alpha helix are shown as helical ribbons. The pairs of spheres represent disulphide bridges. The molecule of beta-2-microglobulin (β₂m) is bound to the junction of the α1 and α2 domains, and to the α3 domain by non-covalent interactions only. Not shown in Figure 1 is the presence of a short peptide bound non-covalently in the groove between the alpha helices of α1 and α2 domains. The combination of peptide and adjacent portions of alpha helices makes up the epitope seen by CD8+ T cells. The transmembrane and cytosolic portion of the HLA-A2 molecule are not shown, but it will be appreciated that the transmembrane portion extends from about amino acid residue 283 onwards.

The analysis of the primary amino acid sequence of the HLA-A2 molecule was performed using the widely available algorithm TEPITOPE (Sturniolo, T., et al., Generation of tissue-specific and promiscuous HLA ligand databases using DNA microarrays and virtual HLA class II matrices. Nat Biotechnol, 1999. 17(6): p. 555-61). The programme was used to identify sequences that share HLA-binding motifs with previously identified and characterised epitopes. However, because such *in silico* approaches may omit epitopes that do not conform to standard motifs, the inventor conducted additional literature and database searches in order to identify all previously reported MHC class II-binding sequences from the HLA-A2 molecule.

### 2) Solubility analysis

Sequences predicted to bind to MHC class II molecules were evaluated for solubility using predictive computer-based algorithms such as that at EXPASY (www.expasy.org/). The inventor believed that such solubility analysis was an important precursor to physical binding assays as it allowed identification of any peptide sequences that contain important, naturally processed, but nominally insoluble MHC-binding core motifs. Frequently, such epitopes can be rendered soluble by the addition of various flanking residues that occur naturally in the source protein (HLA-A*0201 in this case). This approach avoided time-consuming and costly synthesis of peptides that would not generate data in MHC-binding assays, and would therefore have ultimately been of little use for clinical intervention.

### 3) Peptide synthesis

A series of 60 overlapping 15mer peptides that spanned the primary sequence of HLA*020101 was designed. However, only 53 (pl-p53) could be synthesised. Hence, a series of 53 synthetic overlapping 15mers offset by 2 to 5 residues, corresponding to soluble, MHC class II-binding motifs, were generated as HCl salts by standard Fmoc chemistry and supplied as lyophilised powder (NeoMPS SA, Strasbourg, France). Peptides were reconstituted in 10⁻⁴M HCl.

Referring to Figure 2, there are shown details of all 53 peptides, designated p1 to p53. Figure 2 shows the position, molecular weight and sequence of each peptide.

### 4) Peptide binding to purified MHC molecules in vitro

The peptide-MHC binding assay was a competition assay in which "query" peptides are titrated into wells containing immobilised, purified MHC molecules and known concentrations of biotinylated reference peptides that are known to bind to the molecule in question with high affinity.

The MHC molecules for study included: DRB1*0101 (DR1), DRB1*0301 (DR3), DRB1*0401 (DR4), DRB1*0701 (DR7), DRB1*1101 (DR11), DRB1*1301 (DR13), DRB1*1501 (DR15); DRB3*0101 (DRB3), HLA-DRB4*0101 (DRB4) and HLA-DRB5*0101 (DRB%); HLA-DPA1*0103/DPB1*0401 (DP401) and HLA-DPA1*0103/DPB1*0402 (DP402). These were undertaken at CEA-Saclay using techniques that are well established in his laboratory for the analysis of other antigens (Texier, C., et al., HLA-DR restricted peptide candidates for bee venom immunotherapy. J Immunol, 2000. 164(6): p. 3177-84).

Binding experiments were repeated on three separate occasions to confirm results. Results were expressed as both nm IC50 and as the ratio of binding of each peptide compared to a reference peptide (also screened in the assay) of known high affinity binding to each particular MHC allele.

Binding ratios of less than 20 are considered high affinity binders and those with a ratio of 21-100, moderate binders. Previous data indicated that important T cell epitopes only rarely have binding affinities outside of these windows, although there are exceptions.

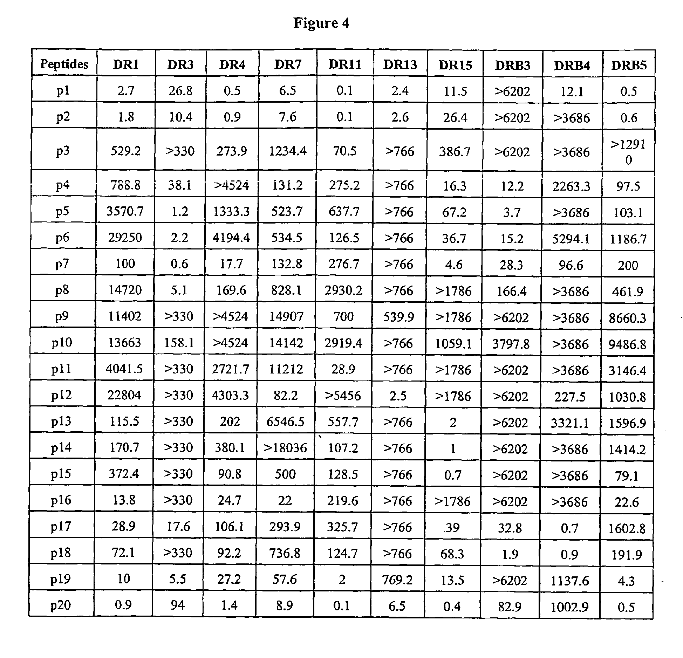

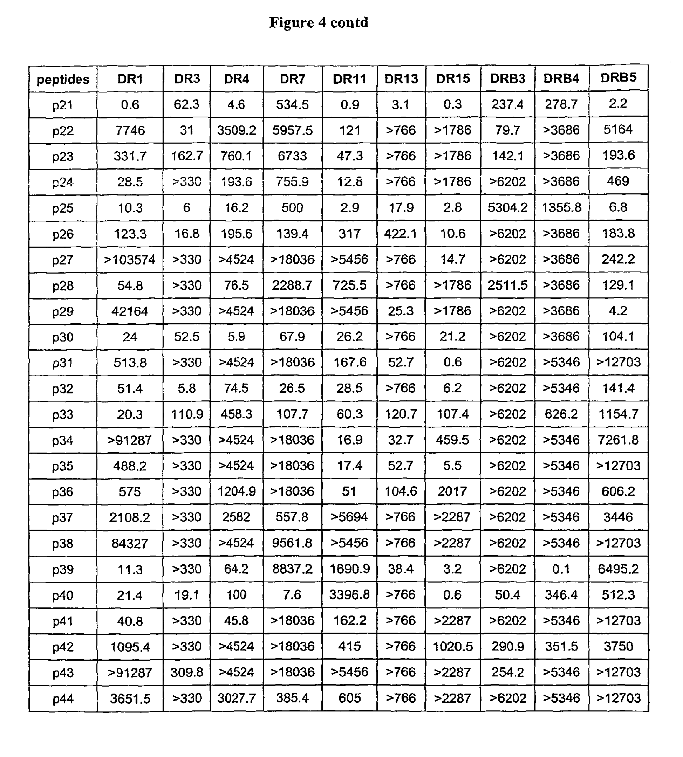

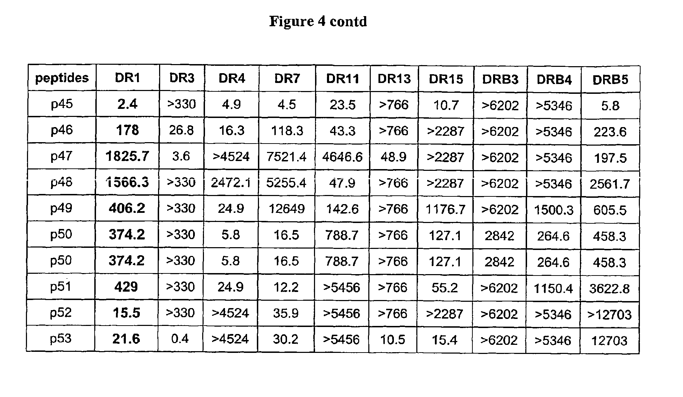

Referring to Figure 4, there is shown the binding studies data generated. These data show the ratio of the 50% inhibitory concentration (IC50) of test peptide compared to the 50% binding concentration of control peptide, to the MHC class II molecule under investigation.

### 5) In vitro Functional Analysis - Immune responses of peripheral blood mononuclear cells

The studies described above identified that a wide range of peptides bound to at least one MHC class II molecule, although a limited number showed promiscuous binding. On the basis of these studies, the number of peptides for future study was reduced from 53 to 30. 23 peptides which showed little or no significant binding to MHC class II, that is the ratio of their binding affinity to that of control peptide was >100, were not studied further. This was undertaken in order to limit the amount of blood that was required from each patient under study.

The presence of lymphocytes specific for any given peptide was determined using γ interferon elispot with 500,000 peripheral blood mononuclear cells per well in quadruplicate. (Briefly, peripheral blood mononuclear cells (PBMCs) were separated from heparinised whole blood by density centrifugation. These were washed and resuspended in complete culture medium (containing 10% AB serum). Cells were cultured with individual peptides in 96 well γ interferon elispot plates, (Mabtech, Nacka Strand, Sweden) in 100ul complete medium, with peptide at a concentration of 4µg and 20µg/ml. The frequency of γ-interferon producing cells was evaluated by ELIspot, as shown in Figure 7 for a single example patient. The frequency and specificity of peptide specific responses was then compared between individuals in the sensitised and unsensitised groups.

### 6) Recruitment of subjects for study

Patient subjects were recruited from the University Hospital Birmingham renal transplant waiting list Patients on dialysis at University Hospital Birmingham were approached initially, following receipt of local research ethical committee approval. Subjects who were currently receiving immunosuppression or who had a haemoglobin < 10g/dl were excluded. Having obtained fully informed consent, 50 ml of venous blood was obtained at the time of routine venesection.

The HLA type determined by standard molecular techniques was available for each subject from routine clinical assessment by the West Midlands Blood Transfusion Service Immunogenetics Laboratory. This laboratory characterised anti-HLA antibody specificities using standard flow-cytometric based techniques including the use of monospecific beads, within the context of routine clinical practice and provided relevant historical information on antibody specificities for the respective subjects.

### Results

The inventor examined 5 groups of patients summarised in the Table below.

**Table 3 - Patient groups**

| **Patient anti-HLA antibody status** | **Patient HLA type** | |
|---|---|---|
| | **A2+** | **A2-** |
| **Sensitised with anti-HLA A2** | | 12/15 (Group 1) |
| **Sensitised no anti-HLA A2** | 0/6 | 1/5 |
| | (Group 2) | (Group 3) |
| **Unsensitised** | 1/6 | 2/8 |
| | (Group 4) | (Group 5) |

The presence or absence of antibodies was determined using standard solid phase, flow cytometric based techniques (`Luminex™'). The denominator in each group identifies the number of patients studied and the numerator, the number in each group that responded to at least 1 peptide.

In group 1, 10 patients responded to peptides p20/21. This is from the highly polymorphic region of HLA-A2. Closely overlapping peptides have been eluted from human B lymphocyte HLA DR1, i.e. it has been shown to be a 'naturally processed' and presented endogenous protein. A larger peptide containing these sequences has also been identified as a DR15 restricted epitope in a patient who rejected an A2 positive kidney.

In group 1: 10 patients responded to peptides derived from the α3 and transmembrane domains that show very limited polymorphism. These peptides were most commonly p39 (192-206), p50/51(268-284) and p52/53 (280-296), although a small number of other sites also resulted in responses including p40 (202-216) and p45/46 (239-256). These peptides have not previously been identified as T cell epitopes. In two of the patients, some of the α3 / transmembrane peptides were autologous epitopes, for example patient 1 is A68 positive and therefore shares the sequences of p39, p52/53 with HLA-A2.

Referring to Figure 5, there is shown the sequence of peptide 39. The left hand circle shows the HLA types in which the sequence of the homologous region is shared with HLA-A2 and the right hand circle, those HLA types in which the homologous region is different from HLA-A2, and the two amino acids by which they differ (alanine to proline at 193 and valine to isoleucine at 194).

Referring to Figure 6, there is shown the sequences of peptides p50 and p51. The left hand circle shows the HLA types in which the sequence of the homologous region is shared with HLA-A2 and the other circles, those in which the homologous region is different from HLA-A2, and the amino acids by which they differ, (proline to leucine at 276, for A1, A3, A11, A30, A36 etc).

Referring to Figure 7, there is shown the sequences of peptides p52 and p53. The left hand circle shows the HLA types in which the sequence of the homologous region is shared with HLA-A2 and the other circles, those in which the homologous region is different from HLA-A2, and the amino acids by which they differ, (phenylalanine to leucine at 294 for A1, A3, A11, A30, A36 etc).

In group 2: there was no response.

In group 3: 1/5 patients made a response to peptides from the α3 and transmembrane domains. This again is consistent with the hypothesis that patients sensitised to other HLA-A, not HLA-A2 are likely to make responses to shared sequences from the α3 and transmembrane domains.

In group 4: 1/6 patients made a response. This is an auto response to p39, 50/51, 52/53 and p11. Such a response has also been observed in 1/6 'non-dialysis' normal controls.

In group 5: 2/8 made a response p39 or p50/51 & p52/53. These patients had received previous transfusions and may therefore have been sensitised at the T lymphocyte level without producing anti-HLA antibody.

### Example Patient

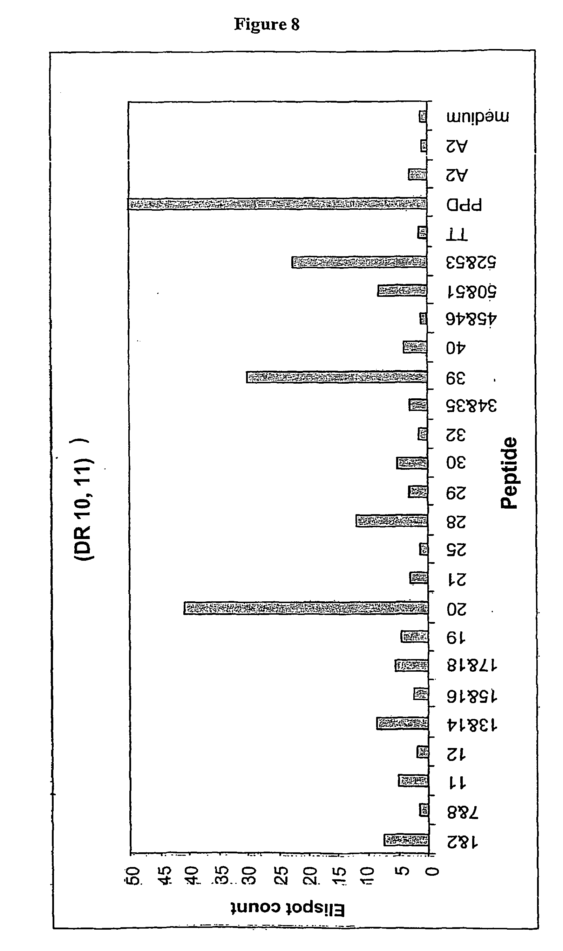

Referring to Figure 8, there is shown an Elispot count of peptide reactive cells/500,000 PBMCs. The concentration of peptide was 20µg/ml. The data show that this individual, who has made high levels of anti-HLA antibody for approximately 10 years, on the background of previous pregnancy and transfusion, makes high frequency responses to p20, p39 and a mixture of p52 and p53. Her tissue type is A1,68; B37,44; C6,7 DR10,11. Hence, the patient responded to the highly polymorphic region and in particular to a sequence within the HLA-A2 sequence residues 105-121 covered by p20 & 21, that the inventor demonstrated binds promiscuously to MHC class II. She also responded to peptides from the α3 and transmembrane region of HLA-A2, the properties of which are described above. Indeed in her case, these responses are autoreactive because these sequences are shared with HLA-A68. The positive control response is to purified protein derivative of mycobacterium tuberculosis (PPD) and the negative control to medium alone.

### Summary

In summary, the results show that peptides of limited polymorphism from the α3 and transmembrane domains frequently induce an immune response. This is usually allogeneic, but sometimes autoimmune, in specificity. The association of responses to these peptides with sensitisation to HLA-A2 implies that they are relevant to anti-HLA antibody formation. They also constitute indirectly presented epitopes that are likely to drive rejection through T cell mediated mechanisms of damage. As these peptides are so widely expressed, they are ideal targets for therapeutic desensitisation. Furthermore, peptide analogues disclosed herein comprising either one or two amino acid substitutions provide almost complete representation of HLA-A.

Furthermore, each of the preferred peptides in accordance with the invention may be expressed by many other HLA-A molecules, and not just HLA-A2. Hence, the benefits of using such peptides in methods according to the invention, and in particular for uses in predicting graft rejection, may be much wider than to HLA-A2 antigen alone.

Without being bound by any hypothesis, it is suggested that a small mixture or "cocktail" of peptides, variants or analogues in accordance with the invention may be used detect T cell responses to the majority of MHC class I molecules, and therefore enable a more accurate determination of whether or not a patient is at risk of graft rejection.

### Example 2

### Materials and Methods

### Design of peptides

60 overlapping peptides, 15 or 16 amino acids long, were designed so as to give optimal coverage of HLA-A*020101 for putative T cell epitopes, using the programme Tepitope (33). Solubility of peptides was predicted using the programme at **www.expasy.org**. Peptides were synthesized (NeoMPS, Strasbourg, France) using Fmoc chemistry as previously described, but of those designed, only 53 could be made. Biotinylation of control peptides was achieved by reaction with biotinyl-6-aminocaproic acid (Fluka Chimie, St. Quentin Fallavier, France) at the NH₂ terminus of the molecule. All peptides were purified by reverse-phase HPLC on a C 18 Vydac column, and their quality was assessed by electrospray mass spectroscopy and analytical HPLC.

### Purification of HLA-DR Molecules

HLA-DR molecules were purified from HLA-homozygous EBV cell lines by affinity chromatography using the monoclonal anti-DR antibody L-243, coupled to protein A-Sepharose CL 4B gel (Amersham Pharmacia Biotech, Orsay, France) as previously described (Texier et al., J Immunol 2000; 164(6):3177-84).

### HLA-DR Peptide Binding Assays

Binding of peptides to different HLA-DR molecules was performed in a competition assay as previously described (Texier et al., Eur J Immunol 2001;31(6):1837-46). Biotinylated control peptides that are good binders to the MHC class II molecule under investigation were used (see Figure 9) and test peptides titrated into the binding assay with purified, immobilised MHC class II. Maximal binding was determined by incubating the biotinylated peptide with the MHC class II molecule in the absence of competitor. Data are expressed as the concentration of peptide that prevented binding of 50% of the labelled peptide (IC50). An IC50 ratio of control peptide to test peptide of < 20 was considered high affinity binding and 20-100 as moderate affinity.

### Study Population

This study was performed with the approval of the South Birmingham research ethics committee. All subjects were on dialysis at University Hospital Birmingham or St James Hospital, Leeds. Blood samples were obtained into heparin from dialysis patients at the time of routine venepuncture. HLA genotypes were determined using standard molecular techniques at the National Blood Service Laboratory, Birmingham, UK, from which the history of alloantibody formation for each subject was available, screened by standard cytotoxicity, flow cytometry and from 2004, solid phase assay.

Patients were excluded on the basis of anaemia (Hb <10g/dl) or if they had received immunosuppressive drugs within 3 months of the investigation. Subjects were divided into 5 groups on the basis of tissue type and history of anti-HLA antibody synthesis (see Table 4).

**Table 4 - Patient groups**

| | |
|---|---|
| Group 1 | HLA-A2 negative with anti HLA antibodies to HLA-A2 |
| Group 2 | HLA-A2 negative with anti HLA antibodies to none -A2 HLA |
| Group 3 | HLA-A2 negative with no history of anti HLA antibody formation |
| Group 4 | HLA-A2 positive with anti HLA antibodies to none -A2 HLA |
| Group 5 | HLA-A2 positive with no history of anti HLA antibody formation |

### Enzyme-Linked Immunosorbent Spoi Assay

PBMC's were isolated from peripheral blood by Ficoll density-gradient centrifugation prior to use. Viable cells were enumerated by trypan blue exclusion. A γ-interferon elispot assay was used according to the manufacturer's instructions (Mabtech, Nacka Strand, Sweden). A total of 5x10⁵ PBMC's were added to each well, in a final volume of 100µl of 'complete medium': 95% RPMI 1640 medium (Sigma, Poole, UK)/5% human AB serum (PAA laboratories, Somerset, UK), with L-glutamine and penicillin/streptomycin (Sigma) along with peptide at a final concentration of 4µgml⁻¹ or 20µgml⁻¹. Peptides were used either singly or for some in pairs if the sequences were offset by 2 amino acids.

Negative control wells contained responder PBMC's plus medium alone. Positive control wells contained PPD (SSI, Copenhagen, Denmark) at a final concentration of 10µgml⁻¹, tetanus toxoid at (Calbiochem, Merck Biosciences, UK) at 1 µg/ml, or latterly a positive control (anti-CD3 based) supplied by the manufacturer of the elispot assay. PBMC's were cultured at 37°C, 5% CO₂ for 48 hours, then discarded and the plate washed 6 times with PBS. Plates were then incubated at room temperature for 2 hours with the one-step detection reagent (alkaline phosphatase-conjugated detection monoclonal antibody 7-B6-1, prepared by diluting to 1:200 in filtered PBS containing 0.5% fetal calf serum (Sigma). This was then discarded and plates washed 5 times with PBS. Filtered ready-to-use chromogenic alkaline phosphatase substrate ((nitroblue tetrazolium/5-bromo- 4-chloro-3-indolyl phosphate (BCIP/NBT-plus)) provided by manufacturer was added at a volume of 100µl/well. The plates were allowed to develop and reaction terminated once spots emerged. Plates were washed extensively under tap water and then air-dried in darkness for at least 12 hours before analysis. The number of spots in each well was then counted using an AID Elispot plate reader (Strassberg, Germany). A positive response to test peptide was defined if the number of spots per well was significantly greater than control by ANOVA.

### Inhibition of indirect alloimmune responses with anti-MHC class II antibodies

The MHC restriction of responses to peptides was investigated by addition of a mixture of an anti-MHC class II monoclonal antibody (Tu39, Becton Dickinson, Oxford, United Kingdom) and an anti-DR monoclonal antibody (L243, Becton Dickinson) to cell cultures.

### Assessment of proliferation by CFSE

PBMC's were resuspended at 10⁶cells/ml in warm RPMI at 37°C to which CFSE (Molecular Probes, Cambridge Biosciences) was added at a final concentration of 10µM. This was incubated at 37°C, 5% CO₂ with intermittent agitation for 10 minutes, when human AB serum (Sigma) was added to terminate labelling, and cells were then washed twice. Cells were resuspended at 2.5 x 10⁵ /well in 96-well round-bottom culture plates in 200µl of complete medium, in the absence or presence of test peptide at a final concentration of 4µgml⁻¹ or 20µgml⁻¹ or PPD at 10µgml⁻¹, then cultured at 37°C, 5% CO₂ for 10 days. Cells were recovered from 3 or more wells, washed twice and stained with antibody to CD4 (SK3-PerCP, Becton Dickinson) or CD8 (SKI-PerCP, Becton Dickinson), or with isotype control (IgGI-PerCP, Becton Dickinson). Samples were analysed using a FACSCalibur flow cytometer (Becton Dickinson) using Winmdi 2.8 software (Scripps Research), acquiring information from a total of 50,000 viable cells.

### Results

### Peptide binding studies demonstrate multiple regions of promiscuous binding to HLA-DR

A total of 60, 15mer peptides spanning HLA-A2 were designed and 53 could be made. The other 7 peptides, largely derived from the transmembrane region, were insoluble and could not therefore be satisfactorily synthesised. The affinity of peptide binding to HLA-DR is shown in Figure 9.

The data show that several sequences exhibit promiscuous binding to a range of HLA-DR. These include peptide 20 (105-119), the closely overlapping peptide 21 (107-121) and peptide 7 (31-45) which correspond to sequences previously reported from experiments in which peptide was eluted from MHC class II. It is apparent that large numbers of different epitopes from HLA-A2 bind to MHC class II of different specificities (Figure 9). This is of particular interest since a composite epitope of self derived MHC peptide presented in the context of self MHC class II, would be a secure means by which to define self.

Peptides 20 and 21 also correspond to the epitope (92-120) recognised by DR15 restricted T cell clones generated from the recipient of a failed HLA-A2 mismatched renal transplant.

On the basis of the relatively common finding of high or moderate affinity binding to MHC class II, 30 peptides were further assessed in functional experiments. A small number of peptides offset by two amino acids were used as a mix of two peptides, in order to optimise the utilisation of PBMCs.

### γ-interferon production in response to peptides from HLA-A2

The details of subjects recruited are shown in Figure 10. The number of cells producing γ-interferon in response to PPD: 143 (15 - 422)/10⁶ PBMCs was significantly lower in patients on dialysis who had made alloantibody than in (unmatched) healthy controls 337 (40 - 885)10⁶ PBMCs (p=0.007). The medium controls in these subjects varied between 0 - 9/10⁶ PBMCs and the maximum response to peptide was 122/10⁶ PBMCs.

The number of patients responding to at least one peptide, assessed by γ-interferon elispot is shown in Table 5. The number of patients responding to at least one HLA-A2 derived peptide is shown for each patient group. Patients who make anti-HLA-A2 antibody are significantly more likely than other patients to make an indirect response to HLA-A2 derived peptide.

**Table 5 - Summary of results in each of the 5 groups**

| | **Tissue type** | |
|---|---|---|
| **Antibody** | **A2+** | **A2-** |
| **Anti-HLA with anti-A2** | | ***Group1*** |
| | | **14/18** |
| **Anti-HLA with no anti-A2** | ***Group 4*** | ***Group 2*** |
| | **1/9** | **3/8** |
| **No anti HLA** | ***Group 5*** | ***Group 3*** |
| | **1/10** | **2/10** |

In most patients the number of responding cells was highest with peptide at a concentration of 20µgml⁻¹ but in a small number the optimal concentration was 4µgml⁻¹. In Figure 1 the response to peptide at 20µgml⁻¹ is shown for each individual.

In subject group 1, 14/18 patients made a response to at least one peptide from HLA-A2, shown in Fig 1a. The number of patients making a response in group 1 was statistically significantly greater than in the other groups combined (p<0.0001) (although compared to group 2 alone the difference did not reach significance p=0.08 by Fishers exact test). The association of indirect alloimmune responses to HLA-A2 and the formation of specific antibody against HLA-A2, is consistent with animal models of alloantibody formation (Lovegrove et al., J Immunol 2001;167(8):4338-44).

A less expected finding was the observation that although 12 'group 1' subjects responded to peptides from the α1 and α2 hypervariable region (most commonly p20 or p21), of these subjects, 8 also responded to other peptides from the α3 and early transmembrane region; and two more responded to these alone. Responses to peptides from these regions have to the inventors' knowledge, not previously been reported in human or animal studies. These sequences are of very limited polymorphism, they are expressed by a wide range of alleles as illustrated in Table 6.

**Table 6 - Illustration of HLA types sharing sequences with HLA-A2 in the region of peptides p39, 40, 50, 51, 52 and 53**

| Peptide | Site | Sequence(s) | Sequenced shared by | and |
|---|---|---|---|---|
| P39 | 192-206 | HAVSDHEATLRCWAL | A2, 25, 26, 29, 31, 32, 33, 43, 66, 68, 69, 74 | A34 |
| P40 | 202-216 | RCWALSFYPAEITLT | | A34, 80 |
| P50 | 268-282 | KPLTLRWEPSSQPTI | | A80 |
| P51 | 270-284 | LTLRWEPSSQPTIPI | | A80 |
| P52 | 280-294 | PTIPIVGIIAGLVLF | | A3402 |
| P53 | 282-296 | IPIVGIIAGLVLFGA | | A3402 |

Furthermore 3 patients from 'group 1' made significant responses to peptides from the α3 domain that were shared between HLA-A2 and self HLA-A68 (subject 1.1 responded to p39 and p52/53), HLA-A2 and self HLA-A33 (subject 1.10 responded to p52/53) or HLA-A2 and self HLA-A32 (subject 1.17 responded to p39 and p52/53).

In subject 'group 2', 3 patients responded to a range of HLA-A2 derived peptides (results not shown). For example subject 2.1 responded to peptide p30, p40, 45/46, 50/51 & 52/53 present in a range of different HLA-A (Table 5), including the hypervariable region peptide p30. A similar pattern of response to both hypervariable and α3 and transmembrane region was observed with subject 2.2 and 2.3. None of these peptides are unique to HLA-A2 so the findings remain consistent with the history of antibody production. In patient 2.2 responses were made to sequences p39 and p52/53 shared with self HLA-A68.

In 'group 3', 3 subjects made significant responses to HLA-A2 derived peptides. Subject 3.3 responded to p39, 3.1 to p1/2 and p39 and 3.7 responded to p19 and p52/53. Although these patients did not make anti-HLA antibody detectable on current or historic sera, all had been multiply transfused and it is therefore possible that all had been primed to these peptides without making a humoral response. In patient 'group 4' one patient 4.9, who had rapidly lost a renal transplant (A3,11) to Banff III rejection 17 months prior to testing, made an autoimmune response to p 39, p 50/51 and p52/53 but also to p20 which is unique to HLA-A2. One patient, 5.1 from 'group 5' also made an autoimmune response to peptides p39, p50/51 and p52/53. A group of 15 normal controls, (designated group 6) with no known prior sensitising events were also tested. One male (6.3) made an immune response to HLA-A2 derived peptides including p39, and p50/51 and p52/53. This individual was HLA-A32 positive and these responses were therefore autoimmune. No other normal controls made any detectable response.

### γ-interferon production in response to HLA-A2 peptides are inhibited by antibody against MHC class II

The class II restriction of responses was inferred from inhibition of interferon production by antibodies against MHC class II in 3 individuals. A representative experiment is shown in Figure 11.

### Proliferation of CD4⁺ T cells in response to HLA-A2 peptides

The correspondence of proliferation with γ-interferon production was studied in the 'normal control': 6.3 who responded to peptides p39 and p52/53 assessed by elispot. As shown in Figure 12, there was proliferation of CD4+ cells in the presence of these peptides but not in the presence of control peptides that did not stimulate interferon production.

### Discussion

The contribution of indirect allorecognition to rejection is well established in various experimental models of transplantation (for example, Benichou et al., J Exp Med 1992;175(1):305-8). In clinical studies, indirect allorecognition has been associated with rejection of the kidney, heart and lung. The antigens used in these clinical studies include: freeze-thaw lysed cells, peptides spanning the β1 domain of HLA-DR and peptides from the α1 domain of class I HLA. The indirect alloresponse has been detected by the production of IL2, cellular proliferation (in primary or secondary cultures) or cytokine production by elispot.

In both experimental and clinical studies the presence of a single 'immunodominant' epitope is frequently reported, but these findings may be determined by experimental design, such as the use of secondary cultures to detect responses following preliminary expansion of T lymphocyte numbers in vitro. In fact multiple epitopes may be detected using these methods but at different time points post transplantation. Furthermore in animal models of transplantation it is apparent that there may be recognition of multiple epitopes apart from the 'proliferatively immunodominant' epitope; and these responses may include 'cryptic self.

Use of the elispot technique allows the detection of antigen specific T lymphocytes present at relatively low frequencies, in primary culture. It has allowed assessment of indirect allorecognition in patients on the renal transplant waiting list. In particular the inventor analysed the immune responses of patients who make an alloantibody of known specificity against HLA-A2. The frequency of responses the inventor observed are consistent with those seen in renal transplant recipients stimulated with 20mer peptides derived from donor HLA-DR similarly assessed by γ-interferon elispot. These are of the same order of magnitude to frequencies reported from renal transplant recipients stimulated by donor PBMC lysates assessed by DNA synthesis in limiting dilution analysis and lung transplant recipients with bronchiolitis obliterans stimulated by either one or two α1 domain peptides (A1, A2, B8 and B44 derived) also assayed by DNA synthesis in limiting dilution in another study. In this study a 25mer peptide: 60-84 from the α1 domain of HLA-A2 was used, but the inventor observed a response to this region in only one patient.

The results demonstrate that although responses to hypervariable region peptides were relatively common, as expected from the literature, there was the unexpected finding of responses to peptides from the α3 and early transmembrane region. As illustrated in table 6, peptide sequences such as p39, p40, p50/51 and p52/53 are widely represented in HLA-A and in those in which these sequences are not present there is commonly a single or dual amino acid polymorphism that encompasses most other types. These peptides could therefore be described as 'public T cell epitopes', the implication of which is that exposure to one HLA molecule can result in the priming of T lymphocytes that respond to a range of other HLA-A family members or vice versa. Since these epitopes are distinct from those recognised by antibody this is one potential mechanism for the diminished graft survival in sensitised patients, irrespective of the detection of donor specific antibodies. Similarly it suggests a mechanism whereby blood transfusion could influence anamnestic antibody responses, irrespective of the presence of a recognised B cell epitope.

Since sequences identified as 'public T cell epitopes' in HLA-A2 show only very limited polymorphism, it is possible that a portion of the allogeneic response will cross react with self-peptide, as has been reported by Benichou and colleagues for the immune response to MHC class I peptides in mice (Tam et al., J Immunol 1996;156(10):3765-71); and this possibility is currently under investigation. Indeed a number of the responses described in Fig 2 are truly autoimmune, since the sequence of the peptide to which a response is made is shared between HLA-A2 and self. This is illustrated by patients 1.1, 1.10 and 1.17. It is similar to the response seen by Lovegrove and colleagues to cryptic self-epitopes, in a rat model of rejection associated with alloantibody formation (Lovegrove et al., J Immunol 2001;167(8):4338-44).

The finding of 'public T cell epitopes' is also relevant to the mechanism of the blood transfusion effect: the benefit of prior blood transfusion on renal transplant survival, mostly observed before the widespread use of calcineurin inhibitors. Experimental models of the transfusion effect suggest that there is induction of regulatory T cells specific for indirectly presented alloantigen (Kishimoto et al., J Am Soc Nephrol 2004; 15(9):2423-8). Although these generally report donor antigen specific tolerance there are now models, attempting to mimic the clinical scenario, in which random blood transfusions induce regulatory cells that protect against rejection (Bushell et al., Transplantation 2003;76(3):449-55) apparently without non-specific depression of the immune response (Bushell et al., J Immunol 2005;174(6):3290-).

In humans the blood transfusion effect requires blood donor and recipient to share at least one HLA-DR and a relatively small number of transfusions for maximum benefit. This is despite the wide range of alloantigen that recipients may encounter. This could be accounted for by the induction of self restricted regulatory cells, specific for allogeneic epitopes that are though common to a wide range of donors. Whilst these need not necessarily be from the MHC, the 'public T cell epitopes' described above fulfil the properties necessary to account for much of the available evidence.

A second property of many of the peptide epitopes derived from HLA-A2 is that they bind with some promiscuity to MHC class II, and correspondingly induce responses in a relatively high proportion of sensitised patients. This was true both of epitopes unique to HLA-A2 and of 'public T cell epitopes'. This, in part permitted conclusions to be drawn from the study of only 55 subjects unselected for HLA type. The implications are though wider with respect to the potential use of such peptides in desensitisation protocols.

There has been longstanding interest in the modulation of rejection by alloantigen derived peptides. These effects are generally antigen specific which if translated into the clinic would limit the usefulness of such an approach. Those HLA derived peptides that have stimulated greatest interest have therefore impacted upon pathways independent of conventional TCR based antigen recognition. This is distinct from many animal models of peptide induced tolerance in which there is evidence of antigen specificity through the induction of regulatory T cells. The properties exhibited by epitopes from the α3 and trans-membrane domains, that is of being both public and promiscuous, identify ideal candidates with which to explore peptide immunotherapy in transplantation. The induction of regulatory T cell activity has for example been reported in allergic patients treated with peptide desensitisation protocols.

In summary the identification of indirect allo-epitopes from the α3 and trans-membrane regions of MHC class I has important implications for the allogeneic immune response, its regulation and the development of antigen specific therapy.

### Example 3

The inventor compared the sequence of some of the 15mer allo-epitopes identified in Examples 1 and 2 with the corresponding sequences in other HLA polypeptides. The inventor thereby identified additional allo-epitopes. The results are shown in Figures 13 to 16.

### Example 4

In a preliminary study, the Inventor has tested T cell responses to HLA class I peptides in two chronic Graft Versus Host Disease (cGVHD) individuals who have received bone marrow transplants. The peptides tested are as shown in Table 7. For all peptides in Table 7, the suffix "a1" indicates that a peptide is "analogue 1" of the correspondingly numbered peptide.

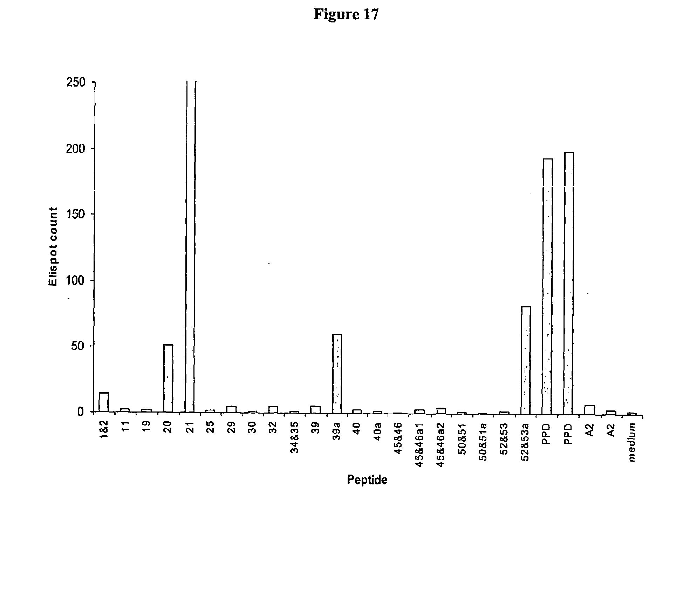

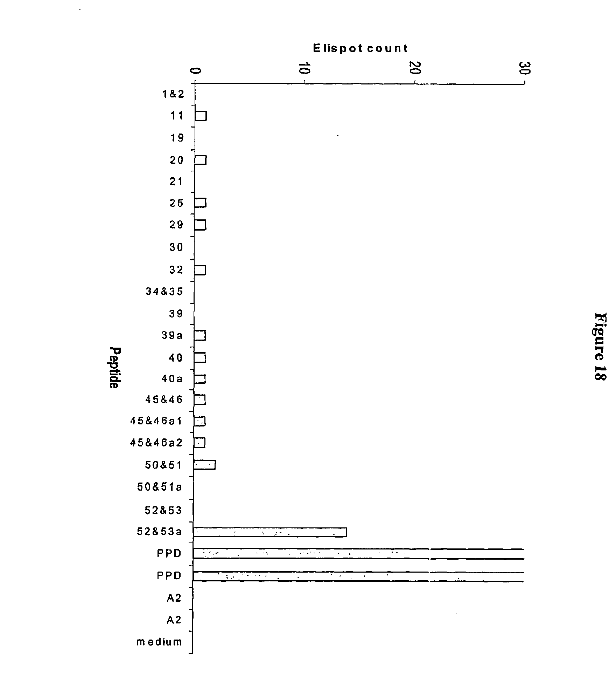

Responses to each peptide were determined by ELISPOT assay for interferon-gamma production as described in Example 1. Peptides were tested at a final concentration of 20µgml⁻¹. The results are shown in Figures 17 and 18.

**Table 7**

| Peptide | Position in HLA-A2 | **Amino acid sequence** | | | | | | | | | | | | | | | |
|---|---|---|---|---|---|---|---|---|---|---|---|---|---|---|---|---|---|
| | | | | | | | | | | | | | | | | | |
| P1 | 3-17 | H | S | M | R | Y | F | F | T | S | V | S | R | P | G | R | |
| P2 | 5-19 | M | R | Y | F | F | T | S | V | S | R | P | G | R | G | E | |
| P11 | 57-71 | P | E | Y | w | D | G | E | T | R | K | V | K | A | H | S | |
| P19 | 101-115 | C | D | V | G | S | D | W | R | F | L | R | G | Y | H | Q | |
| P20 | 105-119 | S | D | W | R | F | L | R | G | Y | H | Q | Y | A | Y | D | |
| P21 | 107-121 | W | R | F | L | R | G | Y | H | Q | Y | A | Y | D | G | K | |
| P25 | 121-135 | K | D | Y | I | A | L | K | E | D | L | R | S | W | T | A | |
| P29 | 136-150 | A | D | M | A | A | Q | T | T | K | H | K | W | E | A | A | |
| P30 | 145-159 | H | K | W | E | A | A | H | V | A | E | Q | L | R | A | Y | |
| P32 | 154-168 | E | Q | L | R | A | Y | L | E | G | T | C | V | E | W | L | |
| P34 | 163-177 | T | C | V | E | W | L | R | R | Y | L | E | N | G | K | E | |
| P35 | 165-179 | V | E | W | L | R | R | Y | L | E | N | G | K | E | T | L | |
| P39 | 192-206 | H | A | V | S | D | H | E | A | T | L | R | C | W | A | L | |
| P39a1 | 192-206 | H | P | I | S | D | H | E | A | T | L | R | C | W | A | L | |
| P40 | 202-216 | R | C | W | A | L | S | F | Y | P | A | E | I | T | L | T | |
| P40a1 | 202-216 | R | C | W | A | L | G | F | Y | P | A | E | I | T | L | T | |
| P45 | 239-253 | G | T | F | Q | K | W | A | A | V | V | V | P | S | G | Q | |
| P46 | 241-256 | F | Q | K | W | A | A | V | V | V | P | S | G | Q | E | Q | R |
| P45a1 | 239-253 | G | T | F | Q | K | W | A | A | V | V | V | P | S | G | E | |
| P46a1 | 241-256 | F | Q | K | W | A | A | V | V | V | P | S | G | E | E | Q | R |
| P45a2 | 239-253 | G | T | F | Q | K | W | A | S | V | V | V | P | S | G | Q | |
| P46a2 | 241-256 | F | Q | K | W | A | S | V | V | V | P | S | G | Q | E | Q | R |
| P50 | 268-282 | K | P | L | T | L | R | W | E | P | S | S | Q | P | T | I | |
| P51 | 270-284 | L | T | L | R | W | E | P | S | S | Q | P | T | I | P | I | |
| P50a1 | 268-282 | K | P | L | T | L | R | W | E | L | S | S | Q | P | T | I | |
| P51a1 | 270-284 | L | T | L | R | W | E | L | S | S | Q | P | T | I | P | I | |
| P52 | 280-294 | P | T | I | P | I | V | G | I | I | A | G | L | V | L | F | |
| P53 | 282-296 | I | P | I | V | G | I | I | A | G | L | V | L | F | G | A | |
| P52a1 | 280-294 | P | T | I | P | I | V | G | I | I | A | G | L | V | L | L | |
| P53a1 | 282-296 | I | P | I | V | G | I | I | A | G | L | V | L | L | G | A | |

### Example 5

In a further preliminary study, the Inventor has tested T cell responses to HLA class I peptides in three Chronic Allograft Nephropathy (CAN) individuals. The peptides tested are as shown in Table 8.

For all peptides in Table 8, suffixes to the peptide numbers indicate variants or analogues of a given peptide, with the changes from the original sequence highlighted in bold or italic font. In particular, the suffix "a1" indicates that a peptide is "analogue 1" of the correspondingly numbered peptide. Similarly, "a2" indicates "analogue 2" and so on. The suffix "(ser)" indicates that a peptide is a variant in which a cysteine residue in the correspondingly numbered peptide has been replaced with serine. The suffix "(b)" indicates that a peptide is a variant in which a cysteine residue in the correspondingly numbered peptide has been replaced with aminobutyric acid. The suffix "rk" indicates that a Lysine and an Arginine residue (in that order, N-C terminus) have been added at the N terminus of the correspondingly numbered peptide, and an Arginine and a Lysine residue (in that order, N-C terminus) have been added to the C terminus as shown.

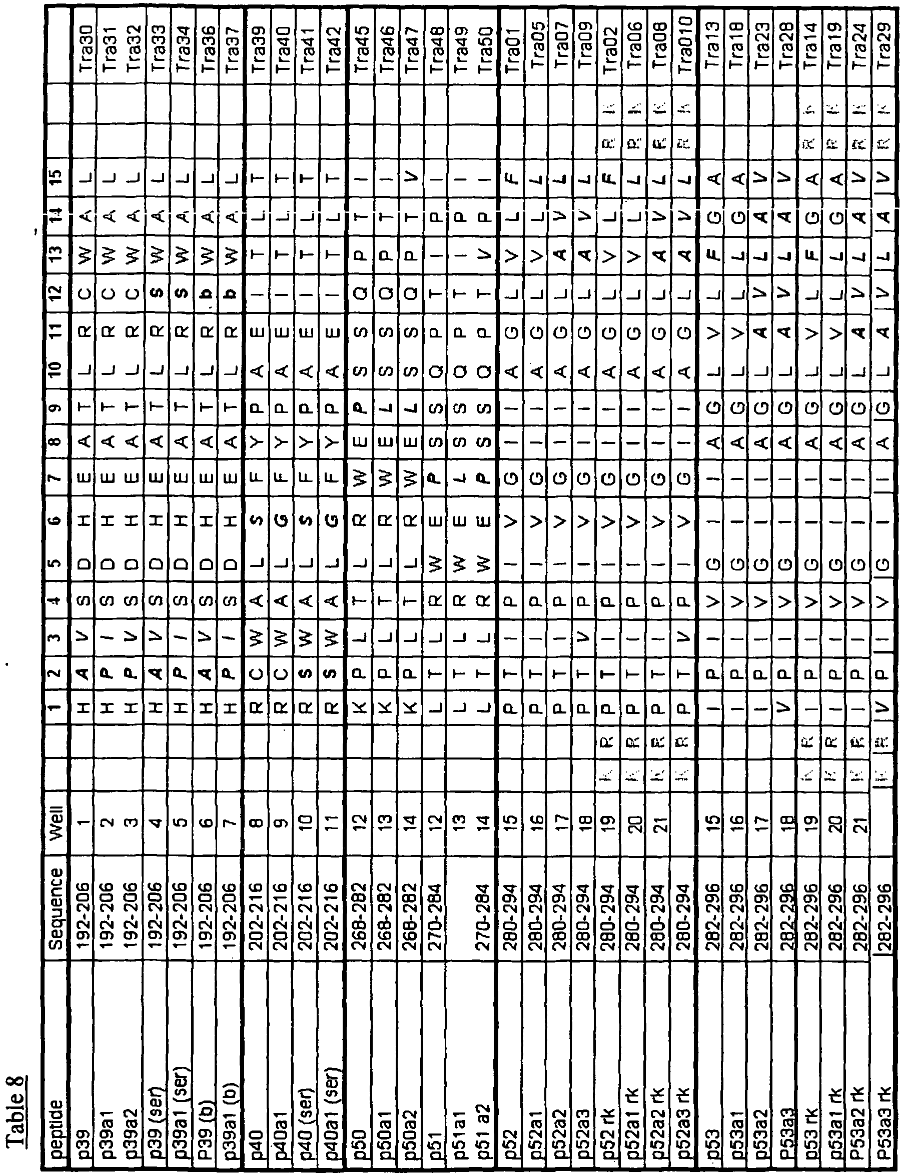

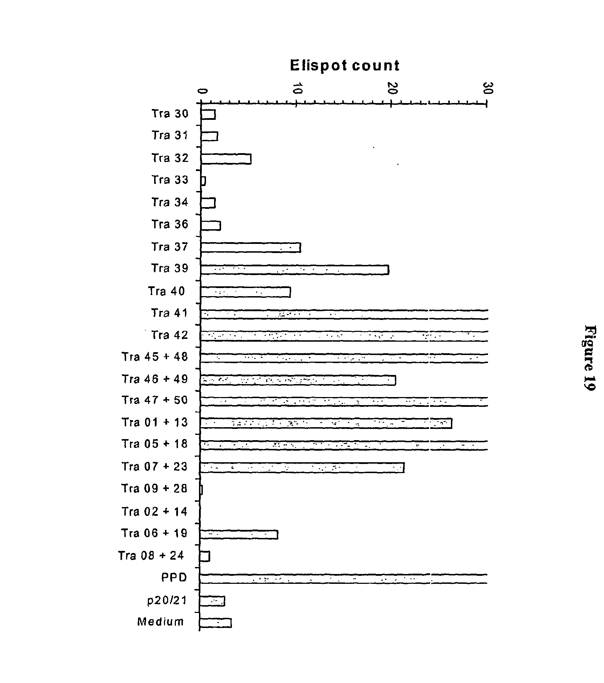

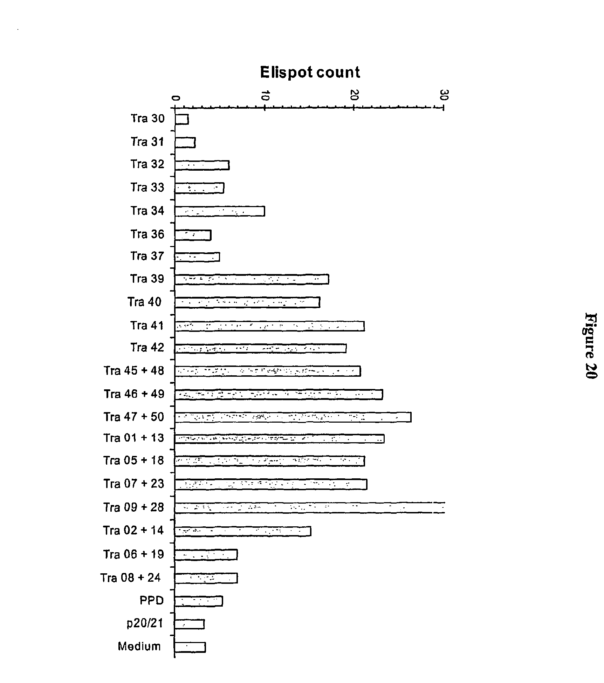

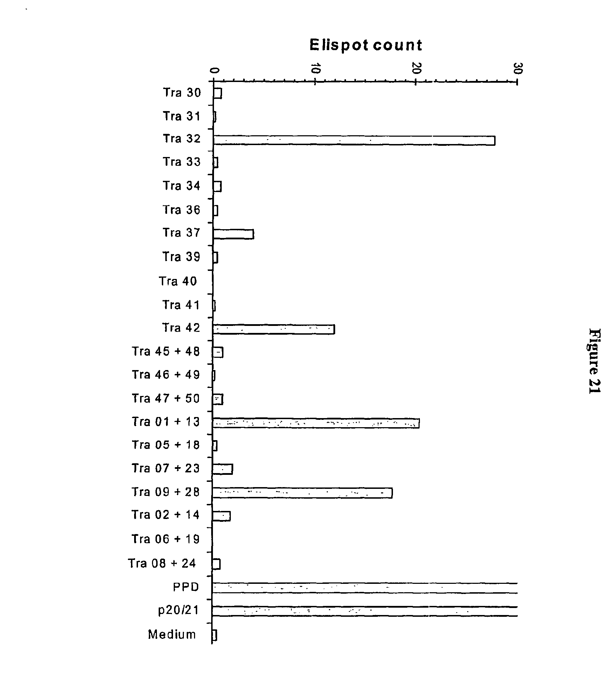

Responses for each peptide were determined by ELISPOT assay for interferon-gamma production as described in Example 1. Peptides were tested at a final concentration of 20µgml⁻¹. The results are shown in Figures 19 to 21.

### Example 6

The inventor has carried out a larger study testing T cell responses to HLA class I peptides in 40 individuals who have received bone marrow transplants (BMT). 21 of these individuals were diagnosed with chronic cGVHD (patient series C). 19 individuals were classified as not having cGVHD (patient series B). 30 "normal" individuals with no history of allosensitisation were tested to establish a baseline (control series P).

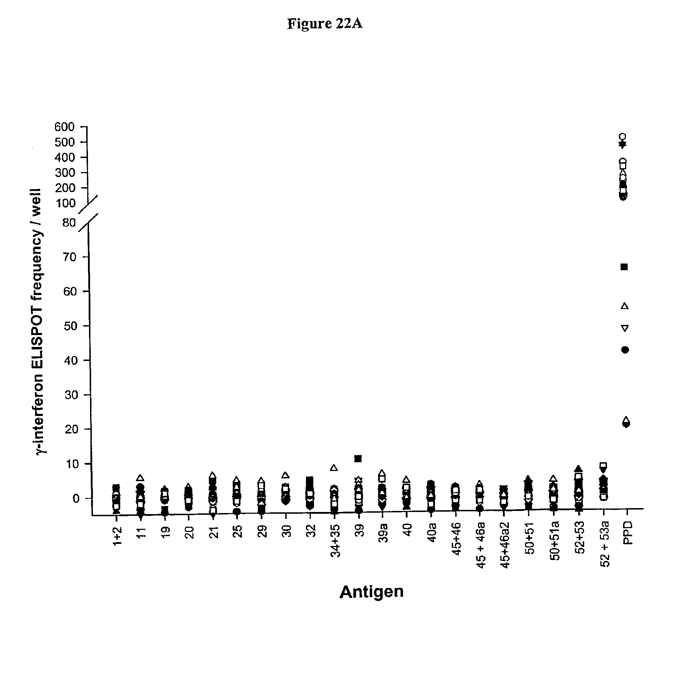

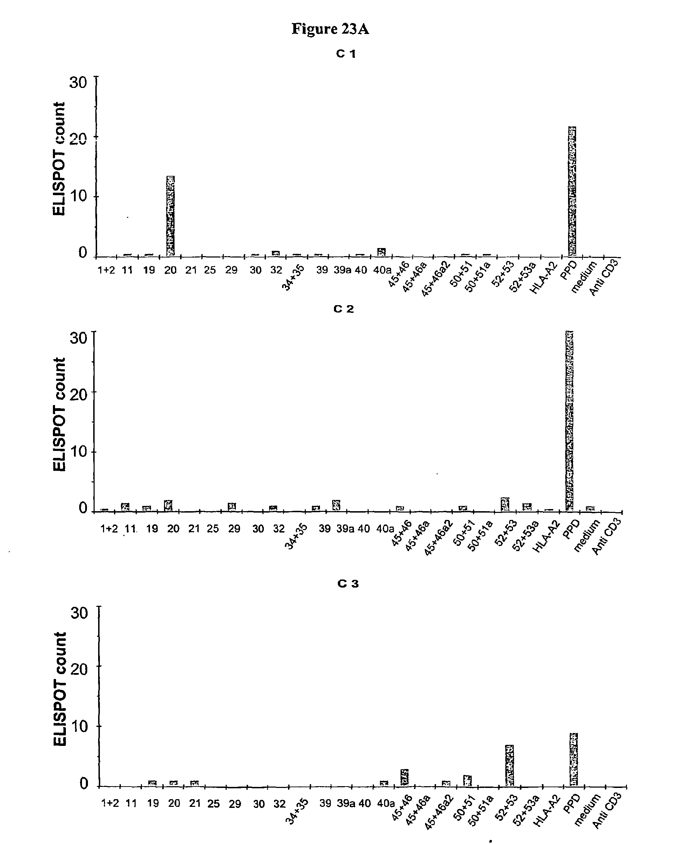

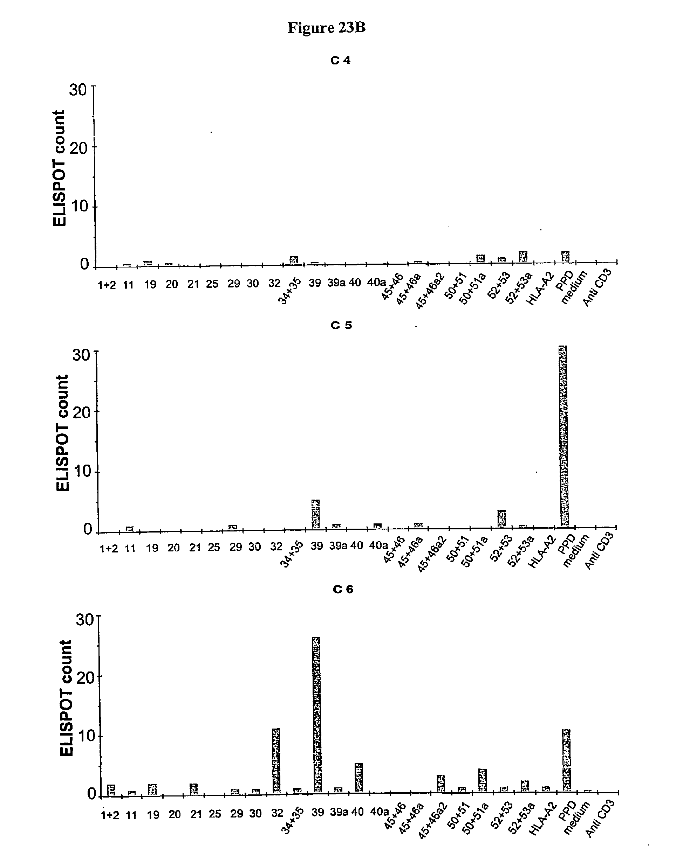

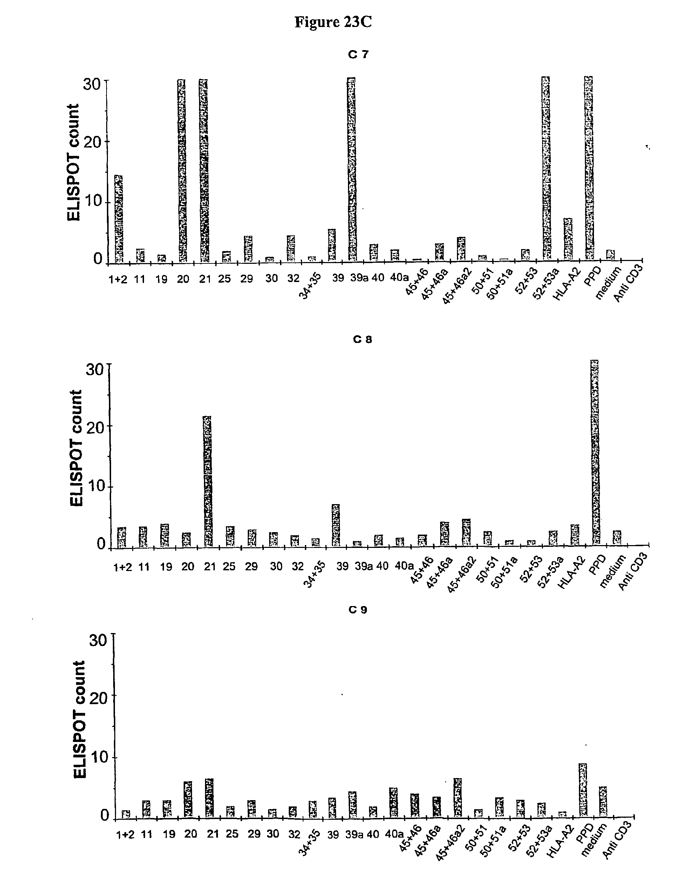

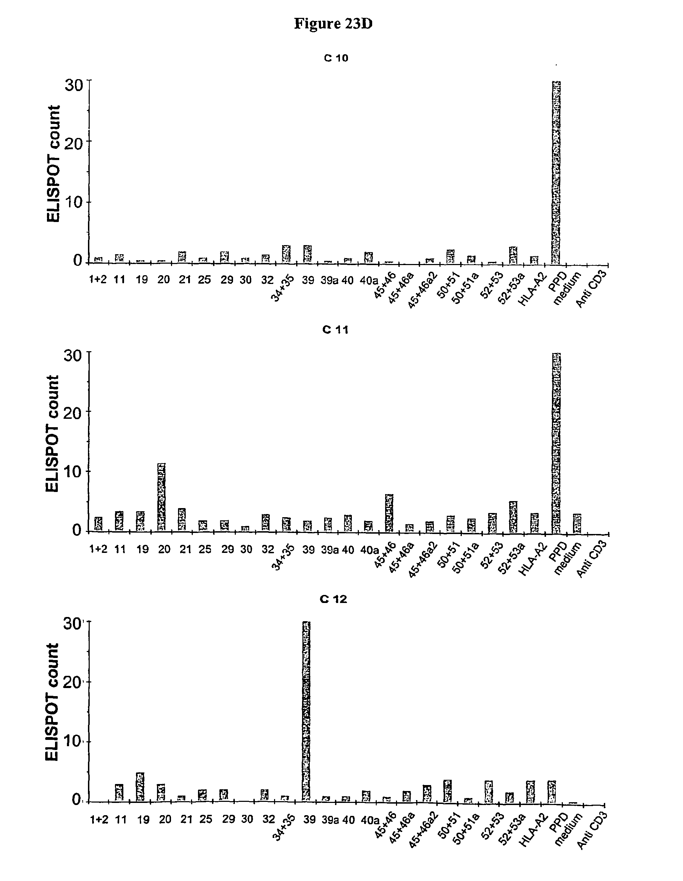

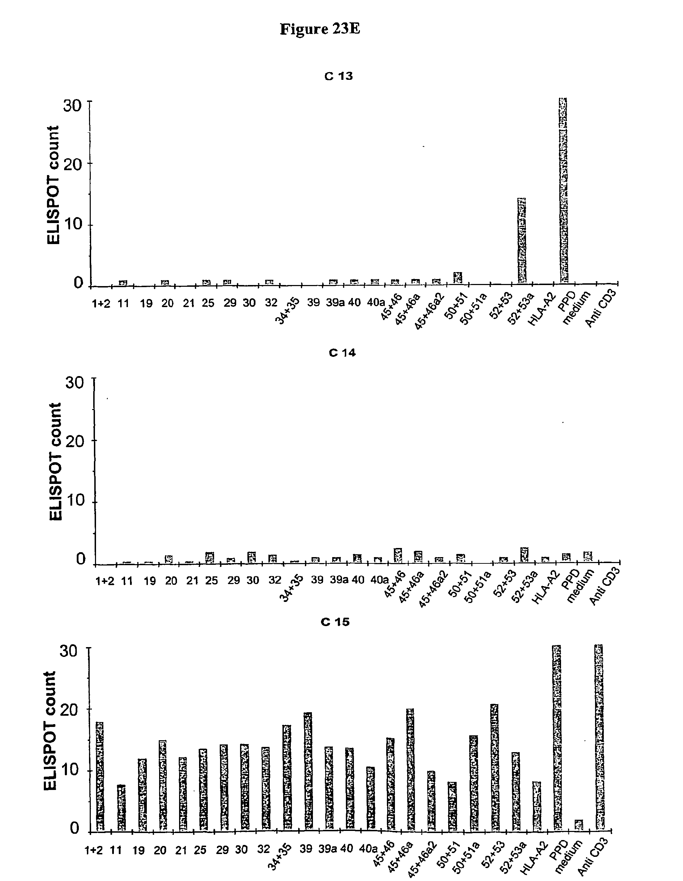

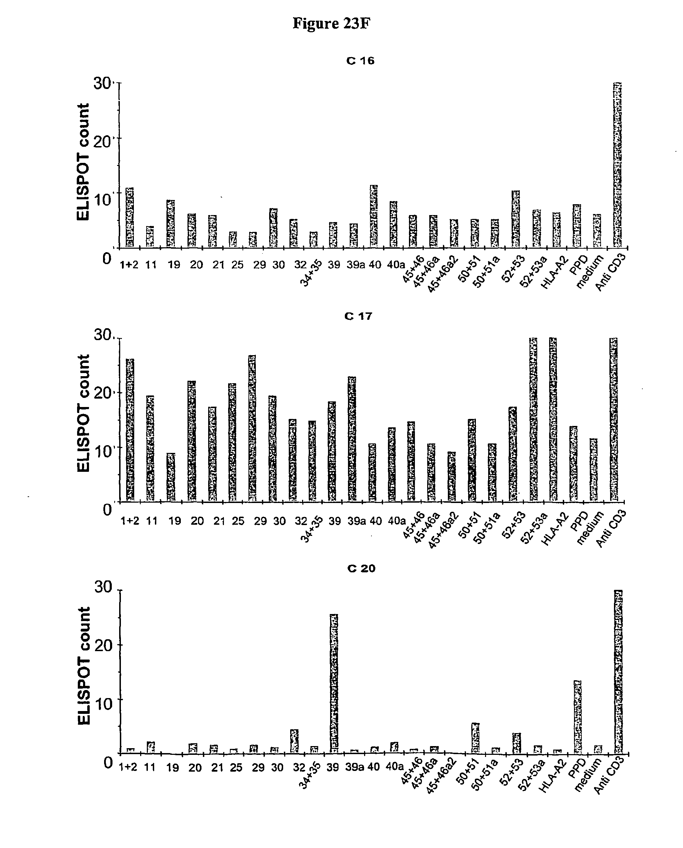

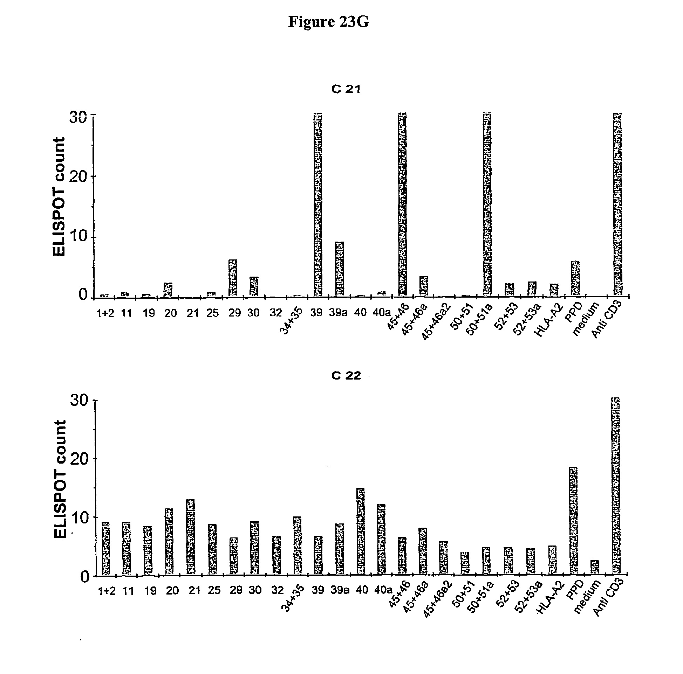

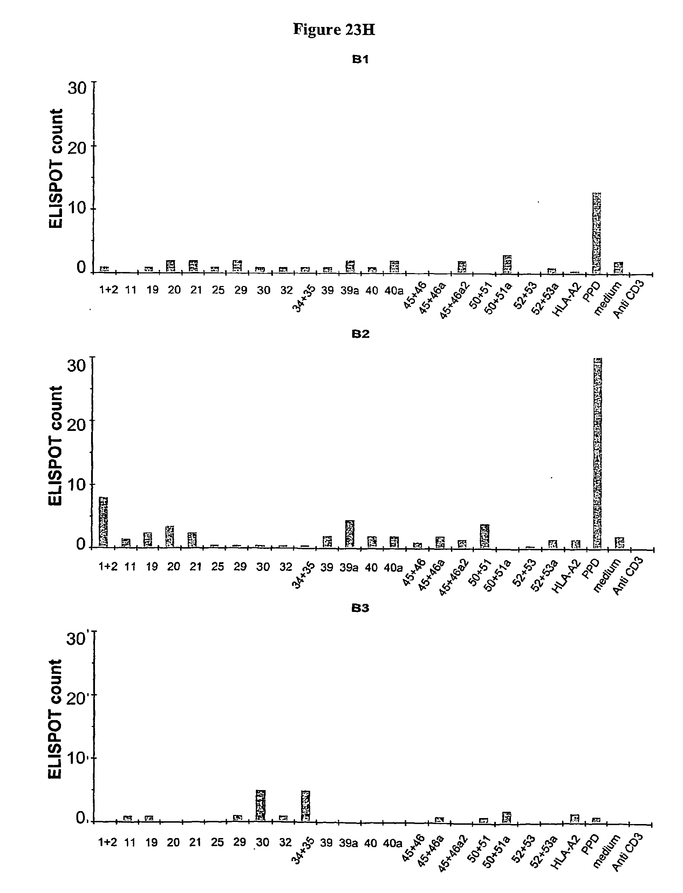

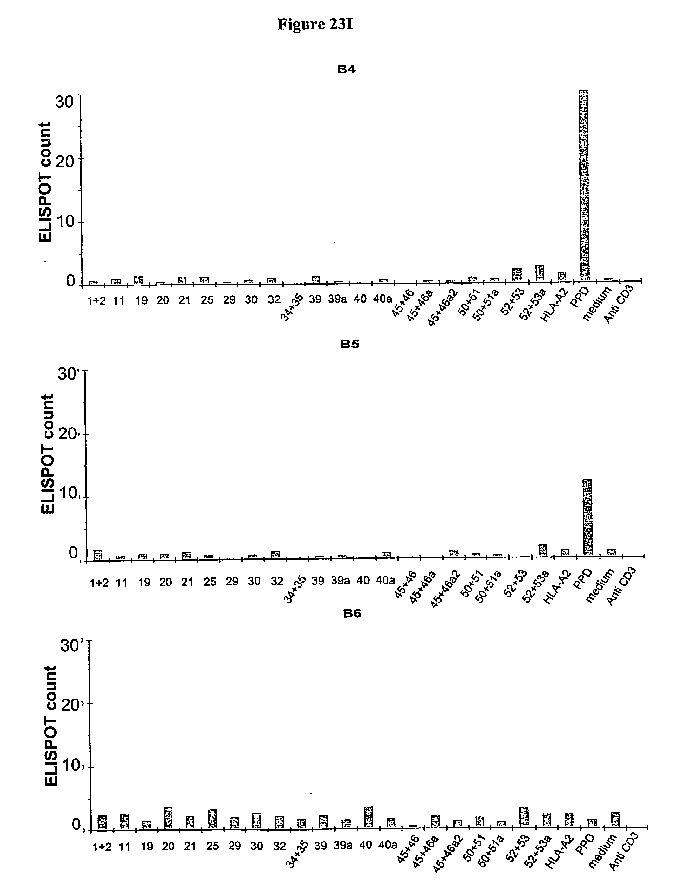

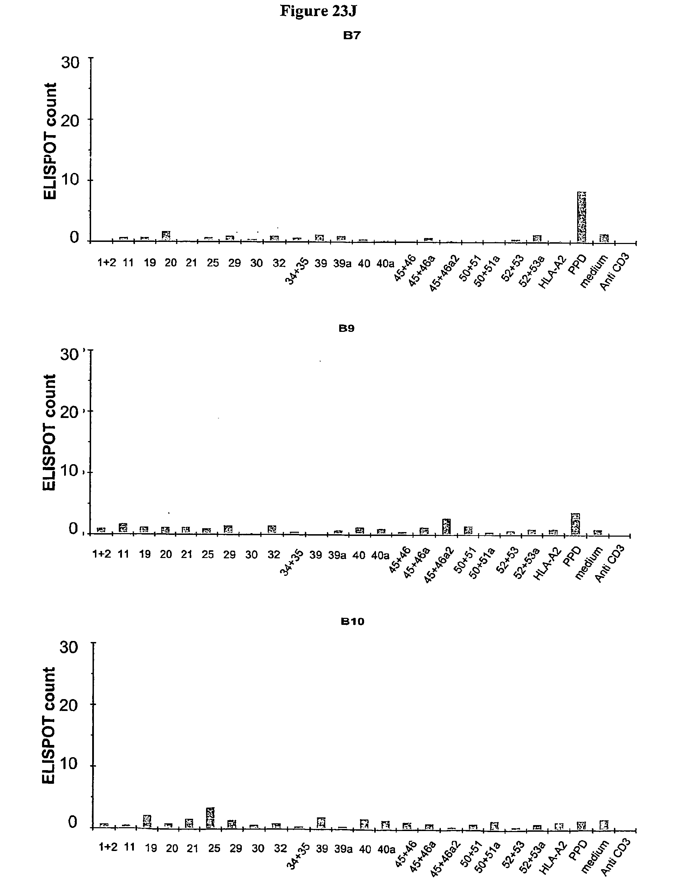

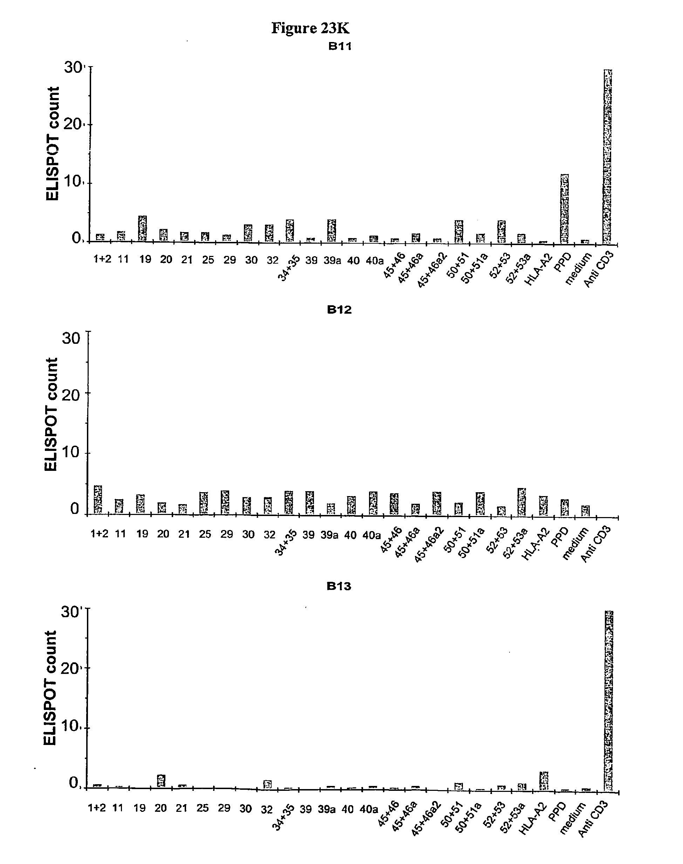

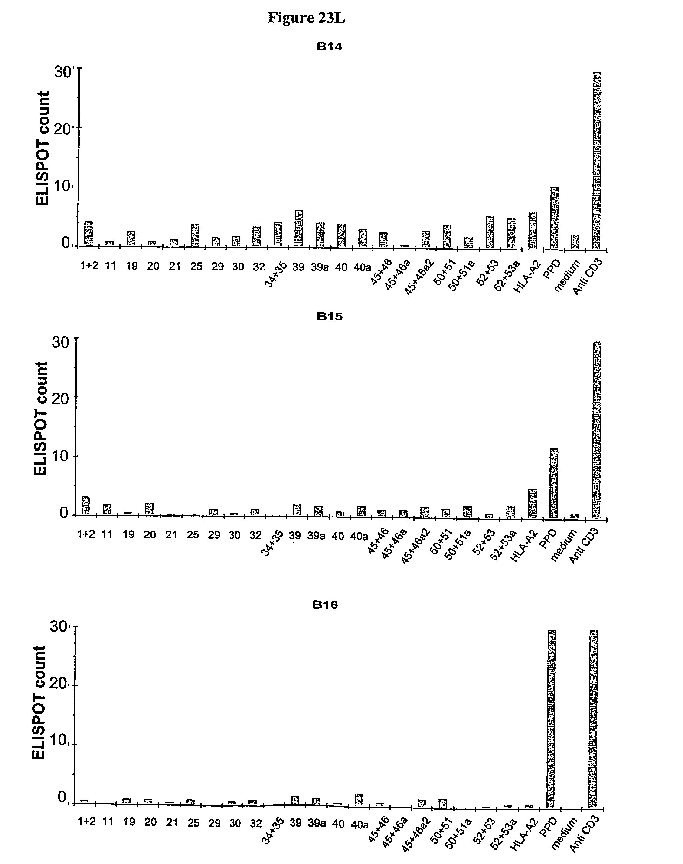

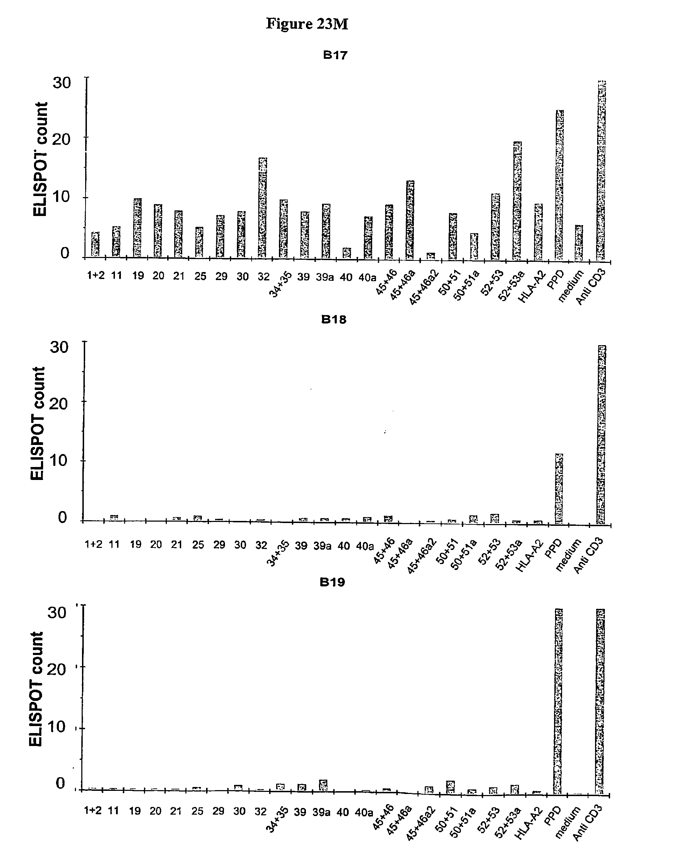

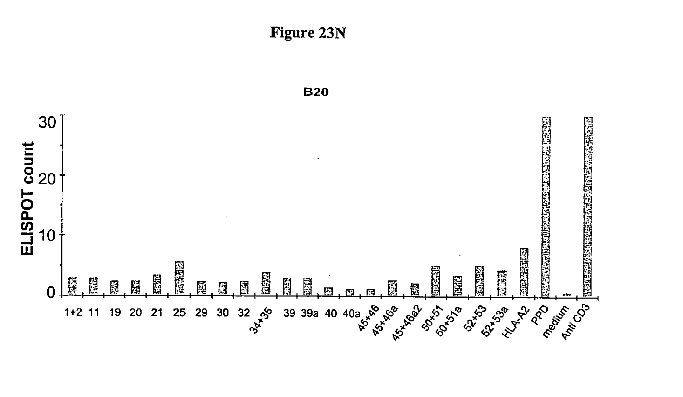

Responses for each peptide were determined by ELISPOT assay for interferon-gamma production as described in Example 1. Peptides were tested at a final concentration of 20µgml¹. ELISPOT numbers were compared between different populations by Mann Whitney U statistic. The number of responders in each group were compared by Fisher's exact test. Results for each series are shown in Figures 22A to C. Individual patient results for series C and B are shown as separate panels in Figures 23A and B respectively.

### Results

### γ-interferon production in response to HLA derived peptides are associated with a history of cGVHD

The responses to peptide are shown as: (mean number of spots per well with specified peptide - mean number of spots with medium control). The medium control in patients was in the range 0-6 per well. A positive result was defined as a mean number of spots per well (corrected for medium control) > 7.5. This was defined by the frequency distribution of responses in peptide containing wells from the control series P (shown in Figure 22A). This allows for the fact that multiple comparisons are made (21 different peptide combinations) in each individual. In the control population, there was no significant difference between responses in peptide-containing wells and response in medium control wells (data not shown). The threshold of 7.5 spots per well gives a probability of <<0.001 of achieving a positive result by chance.

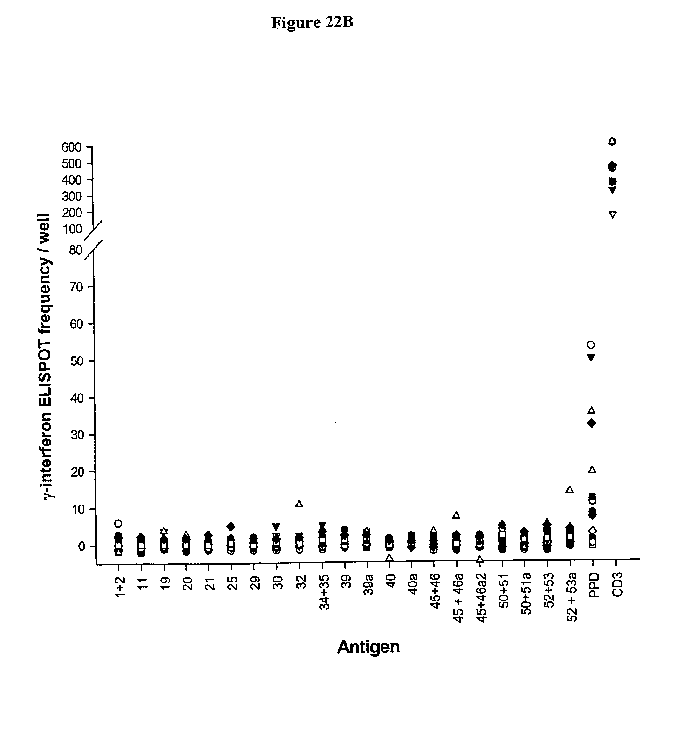

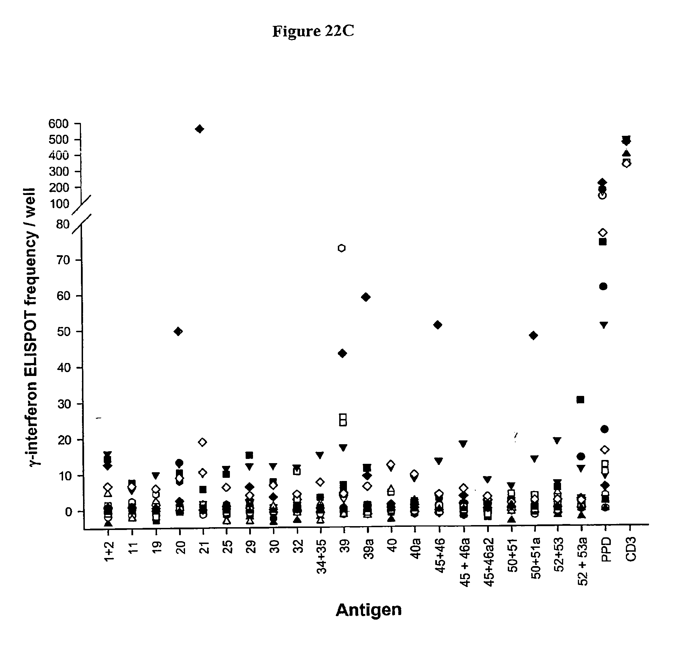

The number of individuals responding to at least one peptide in the ELISPOT assay was 13/21 patients from series C (BMT recipients with cGVHD; Figure 22B), 1/19 patients from series B (BMT recipients without cGVHD; Figure 22C) and 1/30 individuals from control series P (Figure 22A). The number of responders in series C was statistically significantly greater than the other groups (p< 0.0002 for patient series B and p< 0.0001 for control series P).

The medium controls in BMT recipients varied between 0-6 spots /well (5x10⁵ PBMCs). There was no significant difference in the response to stimulation by anti-CD3 with respect to the number of responding cells counted by γ-interferon ELISPOT (patient series C = 393 /well, patient series B = 520 /well & control series P = 318 /well).

The frequency of cells responding to PPD in control series P was 142/5x10⁵, significantly higher than the response in series C (49.6/5x10⁵, (p<10⁻⁷)) and series B (19.8/5x10⁵, (p<10⁴)). 3 individuals in series C and 6 in series B made no detectable response to PPD whereas all 37 individuals in control series P made a response significantly above background. The response to PPD in series C was slightly higher than in series B; it approached statistical significance (p=0.05)

These data show that detection of interferon-gamma production by PBMCs on incubation with HLA derived peptides is a rare event in normal controls under the experimental conditions described above. They also demonstrate that interferon-gamma (γ) production by PBMCs from BMT recipients incubated with HLA derived peptide is associated with a diagnosis of cGVHD. The differences observed are unlikely to be accounted for by discrepancies in the number of lymphocytes with the potential to synthesise interferon- γ given the similar results in all 3 groups following stimulation with anti-CD3. Although there was a slightly higher response to nominal antigen (PPD) in BMT recipients with cGVHD (series C) than in those without (series B), non-specific up-regulation of interferon- γ production in association with disease is unlikely to account for the results because normal controls make excellent responses to PPD without making a response to peptide.

The response to individual peptides was often of high frequency in series C compared to allosensitised dialysis patients and renal transplant recipients (data not shown).

BMT recipients' responses were largely autoimmune, that is to peptides the sequences of which are common to BMT donor and recipient HLA. This contrasts with the findings in allosensitised patients awaiting renal transplantation and in animal models of transplantation in which there is generally a mixture of alloimmune and autoimmune responses.

### Immune responses to α-3 domain peptide analogues

The amino acid sequences of HLA-A & B indicate that for any α-3 domain peptide epitope, the number of alleles are small (between 2 and 4) with relatively limited changes in sequence. For example p39 and p39a1 cover the entire repertoire of HLA-A molecules (and are present in much of HLA-B), they differ in sequence at two consecutive amino acids 193 and 194. Nevertheless patients in series C responded mostly to one or the other peptide, at least with respect to an in vitro assay of polyclonal γ-interferon production. This is observed in four patients (C6, C7, C12 & C20). In contrast, two patients (C21 and C22) demonstrated significant (albeit lower) responses to p39a as well as to p39. This observation also holds true for other α-3 domain peptides to which a response was identified.

In summary this study identifies immune responses to HLA derived peptide epitopes in patients with cGVHD. Therefore there is a clear correlation between GVHD and immune responses to HLA derived peptide epitopes in transplant patients. Therefore the peptides represent biomarkers that can be used for pre-transplantation testing of donors and the post transplant testing of recipients.

### Example 7

Following the preliminary study of Example 5, the inventor has carried out a larger study testing T cell responses to HLA class I peptides in 100 individuals who have received renal transplants more than one year prior to study. The patients were not selected on the basis of renal function. 30 "normal" individuals with no history of allosensitisation were tested as controls. The peptides tested are as shown in Table 8. Responses for each peptide were determined by ELISPOT assay for interferon-gamma production as described in Example 1. Peptides were tested at a final concentration of 20µgml⁻¹.

On the basis of their responses, patients were divided into three groups:
- Red (mean elispot count with at least one peptide > 15) (25 patients)
- Amber (mean elispot count with at least one peptide between 7.5 and 15) (29 patients)
- Green (mean elispot count with any peptide < 7.5) (46 patients).

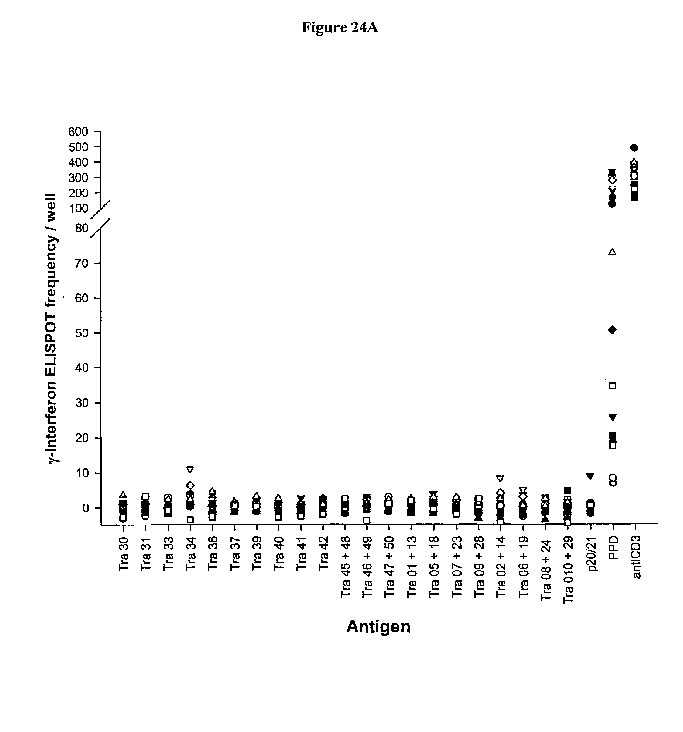

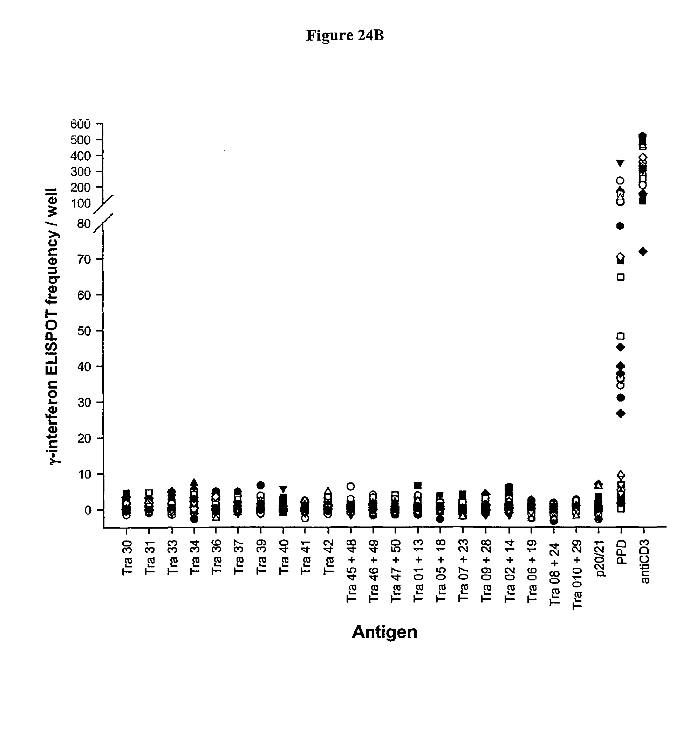

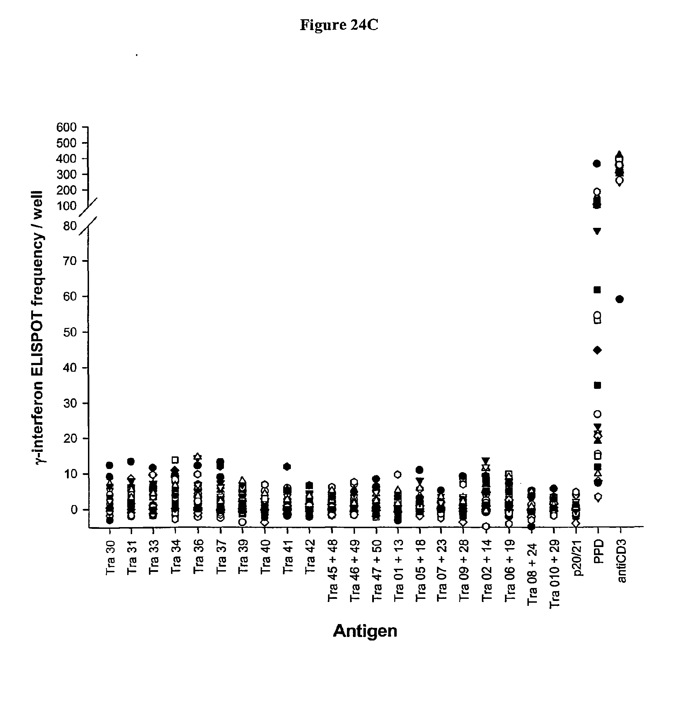

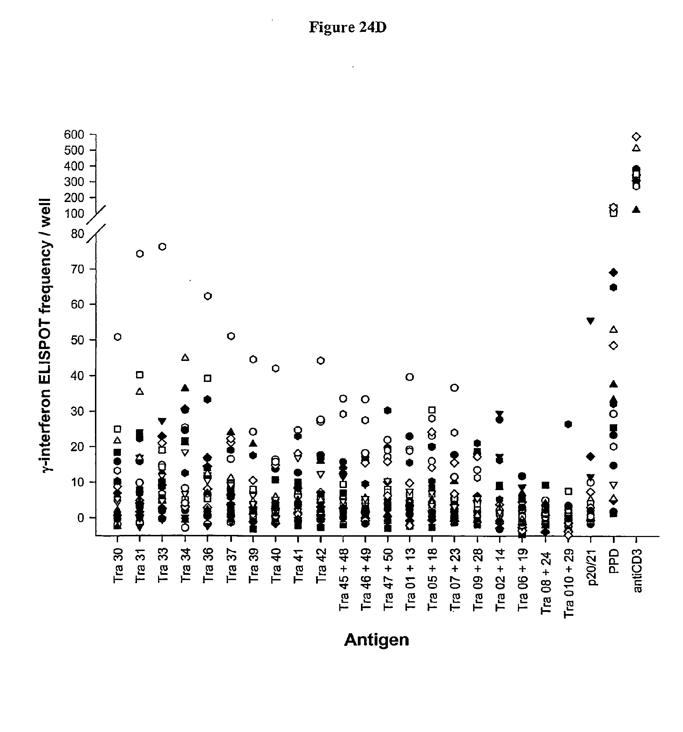

The results are shown in Figures 24A (control), 24B (Green), 24C (Amber) and 24D (Red). As is shown, response to the peptides of the invention is associated with alloimmunity, based on a comparison of the responses in transplant recipients with the control group.

To provide additional information on the association between graft dysfunction and responses to the peptides, the data from the patient groups was further analysed by comparing the number of patients in the Green and Red groups who had undergone a biopsy for chronic allograft dysfunction. This is defined as any renal biopsy taking place >1 year after transplant and is used here as an indicator of incipient graft damage or rejection. It was determined that only 20% of patients in the Green group underwent biopsy compared to 33% in the Red group (p<0.001). Therefore there is a clear correlation between chronic graft injury that indicated the need for further investigation and the subsequent detection of immune responses to HLA derived peptide epitopes in renal transplant patients. Therefore the peptides represent biomarkers that can be used for pre-transplantation testing of donors and the post transplant testing of recipients.

## Claims

1. An *in vitro* method of determining whether an individual is at risk of, or undergoing, graft damage or rejection of immune origin and/or damage of immune origin to non-graft tissue, wherein the graft is an allograft or a xenograft, the method comprising:
(a) testing whether the individual has T cells which respond to a peptide of 9 to 30 amino acids including at least one MHC class II-binding T cell epitope, said peptide comprising a sequence of contiguous amino acids from the α3 domain and/or transmembrane domain of a MHC class I molecule which is recognised by a T cell which recognises an epitope from a MHC class I molecule; and
(b) thereby determining whether the individual is at risk of, or undergoing, graft damage or rejection of immune origin and/or damage of immune origin to non-graft tissue.

2. A method according to claim 1 wherein:
- the individual requires or has received a graft; and/or
- the individual does or does not have an antibody response to a MHC class I molecule; and/or
- the graft damage or rejection is acute or chronic graft damage or rejection; and/or
- the damage of immune origin to non-graft tissue is acute or chronic damage.

3. A method according to claim 1 or 2 wherein a T-cell immune response to said molecule is measured by contacting the peptide with T cells in a sample taken from the subject, under conditions which allow the peptide and the T cells to interact; and determining whether or not any of the T cells are stimulated and thereby determining whether or not a T-cell immune response is present or absent.

4. A method according to any one of the preceding claims wherein the graft is an organ graft or transplant, and optionally wherein the organ is lung, liver, heart, skin, bone marrow or kidney.

5. A method according to any one of the preceding claims, wherein the individual has, is suspected of having, or is considered to be at risk of, chronic graft rejection.

6. A method according to any one of the preceding claims, wherein the graft is bone marrow and wherein the non-graft tissue is at least one selected from liver, skin, mucosa, gastrointestinal tract, lungs, eye, thymus, connective tissue, and exocrine gland.

7. A method according to claim 6, wherein the individual has, is suspected of having, or is considered to be at risk of Graft Versus Host Disease (GVHD), particularly chronic GVHD.

8. A method according to any one of the preceding claims, wherein the MHC class I molecule is HLA-A, HLA-B or HLA-C, and preferably HLA-A2.

9. A method according to any one of the preceding claims which is carried out as defined in claim 3 and the T cells are present in a population of PBMCs isolated from a blood or serum sample taken from the individual.

10. A method according to any one of the preceding claims wherein determining whether T cells of the individual recognise the peptide is carried out by measuring the production of a cytokine by the T cells.

11. The method according to claim 10 wherein the cytokine is interferon-gamma.

12. A method according to claim 10 or 11 wherein the production of cytokine is detected by an ELISPOT assay.

13. A method according to any one of the preceding claims wherein the MHC class I molecule is HLA-A2, and wherein the α3 domain is defined as residues 183 to 274 of SEQ ID NO: 2, and transmembrane domain is defined as residues 275 to 314 of SEQ ID NO: 2.

14. A method according to any one of the preceding claims wherein the peptide consists of the amino acid sequence of any one of SEQ ID NOS: 42, 43, 48, 49, 53 to 74, 97 to 120 and 129 to 132.

## Patentansprüche

1. In-vitro-Verfahren zum Bestimmen, ob für ein Individuum das Risiko einer Transplantatschädigung oder -abstoßung besteht, oder es bereits Transplantatschädigung oder - abstoßung mit Immunursprung und/oder Schädigung an Nicht-Transplantat-Gewebe mit Immunursprung erfährt, wobei das Transplantat ein Allotransplantat oder ein Xenotransplantat ist, wobei das Verfahren umfasst:
(a) Testen, ob das Individuum T-Zellen hat, die auf ein Peptid von 9 bis 30 Aminosäuren, beinhaltend mindestens ein MHC-Klasse-II-bindendes T-Zell-Epitop, antworten, wobei das Peptid eine Sequenz von zusammenhängenden Aminosäuren von der α3-Domäne und/oder der Transmembran-Domäne von einem MHC-Klasse-I-Molekül umfasst, das von einer T-Zelle erkannt wird, die ein Epitop eines MHC-Klasse-I-Moleküls erkennt; und
(b) dadurch Bestimmen, ob für das Individuum das Risiko einer Transplantatschädigung oder -abstoßung besteht, oder es bereits Transplantatschädigung oder -abstoßung mit Immunursprung und/oder Schädigung an Nicht-Transplantat-Gewebe mit Immunursprung erfährt.

2. Verfahren gemäß Anspruch 1, wobei:
- das Individuum ein Transplantat benötigt oder erhalten hat; und/oder
- das Individuum eine Antikörperantwort gegen ein MHC-Klasse-I-Molekül hat oder nicht hat; und/oder
- die Transplantatschädigung oder -abstoßung akute oder chronische Transplantatschädigung oder -abstoßung ist; und/oder
- die Schädigung an Nicht-Transplantat-Gewebe mit Immunursprung akute oder chronische Schädigung ist.

3. Verfahren gemäß Anspruch 1 oder 2, wobei eine T-Zell-Immunantwort gegen das Molekül durch Inkontaktbringen des Peptids mit T-Zellen in einer Probe, entnommen von dem Subjekt, bei Bedingungen, die gestatten, dass das Peptid und die T-Zellen interagieren, gemessen wird; und Bestimmen, ob oder ob nicht eine beliebige der T-Zellen stimuliert wird und dadurch Bestimmen, ob oder ob nicht eine T-Zell-Immunantwort anwesend oder abwesend ist.

4. Verfahren gemäß einem beliebigen der vorangehenden Ansprüche, wobei das Transplantat ein Organ-Transplantat oder Transplantat ist, und gegebenenfalls, wobei das Organ Lunge, Leber, Herz, Haut, Knochenmark oder Niere ist.

5. Verfahren gemäß einem beliebigen der vorangehenden Ansprüche, wobei das Individuum chronische Transplantatabstoßung hat, bei ihm vermutet wird, dass es sie hat, oder man annimmt, dass es ein Risiko dafür hat.

6. Verfahren gemäß einem beliebigen der vorangehenden Ansprüche, wobei das Transplantat Knochenmark ist und wobei das Nicht-Transplantatgewebe mindestens eines ist, ausgewählt aus Leber, Haut, Schleimhäute, Magen-Darm-Trakt, Lungen, Auge, Thymus, Bindegewebe und exokriner Drüse.

7. Verfahren gemäß Anspruch 6, wobei das Individuum Graftversus-Host-Erkrankung (GVHD), speziell chronische GVHD hat, man annimmt, dass es sie hat, oder ein Risiko besteht, dass es sie hat.

8. Verfahren gemäß einem beliebigen der vorangehenden Ansprüche, wobei das MHC-Klasse-I-Molekül HLA-A, HLA-B oder HLA-C, und bevorzugt HLA-A2, ist.

9. Verfahren gemäß einem beliebigen der vorangehenden Ansprüche, das wie in Anspruch 3 definiert ausgeführt wird und wobei die T-Zellen in einer Population von PBMCs anwesend sind, die aus einer Blut- oder Serum-Probe, die von dem Individuum entnommen wurde, isoliert wurden.

10. Verfahren gemäß einem beliebigen der vorangehenden Ansprüche, wobei das Bestimmen, ob T-Zellen des Individuums das Peptid erkennen durch Messen der Produktion eines Cytokins durch die T-Zellen durchgeführt wird.

11. Verfahren gemäß Anspruch 10, wobei das Cytokin Interferon-gamma ist.

12. Verfahren gemäß Anspruch 10 oder 11, wobei die Produktion von Cytokin durch einen ELISPOT-Assay nachgewiesen wird.

13. Verfahren gemäß einem beliebigen der vorangehenden Ansprüche, wobei das MHC-Klasse-I-Molekül HLA-A2 ist und wobei die α3-Domäne als Reste 183 bis 274 von SEQ ID NO:2 definiert ist und die Transmembran-Domäne als Reste 275 bis 314 von SEQ ID NO:2 definiert ist.

14. Verfahren gemäß einem beliebigen der vorangehenden Ansprüche, wobei das Peptid aus der Aminosäuresequenz einer beliebigen von SEQ ID NOS:42, 43, 48, 49, 53 bis 74, 97 bis 120 und 129 bis 132 besteht.

## Revendications

1. Procédé *in vitro* permettant de déterminer si un individu présente un risque de subir ou est en train de subir l'endommagement ou le rejet immunitaire d'un greffon et/ou l'endommagement immunitaire de tissu ne faisant pas partie du greffon, la greffe étant une allogreffe ou une xénogreffe, lequel procédé comporte les étapes suivantes :
a) faire un test pour savoir si l'individu possède des lymphocytes T qui répondent à un peptide comportant de 9 à 30 résidus d'acides aminés et incluant au moins un épitope pour lymphocyte T se liant aux molécules de classe II du CMH, lequel peptide comprend une séquence d'acides aminés contigus provenant du domaine α3 et/ou du domaine transmembranaire d'une molécule de classe I du CMH, reconnue par un lymphocyte T qui reconnaît un épitope provenant d'une molécule de classe I du CMH ;
b) et déterminer par là si l'individu présente un risque de subir ou est en train de subir l'endommagement ou le rejet immunitaire d'un greffon et/ou l'endommagement immunitaire de tissu ne faisant pas partie du greffon.

2. Procédé conforme à la revendication 1, dans lequel
- l'individu nécessite une greffe ou en a reçu une ;
- et/ou l'individu présente ou non une réponse par anticorps à une molécule de classe I du CMH ;
- et/ou l'endommagement ou le rejet immunitaire du greffon est un endommagement ou un rejet chronique ou aigu du greffon ;
- et/ou l'endommagement immunitaire de tissu ne faisant pas partie du greffon est un endommagement chronique ou aigu.

3. Procédé conforme à la revendication 1 ou 2, dans lequel on mesure une réponse immunitaire par lymphocytes T à ladite molécule en mettant le peptide en contact avec des lymphocytes T dans un échantillon prélevé chez le sujet, dans des conditions qui autorisent une interaction du peptide et des lymphocytes T, et en déterminant si, oui ou non, certains des lymphocytes T sont stimulés, et par là, s'il y a ou non une réponse immunitaire par lymphocytes T.

4. Procédé conforme à l'une des revendications précédentes, dans lequel la greffe est une greffe ou une transplantation d'organe, et en option, l'organe est un poumon, un foie, un coeur, de la peau, de la moelle osseuse ou un rein.

5. Procédé conforme à l'une des revendications précédentes, dans lequel l'individu est atteint ou est suspecté d'être atteint de rejet chronique de greffon, ou est considéré comme présentant un certain risque de rejet chronique de greffon.

6. Procédé conforme à l'une des revendications précédentes, dans lequel le greffon est de la moelle osseuse et le tissu ne faisant pas partie du greffon est un tissu choisi, au nombre d'au moins un, parmi les suivants : foie, peau, muqueuses, tractus gastro-intestinal, poumons, oeil, thymus, tissu conjonctif et glandes exocrines.

7. Procédé conforme à la revendication 6, dans lequel l'individu est atteint ou est suspecté d'être atteint de réaction du greffon contre l'hôte, ou est considéré comme présentant un certain risque de réaction du greffon contre l'hôte (RGCH), en particulier de réaction chronique du greffon contre l'hôte.

8. Procédé conforme à l'une des revendications précédentes, dans lequel la molécule de classe I du CMH est une molécule HLA-A, HLA-B ou HLA-C, et de préférence la molécule HLA-A2.

9. Procédé conforme à l'une des revendications précédentes, lequel est mis en oeuvre de la manière indiquée dans la revendication 3 et dans lequel les lymphocytes T se trouvent au sein d'une population de cellules mononucléaires de sang périphérique, isolée à partir d'un échantillon de sang ou de sérum prélevé chez l'individu.

10. Procédé conforme à l'une des revendications précédentes, dans lequel on détermine si les lymphocytes T de l'individu reconnaissent le peptide en évaluant la production d'une cytokine par les lymphocytes T.

11. Procédé conforme à la revendication 10, dans lequel la cytokine est l'interféron γ.

12. Procédé conforme à la revendication 10 ou 11, dans lequel la production de cytokine est détectée par dosage ELISPOT.

13. Procédé conforme à l'une des revendications précédentes, dans lequel la molécule de classe I du CMH est la molécule HLA-A2, et le domaine α3 est défini comme étant constitué des résidus n° 183 à 274 de la Séquence N° 2 et le domaine transmbranaire est défini comme étant constitué des résidus n° 275 à 314 de la Séquence N° 2.

14. Procédé conforme à l'une des revendications précédentes, dans lequel le peptide est constitué de l'une des séquences de résidus d'acides aminés données comme étant les Séquences N° 42, N° 43, N° 48, N° 49, N° 53 à N° 74, N° 97 à N° 120, et N° 129 à N° 132.


### SEQUENCE LISTING

<110> Circassia Limited
   The University of Birmingham
<120> PROGNOSTIC ASSAY
<130> N103191A WO SA/GML
<150> PCT/GB07/01690
   <151> 2007-05-09
<150> GB 0715949.4
   <151> 2007-08-15
<150> GB 0722378.7
   <151> 2007-11-14
<160> 132
<170> PatentIn version 3.2
<210> 1
   <211> 365
   <212> PRT
   <213> Homo sapiens
<400> 1
<210> 2
   <211> 341
   <212> PRT
   <213> Homo sapiens
<400> 2
   Gly Ser His Ser Met Arg Tyr Phe Phe Thr Ser Val Ser Arg Pro Gly
   1               5                   10                  15
   Arg Gly Glu Pro Arg Phe Ile Ala Val Gly Tyr Val Asp Asp Thr Gln
               20                  25                  30
   Phe Val Arg Phe Asp Ser Asp Ala Ala Ser Gln Arg Met Glu Pro Arg
           35                  40                  45
   Ala Pro Trp Ile Glu Gln Glu Gly Pro Glu Tyr Trp Asp Gly Glu Thr
       50                  55                  60
   Arg Lys Val Lys Ala His Ser Gln Thr His Arg Val Asp Leu Gly Thr
   65                  70                  75                  80
   Leu Arg Gly Tyr Tyr Asn Gln Ser Glu Ala Gly Ser His Thr Val Gln
                   85                  90                  95
   Arg Met Tyr Gly Cys Asp Val Gly Ser Asp Trp Arg Phe Leu Arg Gly
               100                 105                 110
   Tyr His Gln Tyr Ala Tyr Asp Gly Lys Asp Tyr Ile Ala Leu Lys Glu
           115                 120                 125
   Asp Leu Arg Ser Trp Thr Ala Ala Asp Met Ala Ala Gln Thr Thr Lys
       130                 135                 140
   His Lys Trp Glu Ala Ala His Val Ala Glu Gln Leu Arg Ala Tyr Leu
   145                 150                 155                 160
   Glu Gly Thr Cys Val Glu Trp Leu Arg Arg Tyr Leu Glu Asn Gly Lys
                   165                 170                 175
   Glu Thr Leu Gln Arg Thr Asp Ala Pro Lys Thr His Met Thr His His
               180                 185                 190
   Ala Val Ser Asp His Glu Ala Thr Leu Arg Cys Trp Ala Leu Ser Phe
           195                 200                 205
   Tyr Pro Ala Glu Ile Thr Leu Thr Trp Gln Arg Asp Gly Glu Asp Gln
       210                 215                 220
   Thr Gln Asp Thr Glu Leu Val Glu Thr Arg Pro Ala Gly Asp Gly Thr
   225                 230                 235                 240
   Phe Gln Lys Trp Ala Ala Val Val Val Pro Ser Gly Gln Glu Gln Arg
                   245                 250                 255
   Tyr Thr Cys His Val Gln His Glu Gly Leu Pro Lys Pro Leu Thr Leu
               260                 265                 270
   Arg Trp Glu Pro Ser Ser Gln Pro Thr Ile Pro Ile Val Gly Ile Ile
           275                 280                 285
   Ala Gly Leu Val Leu Phe Gly Ala Val Ile Thr Gly Ala Val Val Ala
       290                 295                 300
   Ala Val Met Trp Arg Arg Lys Ser Ser Asp Arg Lys Gly Gly Ser Tyr
   305                 310                 315                 320
   Ser Gln Ala Ala Ser Ser Asp Ser Ala Gln Gly Ser Asp Val Ser Leu
                   325                 330                 335
   Thr Ala Cys Lys Val
               340
<210> 3
   <211> 3287
   <212> DNA
   <213> Homo sapiens
<400> 3
   ggattcccca actccgcagt ttcttttctc cctctcccaa cctatgtagg gtccttcttc     60
   ctggatactc acgacgcgga cccagttctc actcccatcg ggtgtcgggt ttccagagaa    120
   gccaatcagt gtcgtcgcgg tcgcggttct aaagtccgca cgcacccacc gggactcaga    180
   ttctccccag acgccgagga tggccgtcat ggcgccccga accctcgtcc tgctactctc    240
   gggggctctg gccctgaccc agacctgggc gggtgagtgc ggggtcggga gggaaacggc    300
   ctctgtgggg agaagcaacg ggcccgcctg gcgggggcgc aggacccggg aagccgcgcc    360
   gggaggaggg tcgggcgggt ctcagccact cctcgtcccc aggctctcac tccatgaggt    420
   atttcttcac atccgtgtcc cggcccggcc gcggggagcc ccgcttcatc gcagtgggct    480
   acgtggacga cacgcagttc gtgcggttcg acagcgacgc cgcgagccag aggatggagc    540
   cgcgggcgcc gtggatagag caggagggtc cggagtattg ggacggggag acacggaaag    600
   tgaaggccca ctcacagact caccgagtgg acctggggac cctgcgcggc tactacaacc    660
   agagcgaggc cggtgagtga ccccggcccg gggcgcaggt cacgacctct catcccccac    720
   ggacgggcca ggtcgcccac agtctccggg tccgagatcc gccccgaagc cgcgggaccc    780
   cgagaccctt gccccgggag aggcccaggc gcctttaccc ggtttcattt tcagtttagg    840
   ccaaaaatcc ccccaggttg gtcggggcgg ggcggggctc gggggaccgg gctgaccgcg    900
   gggtccgggc caggttctca caccgtccag aggatgtatg gctgcgacgt ggggtcggac    960
   tggcgcttcc tccgcgggta ccaccagtac gcctacgacg gcaaggatta catcgccctg   1020
   aaagaggacc tgcgctcttg gaccgcggcg gacatggcag ctcagaccac caagcacaag   1080
   tgggaggcgg cccatgtggc ggagcagttg agagcctacc tggagggcac gtgcgtggag   1140
   tggctccgca gatacctgga gaacgggaag gagacgctgc agcgcacggg taccaggggc   1200
   cacggggcgc ctccctgatc gcctgtagat ctcccgggct ggcctcccac aaggagggga   1260
   gacaattggg accaacacta gaatatcgcc ctccctctgg tcctgaggga gaggaatcct   1320
   cctgggtttc cagatcctgt accagagagt gactctgagg ttccgccctg ctctctgaca   1380
   caattaaggg ataaaatctc tgaaggaatg acgggaagac gatccctcga atactgatga   1440
   gtggttccct ttgacacaca caggcagcag ccttgggccc gtgacttttc ctctcaggcc   1500
   ttgttctctg cttcacactc aatgtgtgtg ggggtctgag tccagcactt ctgagtcctt   1560
   cagcctccac tcaggtcagg accagaagtc gctgttccct cttcagggac tagaattttc   1620
   cacggaatag gagattatcc caggtgcctg tgtccaggct ggtgtctggg ttctgtgctc   1680
   ccttccccat cccaggtgtc ctgtccattc tcaagatagc cacatgtgtg ctggaggagt   1740
   gtcccatgac agatgcaaaa tgcctgaatg atctgactct tcctgacaga cgcccccaaa   1800
   acgcatatga ctcaccacgc tgtctctgac catgaagcca ccctgaggtg ctgggccctg   1860
   agcttctacc ctgcggagat cacactgacc tggcagcggg atggggagga ccagacccag   1920
   gacacggagc tcgtggagac caggcctgca ggggatggaa ccttccagaa gtgggcggct   1980
   gtggtggtgc cttctggaca ggagcagaga tacacctgcc atgtgcagca tgagggtttg   2040
   cccaagcccc tcaccctgag atggggtaag gagggagacg ggggtgtcat gtcttttagg    2100
   gaaagcagga gcctctctga cctttagcag ggtcagggcc cctcaccttc ccctcttttc   2160
   ccagagccgt cttcccagcc caccatcccc atcgtgggca tcattgctgg cctggttctc   2220
   tttggagctg tgatcactgg agctgtggtc gctgctgtga tgtggaggag gaagagctca   2280
   ggtggggaag ggtgaagggg tgggtctgag atttcttgtc tcactgaggg ttccaagacc    2340
   caggtagaag tgtgccctgc ctcgttactg ggaagcacca cccacaatta tgggcctacc  2400
   cagcctgggc cctgtgtgcc agcacttact cttttgtaaa gcacctgtta aaatgaagga   2460
   cagatttatc accttgatta cagcggtgat gggacctgat cccagcagtc acaagtcaca   2520
   ggggaaggtc cctgaggacc ttcaggaggg cggttggtcc aggacccaca cctgctttct   2580
   tcatgtttcc tgatcccgcc ctgggtctgc agtcacacat ttctggaaac ttctctgagg   2640
   tccaagactt ggaggttcct ctaggacctt aaggccctga ctcctttctg gtatctcaca   2700
   ggacattttc ttcccacaga tagaaaagga gggagctact ctcaggctgc aagtaagtat   2760
   gaaggaggct gatgcctgag gtccttggga tattgtgttt gggagcccat gggggagctc   2820
   acccacccca caattcctcc tctagccaca tcttctgtgg gatctgacca ggttctgttt   2880
   ttgttctacc ccaggcagtg acagtgccca gggctctgat gtgtctctca cagcttgtaa   2940
   aggtgagagc ctggagggcc tgatgtgtgt tgggtgttgg gcggaacagt ggacacagct   3000
   gtgctatggg gtttctttcc attggatgta ttgagcatgc gatgggctgt ttaaagtgtg   3060
   acccctcact gtgacagata cgaatttgtt catgaatatt tttttctata gtgtgagaca   3120
   gctgccttgt gtgggactga gaggcaagag ttgttcctgc ccttcccttt gtgacttgaa   3180
   gaaccctgac tttgtttctg caaaggcacc tgcatgtgtc tgtgttcgtg taggcataat   3240
   gtgaggaggt ggggagacca ccccaccccc atgtccacca tgaccct                  3287
<210> 4
   <211> 15
   <212> PRT
   <213> Artificial sequence
<220>
   <223> Sequence derived from HLA-A2
<400> 4
   His Ser Met Arg Tyr Phe Phe Thr Ser Val Ser Arg Pro Gly Arg
   1 5 10 15
<210> 5
   <211> 15
   <212> PRT
   <213> Artificial sequence
<220>
   <223> Sequence derived from HLA-A2
<400> 5
   Met Arg Tyr Phe Phe Thr Ser Val Ser Arg Pro Gly Arg Gly Glu
   1 5 10 15
<210> 6
   <211> 15
   <212> PRT
   <213> Artificial sequence
<220>
   <223> Sequence derived from HLA-A2
<400> 6
   Thr Ser Val Ser Arg Pro Gly Arg Gly Glu Pro Arg Phe Ile Ala
   1 5 10 15
<210> 7
   <211> 15
   <212> PRT
   <213> Artificial sequence
<220>
   <223> Sequence derived from HLA-A2
<400> 7
   Pro Arg Phe Ile Ala Val Gly Tyr Val Asp Asp Thr Gln Phe Val
   1 5 10 15
<210> 8
   <211> 15
   <212> PRT
   <213> Artificial sequence
<220>
   <223> Sequence derived from HLA-A2
<400> 8
   Ile Ala Val Gly Tyr Val Asp Asp Thr Gln Phe Val Arg Phe Asp
   1 5 10 15
<210> 9
   <211> 15
   <212> PRT
   <213> Artificial sequence
<220>
   <223> Sequence derived from HLA-A2
<400> 9
   Val Gly Tyr Val Asp Asp Thr Gln Phe Val Arg Phe Asp Ser Asp
   1 5 10 15
<210> 10
   <211> 15
   <212> PRT
   <213> Artificial sequence
<220>
   <223> Sequence derived from HLA-A2
<400> 10
   Thr Gln Phe Val Arg Phe Asp Ser Asp Ala Ala Ser Gln Arg Met
   1 5 10 15
<210> 11
   <211> 15
   <212> PRT
   <213> Artificial sequence
<220>
   <223> Sequence derived from HLA-A2
<400> 11
   Val Arg Phe Asp Ser Asp Ala Ala Ser Gln Arg Met Glu Pro Arg
   1 5 10 15
<210> 12
   <211> 16
   <212> PRT
   <213> Artificial sequence
<220>
   <223> Sequence derived from HLA-A2
<400> 12
   Ser Gln Arg Met Glu Pro Arg Ala Pro Trp Ile Glu Gln Glu Gly Pro
   1 5 10 15
<210> 13
   <211> 15
   <212> PRT
   <213> Artificial sequence
<220>
   <223> Sequence derived from HLA-A2
<400> 13
   Ala Pro Trp Ile Glu Gln Glu Gly Pro Glu Tyr Trp Asp Gly Glu
   1               5                   10                  15
<210> 14
   <211> 15
   <212> PRT
   <213> Artificial sequence
<220>
   <223> Sequence derived from HLA-A2
<400> 14
   Pro Glu Tyr Trp Asp Gly Glu Thr Arg Lys Val Lys Ala His Ser
   1               5                   10                  15
<210> 15
   <211> 15
   <212> PRT
   <213> Artificial sequence
<220>
   <223> Sequence derived from HLA-A2
<400> 15
   Arg Lys Val Lys Ala His Ser Gln Thr His Arg Val Asp Leu Gly
   1               5                   10                  15
<210> 16
   <211> 15
   <212> PRT
   <213> Artificial sequence
<220>
   <223> Sequence derived from HLA-A2
<400> 16
   His Arg Val Asp Leu Gly Thr Leu Arg Gly Tyr Tyr Asn Gln Ser
   1               5                   10                  15
<210> 17
   <211> 15
   <212> PRT
   <213> Artificial sequence
<220>
   <223> Sequence derived from HLA-A2
<400> 17
   Val Asp Leu Gly Thr Leu Arg Gly Tyr Tyr Asn Gln Ser Glu Ala
   1 5 10 15
<210> 18
   <211> 15
   <212> PRT
   <213> Artificial sequence
<220>
   <223> Sequence derived from HLA-A2
<400> 18
   Gly Thr Leu Arg Gly Tyr Tyr Asn Gln Ser Glu Ala Gly Ser His
   1 5 10 15
<210> 19
   <211> 15
   <212> PRT
   <213> Artificial sequence
<220>
   <223> Sequence derived from HLA-A2
<400> 19
   Arg Gly Tyr Tyr Asn Gln Ser Glu Ala Gly Ser His Thr Val Gln
   1 5 10 15
<210> 20
   <211> 15
   <212> PRT
   <213> Artificial sequence
<220>
   <223> Sequence derived from HLA-A2
<400> 20
   His Thr Val Gln Arg Met Tyr Gly Cys Asp Val Gly Ser Asp Trp
   1 5 10 15
<210> 21
   <211> 16
   <212> PRT
   <213> Artificial sequence
<220>
   <223> Sequence derived from HLA-A2
<400> 21
   Val Gln Arg Met Tyr Gly Cys Asp Val Gly Ser Asp Trp Arg Phe Leu
   1 5 10 15
<210> 22
   <211> 15
   <212> PRT
   <213> Artificial sequence
<220>
   <223> Sequence derived from HLA-A2
<400> 22
   Cys Asp Val Gly Ser Asp Trp Arg Phe Leu Arg Gly Tyr His Gln
   1 5 10 15
<210> 23
   <211> 15
   <212> PRT
   <213> Artificial sequence
<220>
   <223> Sequence derived from HLA-A2
<400> 23
   Ser Asp Trp Arg Phe Leu Arg Gly Tyr His Gln Tyr Ala Tyr Asp
   1 5 10 15
<210> 24
   <211> 15
   <212> PRT
   <213> Artificial sequence
<220>
   <223> Sequence derived from HLA-A2
<400> 24
   Trp Arg Phe Leu Arg Gly Tyr His Gln Tyr Ala Tyr Asp Gly Lys
   1 5 10 15
<210> 25
   <211> 15
   <212> PRT
   <213> Artificial sequence
<220>
   <223> Sequence derived from HLA-A2
<400> 25
   Arg Gly Tyr His Gln Tyr Ala Tyr Asp Gly Lys Asp Tyr Ile Ala
   1 5 10 15
<210> 26
   <211> 15
   <212> PRT
   <213> Artificial sequence
<220>
   <223> Sequence derived from HLA-A2
<400> 26
   His Gln Tyr Ala Tyr Asp Gly Lys Asp Tyr Ile Ala Leu Lys Glu
   1 5 10 15
<210> 27
   <211> 15
   <212> PRT
   <213> Artificial sequence
<220>
   <223> Sequence derived from HLA-A2
<400> 27
   Tyr Ala Tyr Asp Gly Lys Asp Tyr Ile Ala Leu Lys Glu Asp Leu
   1 5 10 15
<210> 28
   <211> 15
   <212> PRT
   <213> Artificial sequence
<220>
   <223> Sequence derived from HLA-A2
<400> 28
   Lys Asp Tyr Ile Ala Leu Lys Glu Asp Leu Arg Ser Trp Thr Ala
   1 5 10 15
<210> 29
   <211> 15
   <212> PRT
   <213> Artificial sequence
<220>
   <223> Sequence derived from HLA-A2
<400> 29
   Ile Ala Leu Lys Glu Asp Leu Arg Ser Trp Thr Ala Ala Asp Met
   1 5 10 15
<210> 30
   <211> 15
   <212> PRT
   <213> Artificial sequence
<220>
   <223> Sequence derived from HLA-A2
<400> 30
   Glu Asp Leu Arg Ser Trp Thr Ala Ala Asp Met Ala Ala Gln Thr
   1 5 10 15
<210> 31
   <211> 15
   <212> PRT
   <213> Artificial sequence
<220>
   <223> Sequence derived from HLA-A2
<400> 31
   Arg Ser Trp Thr Ala Ala Asp Met Ala Ala Gln Thr Thr Lys His
   1 5 10 15
<210> 32
   <211> 15
   <212> PRT
   <213> Artificial sequence
<220>
   <223> Sequence derived from HLA-A2
<400> 32
   Ala Asp Met Ala Ala Gln Thr Thr Lys His Lys Trp Glu Ala Ala
   1 5 10 15
<210> 33
   <211> 15
   <212> PRT
   <213> Artificial sequence
<220>
   <223> Sequence derived from HLA-A2
<400> 33
   His Lys Trp Glu Ala Ala His Val Ala Glu Gln Leu Arg Ala Tyr
   1 5 10 15
<210> 34
   <211> 15
   <212> PRT
   <213> Artificial sequence
<220>
   <223> Sequence derived from HLA-A2
<400> 34
   Ala His Val Ala Glu Gln Leu Arg Ala Tyr Leu Glu Gly Thr Cys
   1               5                   10                  15
<210> 35
   <211> 15
   <212> PRT
   <213> Artificial sequence
<220>
   <223> Sequence derived from HLA-A2
<400> 35
   Glu Gln Leu Arg Ala Tyr Leu Glu Gly Thr Cys Val Glu Trp Leu
   1               5                   10                  15
<210> 36
   <211> 15
   <212> PRT
   <213> Artificial sequence
<220>
   <223> Sequence derived from HLA-A2
<400> 36
   Arg Ala Tyr Leu Glu Gly Thr Cys Val Glu Trp Leu Arg Arg Tyr
   1               5                   10                  15
<210> 37
   <211> 15
   <212> PRT
   <213> Artificial sequence
<220>
   <223> Sequence derived from HLA-A2
<400> 37
   Thr Cys Val Glu Trp Leu Arg Arg Tyr Leu Glu Asn Gly Lys Glu
   1               5                   10                  15
<210> 38
   <211> 15
   <212> PRT
   <213> Artificial sequence
<220>
   <223> Sequence derived from HLA-A2
<400> 38
   Val Glu Trp Leu Arg Arg Tyr Leu Glu Asn Gly Lys Glu Thr Leu
   1               5                   10                  15
<210> 39
   <211> 15
   <212> PRT
   <213> Artificial sequence
<220>
   <223> Sequence derived from HLA-A2
<400> 39
   Arg Arg Tyr Leu Glu Asn Gly Lys Glu Thr Leu Gln Arg Thr Asp
   1               5                   10                  15
<210> 40
   <211> 15
   <212> PRT
   <213> Artificial sequence
<220>
   <223> Sequence derived from HLA-A2
<400> 40
   Glu Thr Leu Gln Arg Thr Asp Ala Pro Lys Thr His Met Thr His
   1               5                   10                  15
<210> 41
   <211> 15
   <212> PRT
   <213> Artificial sequence
<220>
   <223> Sequence derived from HLA-A2
<400> 41
   Thr His Met Thr His His Ala Val Ser Asp His Glu Ala Thr Leu
   1               5                   10                  15
<210> 42
   <211> 15
   <212> PRT
   <213> Artificial sequence
<220>
   <223> Sequence derived from HLA-A2
<400> 42
   His Ala Val Ser Asp His Glu Ala Thr Leu Arg Cys Trp Ala Leu
   1               5                   10                  15
<210> 43
   <211> 15
   <212> PRT
   <213> Artificial sequence
<220>
   <223> Sequence derived from HLA-A2
<400> 43
   Arg Cys Trp Ala Leu Ser Phe Tyr Pro Ala Glu Ile Thr Leu Thr
   1               5                   10                  15
<210> 44
   <211> 15
   <212> PRT
   <213> Artificial sequence
<220>
   <223> Sequence derived from HLA-A2
<400> 44
   Leu Ser Phe Tyr Pro Ala Glu Ile Thr Leu Thr Trp Gln Arg Asp
   1               5                   10                  15
<210> 45
   <211> 15
   <212> PRT
   <213> Artificial sequence
<220>
   <223> Sequence derived from HLA-A2
<400> 45
   Ala Glu Ile Thr Leu Thr Trp Gln Arg Asp Gly Glu Asp Gln Thr
   1               5                   10                  15
<210> 46
   <211> 15
   <212> PRT
   <213> Artificial sequence
<220>
   <223> Sequence derived from HLA-A2
<400> 46
   Leu Thr Trp Gln Arg Asp Gly Glu Asp Gln Thr Gln Asp Thr Glu
   1               5                   10                  15
<210> 47
   <211> 15
   <212> PRT
   <213> Artificial sequence
<220>
   <223> Sequence derived from HLA-A2
<400> 47
   Thr Glu Leu Val Glu Thr Arg Pro Ala Gly Asp Gly Thr Phe Gln
   1               5                   10                  15
<210> 48
   <211> 15
   <212> PRT
   <213> Artificial sequence
<220>
   <223> Sequence derived from HLA-A2
<400> 48
   Gly Thr Phe Gln Lys Trp Ala Ala Val Val Val Pro Ser Gly Gln
   1 5 10 15
<210> 49
   <211> 16
   <212> PRT
   <213> Artificial sequence
<220>
   <223> Sequence derived from HLA-A2
<400> 49
   Phe Gln Lys Trp Ala Ala Val Val Val Pro Ser Gly Gln Glu Gln Arg
   1 5 10 15
<210> 50
   <211> 15
   <212> PRT
   <213> artificial sequence
<220>
   <223> Sequence derived from HLA-A2
<400> 50
   Ala Ala Val Val Val Pro Ser Gly Gln Glu Gln Arg Tyr Thr Cys
   1 5 10 15
<210> 51
   <211> 16
   <212> PRT
   <213> Artificial sequence
<220>
   <223> Sequence derived from HLA-A2
<400> 51
   Glu Gln Arg Tyr Thr Cys His Val Gln His Glu Gly Leu Pro Lys Pro
   1 5 10 15
<210> 52
   <211> 15
   <212> PRT
   <213> Artificial sequence
<220>
   <223> Sequence derived from HLA-A2
<400> 52
   Glu Gly Leu Pro Lys Pro Leu Thr Leu Arg Trp Glu Pro Ser Ser
   1               5                   10                  15
<210> 53
   <211> 15
   <212> PRT
   <213> Artificial sequence
<220>
   <223> Sequence derived from HLA-A2
<400> 53
   Lys Pro Leu Thr Leu Arg Trp Glu Pro Ser Ser Gln Pro Thr Ile
   1               5                   10                  15
<210> 54
   <211> 15
   <212> PRT
   <213> Artificial sequence
<220>
   <223> Sequence derived from HLA-A2
<400> 54
   Leu Thr Leu Arg Trp Glu Pro Ser Ser Gln Pro Thr Ile Pro Ile
   1               5                   10                  15
<210> 55
   <211> 15
   <212> PRT
   <213> Artificial sequence
<220>
   <223> Sequence derived from HLA-A2
<400> 55
   Pro Thr Ile Pro Ile Val Gly Ile Ile Ala Gly Leu Val Leu Phe
   1               5                   10                  15
<210> 56
   <211> 15
   <212> PRT
   <213> Artificial sequence
<220>
   <223> Sequence derived from HLA-A2
<400> 56
   Ile Pro Ile Val Gly Ile Ile Ala Gly Leu Val Leu Phe Gly Ala
   1 5 10 15
<210> 57
   <211> 17
   <212> PRT
   <213> Artificial sequence
<220>
   <223> Sequence derived from HLA-A2
<400> 57
   Lys Pro Leu Thr Leu Arg Trp Glu Pro Ser Ser Gln Pro Thr Ile Pro
   1 5 10 15
   Ile
<210> 58
   <211> 17
   <212> PRT
   <213> Artificial sequence
<220>
   <223> Sequence derived from HLA-A2
<400> 58
   Pro Thr Ile Pro Ile Val Gly Ile Ile Ala Gly Leu Val Leu Phe Gly
   1 5 10 15
   Ala
<210> 59
   <211> 18
   <212> PRT
   <213> Artificial sequence
<220>
   <223> Sequence derived from HLA-A2
<400> 59
   Gly Thr Phe Gln Lys Trp Ala Ala Val Val Val Pro Ser Gly Gln Glu
   1 5 10 15
   Gln Arg
<210> 60
   <211> 15
   <212> PRT
   <213> Artificial sequence
<220>
   <223> Sequence derived from HLA-A2
<400> 60
   His Pro Ile Ser Asp His Glu Ala Thr Leu Arg Cys Trp Ala Leu
   1 5 10 15
<210> 61
   <211> 15
   <212> PRT
   <213> Artificial sequence
<220>
   <223> Sequence derived from HLA-A2
<400> 61
   His Pro Val Ser Asp His Glu Ala Thr Leu Arg Cys Trp Ala Leu
   1 5 10 15
<210> 62
   <211> 15
   <212> PRT
   <213> Artificial sequence
<220>
   <223> Sequence derived from HLA-A2
<400> 62
   His Pro Val Ser Asp His Glu Val Thr Leu Arg Cys Trp Ala Leu
   1 5 10 15
<210> 63
   <211> 15
   <212> PRT
   <213> Artificial sequence
<220>
   <223> Sequence derived from HLA-A2
<400> 63
   Arg Cys Trp Ala Leu Gly Phe Tyr Pro Ala Glu Ile Thr Leu Thr
   1 5 10 15
<210> 64
   <211> 17
   <212> PRT
   <213> Artificial sequence
<220>
   <223> Sequence derived from HLA-A2
<400> 64
   Lys Pro Leu Thr Leu Arg Trp Glu Leu Ser Ser Gln Pro Thr Ile Pro
   1 5 10 15
   Ile
<210> 65
   <211> 17
   <212> PRT
   <213> Artificial sequence
<220>
   <223> Sequence derived from HLA-A2
<400> 65
   Pro Thr Ile Pro Ile Val Gly Ile Ile Ala Gly Leu Val Leu Leu Gly
   1               5                   10                  15
   Ala
<210> 66
   <211> 17
   <212> PRT
   <213> Artificial sequence
<220>
   <223> Sequence derived from HLA-A2
<400> 66
   Lys Pro Leu Thr Leu Lys Trp Glu Pro Ser Ser Gln Pro Thr Ile Pro
   1               5                   10                  15
   Ile
<210> 67
   <211> 17
   <212> PRT
   <213> Artificial sequence
<220>
   <223> Sequence derived from HLA-A2
<400> 67
   Lys Pro Leu Thr Leu Arg Trp Glu Pro Ser Ser Gln Pro Thr Val Pro
   1               5                   10                  15
   Ile
<210> 68
   <211> 17
   <212> PRT
   <213> Artificial sequence
<220>
   <223> Sequence derived from HLA-A2
<400> 68
   Lys Pro Leu Thr Leu Arg Trp Glu Pro Ser Ser Gln Pro Thr Val His
   1               5                   10                  15
   Ile
<210> 69
   <211> 17
   <212> PRT
   <213> Artificial sequence
<220>
   <223> Sequence derived from HLA-A2
<400> 69
   Pro Thr Val Pro Ile Val Gly Ile Ile Ala Gly Leu Val Leu Leu Gly
   1               5                   10                  15
   Ala
<210> 70
   <211> 17
   <212> PRT
   <213> Artificial sequence
<220>
   <223> Sequence derived from HLA-A2
<400> 70
   Pro Thr Val His Ile Val Gly Ile Ile Ala Gly Leu Val Leu Phe Gly
   1               5                   10                  15
   Ala
<210> 71
   <211> 17
   <212> PRT
   <213> Artificial sequence
<220>
   <223> Sequence derived from HLA-A2
<400> 71
   Pro Thr Ile Pro Ile Val Gly Ile Ile Ala Gly Leu Ala Val Leu Ala
   1               5                   10                  15
   Val
<210> 72
   <211> 17
   <212> PRT
   <213> Artificial sequence
<220>
   <223> Sequence derived from HLA-A2
<400> 72
   Pro Thr Val Pro Ile Val Gly Ile Ile Ala Gly Leu Ala Val Leu Ala
   1               5                   10                  15
   Val
<210> 73
   <211> 18
   <212> PRT
   <213> Artificial sequence
<220>
   <223> Sequence derived from HLA-A2
<400> 73
   Gly Thr Phe Gln Lys Trp Ala Ala Val Val Val Pro Ser Gly Glu Glu
   1               5                   10                  15
   Gln Arg
<210> 74
   <211> 18
   <212> PRT
   <213> Artificial sequence
<220>
   <223> Sequence derived from HLA-A2
<400> 74
   Gly Thr Phe Gln Lys Trp Ala Ser Val Val Val Pro Ser Gly Gln Glu
   1               5                   10                  15
   Gln Arg
<210> 75
   <211> 45
   <212> DNA
   <213> Artificial sequence
<220>
   <223> Sequence derived from HLA-A2
<400> 75
   cactccatga ggtatttctt cacatccgtg tcccggcccg gccgc 45
<210> 76
   <211> 45
   <212> DNA
   <213> Artificial sequence
<220>
   <223> Sequence derived from HLA-A2
<400> 76
   atgaggtatt tcttcacatc cgtgtcccgg cccggccgcg gggag 45
<210> 77
   <211> 45
   <212> DNA
   <213> Artificial sequence
<220>
   <223> Sequence derived from HLA-A2
<400> 77
   cacaagtggg aggcggccca tgtggcggag cagttgagag cctac 45
<210> 78
   <211> 45
   <212> DNA
   <213> Artificial sequence
<220>
   <223> Sequence derived from HLA-A2
<400> 78
   cacgctgtct ctgaccatga agccaccctg aggtgctggg ccctg 45
<210> 79
   <211> 45
   <212> DNA
   <213> Artificial sequence
<220>
   <223> Sequence derived from HLA-A2
<400> 79
   aggtgctggg ccctgagctt ctaccctgcg gagatcacac tgacc 45
<210> 80
   <211> 45
   <212> DNA
   <213> Artificial sequence
<220>
   <223> Sequence derived from HLA-A2
<400> 80
   aagcccctca ccctgagatg ggagccgtct tcccagccca ccatc 45
<210> 81
   <211> 45
   <212> DNA
   <213> Artificial sequence
<220>
   <223> Sequence derived from HLA-A2
<400> 81
   ctcaccctga gatgggagcc gtcttcccag cccaccatcc ccatc 45
<210> 82
   <211> 45
   <212> DNA
   <213> Artificial sequence
<220>
   <223> Sequence derived from HLA-A2
<400> 82
   cccaccatcc ccatcgtggg catcattgct ggcctggttc tcttt 45
<210> 83
   <211> 45
   <212> DNA
   <213> Artificial sequence
<220>
   <223> Sequence derived from HLA-A2
<400> 83
   atccccatcg tgggcatcat tgctggcctg gttctctttg gagct 45
<210> 84
   <211> 45
   <212> DNA
   <213> Artificial sequence
<220>
   <223> Sequence derived from HLA-A2
<400> 84
   ggaaccttcc agaagtgggc ggctgtggtg gtgccttctg gacag 45
<210> 85
   <211> 48
   <212> DNA
   <213> Artificial sequence
<220>
   <223> Sequence derived from HLA-A2
<400> 85
   ttccagaagt gggcggctgt ggtggtgcct tctggacagg agcagaga 48
<210> 86
   <211> 51
   <212> DNA
   <213> Artificial sequence
<220>
   <223> Sequence derived from HLA-A2
<400> 86
   aagcccctca ccctgagatg ggagccgtct tcccagccca ccatccccat c 51
<210> 87
   <211> 51
   <212> DNA
   <213> Artificial sequence
<220>
   <223> Sequence derived from HLA-A2
<400> 87
   cccaccatcc ccatcgtggg catcattgct ggcctggttc tctttggagc t 51
<210> 88
   <211> 54
   <212> DNA
   <213> Artificial sequence
<220>
   <223> Sequence derived from HLA-A2
<400> 88
   ggaaccttcc agaagtgggc ggctgtggtg gtgccttctg gacaggagca gaga 54
<210> 89
   <211> 45
   <212> DNA
   <213> Artificial sequence
<220>
   <223> Sequence derived from HLA-A2
<400> 89
   tcggactggc gcttcctccg cgggtaccac cagtacgcct acgac 45
<210> 90
   <211> 45
   <212> DNA
   <213> Artificial sequence
<220>
   <223> Sequence derived from HLA-A2
<400> 90
   tggcgcttcc tccgcgggta ccaccagtac gcctacgacg gcaag 45
<210> 91
   <211> 45
   <212> DNA
   <213> Artificial sequence
<220>
   <223> Sequence derived from HLA-A2
<400> 91
   caccccatct ctgaccatga ggccaccctg aggtgctggg ccctg 45
<210> 92
   <211> 51
   <212> DNA
   <213> Artificial sequence
<220>
   <223> Sequence derived from HLA-A2
<400> 92
   aagcccctca ccctgagatg ggagccttct tcccagccca ccatccccat c 51
<210> 93
   <211> 51
   <212> DNA
   <213> Artificial sequence
<220>
   <223> Sequence derived from HLA-A2
<400> 93
   cccaccatcc ccatcgtggg catcattgct ggcctggttc tccttggagc t 51
<210> 94
   <211> 45
   <212> DNA
   <213> Artificial sequence
<220>
   <223> Sequence derived from HLA-A2
<400> 94
   aggtgctggg ccctgggctt ctaccctgcg gagatcacac tgacc 45
<210> 95
   <211> 54
   <212> DNA
   <213> Artificial sequence
<220>
   <223> Sequence derived from HLA-A2
<400> 95
   ggaaccttcc agaagtgggc ggctgtggtg gtgccttctg gagaggagca gaga 54
<210> 96
   <211> 54
   <212> DNA
   <213> Artificial sequence
<220>
   <223> Sequence derived from HLA-A2
<400> 96
   ggaaccttcc agaagtgggc gtctgtggtg gtgccttctg gacaggagca gaga 54
<210> 97
   <211> 15
   <212> PRT
   <213> Artificial sequence
<220>
   <223> Sequence derived from HLA-A2
<400> 97
   His Ala Val Ser Asp His Glu Ala Thr Leu Arg Ser Trp Ala Leu
   1 5 10 15
<210> 98
   <211> 15
   <212> PRT
   <213> Artificial sequence
<220>
   <223> Sequence derived from HLA-A2
<400> 98
   His Pro Ile Ser Asp His Glu Ala Thr Leu Arg Ser Trp Ala Leu
   1 5 10 15
<210> 99
   <211> 15
   <212> PRT
   <213> Artificial sequence
<220>
   <223> Sequence derived from HLA-A2
<400> 99
<210> 100
   <211> 15
   <212> PRT
   <213> Artificial sequence
<220>
   <223> Sequence derived from HLA-A2
<400> 100
<210> 101
   <211> 15
   <212> PRT
   <213> Artificial sequence
<220>
   <223> Sequence derived from HLA-A2
<400> 101
   Arg Ser Trp Ala Leu Ser Phe Tyr Pro Ala Glu Ile Thr Leu Thr
   1 5 10 15
<210> 102
   <211> 15
   <212> PRT
   <213> Artificial sequence
<220>
   <223> Sequence derived from HLA-A2
<400> 102
   Arg Ser Trp Ala Leu Gly Phe Tyr Pro Ala Glu Ile Thr Leu Thr
   1 5 10 15
<210> 103
   <211> 15
   <212> PRT
   <213> Artificial sequence
<220>
   <223> Sequence derived from HLA-A2
<400> 103
   Lys Pro Leu Thr Leu Arg Trp Glu Leu Ser Ser Gln Pro Thr Ile
   1               5                   10                  15
<210> 104
   <211> 15
   <212> PRT
   <213> Artificial sequence
<220>
   <223> Sequence derived from HLA-A2
<400> 104
   Lys Pro Leu Thr Leu Arg Trp Glu Leu Ser Ser Gln Pro Thr Val
   1               5                   10                  15
<210> 105
   <211> 15
   <212> PRT
   <213> Artificial sequence
<220>
   <223> Sequence derived from HLA-A2
<400> 105
   Leu Thr Leu Arg Trp Glu Leu Ser Ser Gln Pro Thr Ile Pro Ile
   1               5                   10                  15
<210> 106
   <211> 15
   <212> PRT
   <213> Artificial sequence
<220>
   <223> Sequence derived from HLA-A2
<400> 106
   Leu Thr Leu Arg Trp Glu Pro Ser Ser Gln Pro Thr Val Pro Ile
   1               5                   10                  15
<210> 107
   <211> 15
   <212> PRT
   <213> Artificial sequence
<220>
   <223> Sequence derived from HLA-A2
<400> 107
   Pro Thr Ile Pro Ile Val Gly Ile Ile Ala Gly Leu Val Leu Leu
   1               5                   10                  15
<210> 108
   <211> 15
   <212> PRT
   <213> Artificial sequence
<220>
   <223> Sequence derived from HLA-A2
<400> 108
   Pro Thr Ile Pro Ile Val Gly Ile Ile Ala Gly Leu Ala Val Leu
   1               5                   10                  15
<210> 109
   <211> 15
   <212> PRT
   <213> Artificial sequence
<220>
   <223> Sequence derived from HLA-A2
<400> 109
   Pro Thr Val Pro Ile Val Gly Ile Ile Ala Gly Leu Ala Val Leu
   1               5                   10                  15
<210> 110
   <211> 19
   <212> PRT
   <213> Artificial sequence
<220>
   <223> Sequence derived from HLA-A2
<400> 110
   Lys Arg Pro Thr Ile Pro Ile Val Gly Ile Ile Ala Gly Leu Val Leu
   1               5                   10                  15
   Phe Arg Lys
<210> 111
   <211> 19
   <212> PRT
   <213> Artificial sequence
<220>
   <223> Sequence derived from HLA-A2
<400> 111
   Lys Arg Pro Thr Ile Pro Ile Val Gly Ile Ile Ala Gly Leu Val Leu
   1               5                   10                  15
   Leu Arg Lys
<210> 112
   <211> 19
   <212> PRT
   <213> Artificial sequence
<220>
   <223> Sequence derived from HLA-A2
<400> 112
   Lys Arg Pro Thr Ile Pro Ile Val Gly Ile Ile Ala Gly Leu Ala Val
   1 5 10 15
   Leu Arg Lys
<210> 113
   <211> 19
   <212> PRT
   <213> Artificial sequence
<220>
   <223> Sequence derived from HLA-A2
<400> 113
   Lys Arg Pro Thr Val Pro Ile Val Gly Ile Ile Ala Gly Leu Ala Val
   1 5 10 15
   Leu Arg Lys
<210> 114
   <211> 15
   <212> PRT
   <213> Artificial sequence
<220>
   <223> Sequence derived from HLA-A2
<400> 114
   Ile Pro Ile Val Gly Ile Ile Ala Gly Leu Val Leu Leu Gly Ala
   1 5 10 15
<210> 115
   <211> 15
   <212> PRT
   <213> Artificial sequence
<220>
   <223> Sequence derived from HLA-A2
<400> 115
   Ile Pro Ile Val Gly Ile Ile Ala Gly Leu Ala Val Leu Ala Val
   1 5 10 15
<210> 116
   <211> 15
   <212> PRT
   <213> Artificial sequence
<220>
   <223> Sequence derived from HLA-A2
<400> 116
   Val Pro Ile Val Gly Ile Ile Ala Gly Leu Ala Val Leu Ala Val
   1 5 10 15
<210> 117
   <211> 19
   <212> PRT
   <213> Artificial sequence
<220>
   <223> Sequence derived from HLA-A2
<400> 117
   Lys Arg Ile Pro Ile Val Gly Ile Ile Ala Gly Leu Val Leu Phe Gly
   1 5 10 15
   Ala Arg Lys
<210> 118
   <211> 19
   <212> PRT
   <213> Artificial sequence
<220>
   <223> Sequence derived from HLA-A2
<400> 118
   Lys Arg Ile Pro Ile Val Gly Ile Ile Ala Gly Leu Val Leu Leu Gly
   1 5 10 15
   Ala Arg Lys
<210> 119
   <211> 19
   <212> PRT
   <213> Artificial sequence
<220>
   <223> Sequence derived from HLA-A2
<400> 119
   Lys Arg Ile Pro Ile Val Gly Ile Ile Ala Gly Leu Ala Val Leu Ala
   1 5 10 15
   Val Arg Lys
<210> 120
   <211> 19
   <212> PRT
   <213> Artificial sequence
<220>
   <223> Sequence derived from HLA-A2
<400> 120
   Lys Arg Val Pro Ile Val Gly Ile Ile Ala Gly Leu Ala Val Leu Ala
   1               5                   10                  15
   Val Arg Lys
<210> 121
   <211> 17
   <212> PRT
   <213> Artificial sequence
<220>
   <223> Sequence derived from HLA-A2
<400> 121
   Ser Asp Trp Arg Phe Leu Arg Gly Tyr His Gln Tyr Ala Tyr Asp Gly
   1               5                   10                  15
   Lys
<210> 122
   <211> 13
   <212> PRT
   <213> Artificial sequence
<220>
   <223> reference peptide HA 306-318
<400> 122
   Pro Lys Tyr Val Lys Gln Asn Thr Leu Lys Leu Ala Thr
   1               5                   10
<210> 123
   <211> 13
   <212> PRT
   <213> Artificial sequence
<220>
   <223> reference peptide YKL
<400> 123
   Ala Ala Tyr Ala Ala Ala Lys Ala Ala Ala Leu Ala Ala
   1               5                   10
<210> 124
   <211> 15
   <212> PRT
   <213> Artificial sequence
<220>
   <223> reference peptide MT 2-16
<400> 124
   Ala Lys Thr Ile Ala Tyr Asp Glu Glu Ala Arg Arg Gly Leu Glu
   1               5                   10                  15
<210> 125
   <211> 16
   <212> PRT
   <213> Artificial sequence
<220>
   <223> reference peptide B1 21-36
<400> 125
   Thr Glu Arg Val Arg Leu Val Thr Arg His Ile Tyr Asn Arg Glu Glu
   1               5                   10                  15
<210> 126
   <211> 15
   <212> PRT
   <213> Artificial sequence
<220>
   <223> reference peptide A3 152-166
<400> 126
   Glu Ala Glu Gln Leu Arg Ala Tyr Leu Asp Gly Thr Gly Val Glu
   1               5                   10                  15
<210> 127
   <211> 20
   <212> PRT
   <213> Artificial sequence
<220>
   <223> reference peptide LOL 191-210
<400> 127
   Glu Ser Trp Gly Ala Val Trp Arg Ile Asp Thr Pro Asp Lys Leu Thr
   1               5                   10                  15
   Gly Pro Phe Thr
               20
<210> 128
   <211> 14
   <212> PRT
   <213> Artificial sequence
<220>
   <223> reference peptide E2/E168
<400> 128
   Ala Gly Asp Leu Leu Ala Ile Glu Thr Asp Lys Ala Thr Ile
   1               5                   10
<210> 129
   <211> 15
   <212> PRT
   <213> Artificial
<220>
   <223> Sequence derived from HLA-A2
<400> 129
   Gly Thr Phe Gln Lys Trp Ala Ala Val Val Val Pro Ser Gly Glu
   1               5                   10                  15
<210> 130
   <211> 15
   <212> PRT
   <213> Artificial
<220>
   <223> Sequence derived from HLA-A2
<400> 130
   Gly Thr Phe Gln Lys Trp Ala Ser Val Val Val Pro Ser Gly Gln
   1               5                   10                  15
<210> 131
   <211> 16
   <212> PRT
   <213> Artificial
<220>
   <223> Sequence derived from HLA-A2
<400> 131
   Phe Gln Lys Trp Ala Ala Val Val Val Pro Ser Gly Glu Glu Gln Arg
   1               5                   10                  15
<210> 132
   <211> 16
   <212> PRT
   <213> Artificial
<220>
   <223> Sequence derived from HLA-A2
<400> 132
   Phe Gln Lys Trp Ala Ser Val Val Val Pro Ser Gly Gln Glu Gln Arg
   1               5                   10                  15